(19) Europäisches Patentamt
European Patent Office
Office européen des brevets

(11) Veröffentlichungsnummer: **0 550 704 B1**

(12) # EUROPÄISCHE PATENTSCHRIFT

(45) Veröffentlichungstag der Patentschrift: **21.06.95**

(51) Int. Cl.[6]: **C07D 311/72**, C07C 401/00, C07C 403/20, C07F 9/117, C07F 9/177, A61K 31/355, A61K 31/59, A61K 31/66

(21) Anmeldenummer: **92908582.7**

(22) Anmeldetag: **16.04.92**

(86) Internationale Anmeldenummer: **PCT/CH92/00072**

(87) Internationale Veröffentlichungsnummer: **WO 92/21670 (10.12.92 92/31)**

Teilanmeldung 94112856.3 eingereicht am 16/08/94.

(54) **BIOTENSIDE ESTER MIT VITAMIN-D-VERBINDUNGEN; IHRE HERSTELLUNG UND AUFARBEITUNG ZU SPONTAN DISPERGIERBAREN kONZENTRATEN, SOWIE IHRE VERWENDUNG ZUR BEHANDLUNG VON TUMOREN.**

(30) Priorität: **04.06.91 CH 1662/91**

(43) Veröffentlichungstag der Anmeldung: **14.07.93 Patentblatt 93/28**

(45) Bekanntmachung des Hinweises auf die Patenterteilung: **21.06.95 Patentblatt 95/25**

(84) Benannte Vertragsstaaten: **DE FR GB IT**

(56) Entgegenhaltungen:
**EP-A- 0 008 573**
**EP-A- 0 231 777**
**WO-A-91/05754**
**DE-A- 2 532 000**
**GB-A- 536 602**

(73) Patentinhaber: **MARIGEN S.A.**
**Eugster, Carl, Hackbergstrasse 40**
**CH-4125 Riehen (CH)**

(72) Erfinder: **EUGSTER, Carl**
**Hackbergstrasse 40**
**CH-4125 Riehen (CH)**
Erfinder: **EUGSTER, Conrad, Hans**
**Herrengütlistrasse 18**
**CH-8304 Wallisellen (CH)**
Erfinder: **HALDEMANN, Walter**
**Zeigerweg 23**
**CH-4102 Binningen/BL (CH)**
Erfinder: **RIVARA, Giorgio**
**Ospedale Maggiore San Giovanni Battista**
**Le Molinette,**
**I-10126 Torino (IT)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Lipids, Band 10, Nr. 10, Oktober 1975, (Champaign, Ill., US) T. Nakamura et al.: "Studies on tocopherol derivatives: V. Intestinal absorption of several d,1-3,4-3H2-alpha-tocopheryl esters in the rat", Seiten 627-633, siehe Tabelle I,II, III

Helvetica Chimica Acta, Band 22, Nr. 1, 1939, (Basel, CH) V. Demole et al.:"Ueber Ester des Alpha-Tocopherols", Seiten 65-67, siehe Spalten 3-4

Chemical Abstracts, Band 108, Nr. 15, 11. April 1988, (Columbus, Ohio, US) V.E.Ashirova et al.: "Synthesis of ethers of dl-alpha-tocopherol with higher fattyacids", siehe Seite 786, Zusammenfassung 132119d, & Khim.-Farm. Zh. 1987,21(2), 210-13

Chemical Abstracts, Band 85, Nr. 17, 25. Oktober 1976, (Columbus, Ohio, US)siehe Seite 672, Zusammenfassung 124243f, & JP, A, 7611113 (BOSO OIL AND FAT)8. April 1976

Chemical Abstracts, Band 68, Nr. 17, 22. April 1968, (Columbus, Ohio, US) D.R.Fraser et al.: "Vitamin D esters synthesized in rats. Detection andidentification", siehe Seite 7336, Zusammenfassung 76143y, & Biochem. J.106(2), 485-90

Chemical Abstracts, Band 68, Nr. 17, 22. April 1968 (Columbus, Ohio, US) D.R.Fraser et al.: "Vitamin D esters synthesized in rats. Turnover and sites ofsynthesis", siehe Seite 7336, Zusammenfassung 76144z, & Biochem. J. 106(2),491-6(1968)

Chemical Abstracts, Band 93, Nr. 24, 15. Dezember 1980, (Columbus, Ohio, US) W.Mueller-Mulot et al.: "Identification of naturally occurring vitamin D3 estersin cod-liver oil - a contribution to vitamin D3 analysis", siehe Seite 321,Zusammenfassung 225681t, & Fette, Seifen, Anstrichm. 1980, 82(5), 177-83

Chemical Abstracts, Band 113, Nr. 19, 5. November 1990, (Columbus, Ohio, US) S.Stamatov et al.:Glyceroamidothiophosphates of cholecalciferol (vitamin D3)", see page 743.

(74) Vertreter: **Haldemann, Walter, Dr.**
**c/o MARIGEN S.A.**
**40, Hackbergstrasse**
**CH-4125 Riehen (CH)**

## Beschreibung

Die vorliegende Erfindung betrifft antitumorale Ester mit Vitamin-D-Verbindungen,sowie Verfahren zu ihrer Herstellung wie auch zur Bildung von spontan dispergierbaren Konzentraten.

Verschiede Ester von Vitamin D-Verbindungen sind in Biochem. J. (1968), 106, 485-490 und Chem. Abstr. 93:225681t ohne Hinweis auf eine mögliche therapeutische Anwendung offenbart.

Die Ester mit Vitamin-D-Verbindungen weisen überraschenderweise eine sehr gute antitumorale Wirkung auf, insbesondere dann, wenn sie in spontan dispergierbare Konzentrate eingearbeitet worden sind.

*BESCHREIBUNG DER ERFINDUNG*

Die Ester mit Vitamin-D-Verbindungen zur Verwendung als Arzneimittel mit antitumoraler Wirksamkeit entsprechen den allgemeinen Formeln (I) bis (IV) :

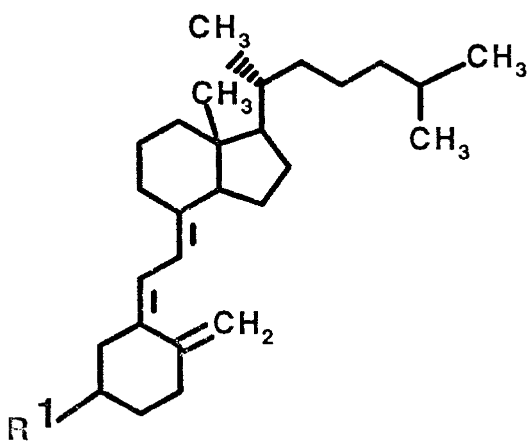

(I)

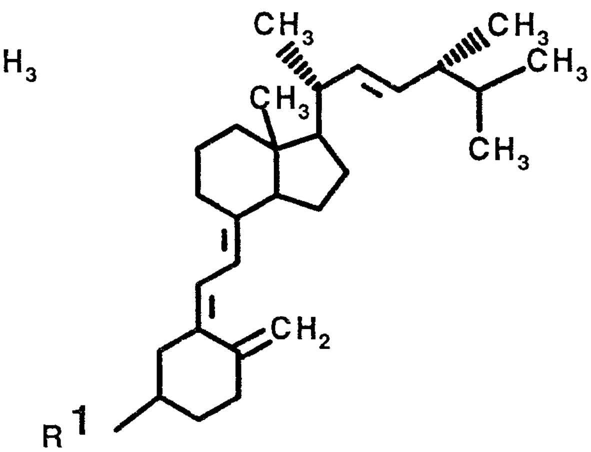

(II)

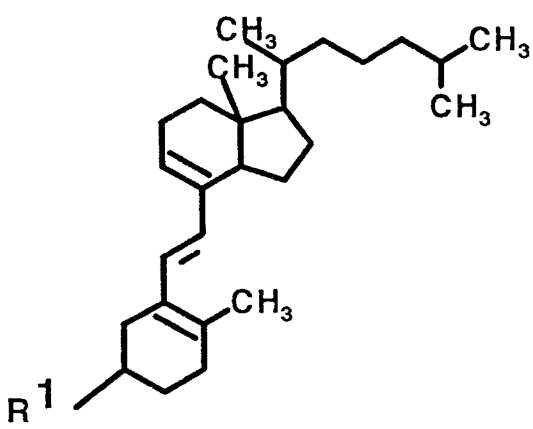

(III)

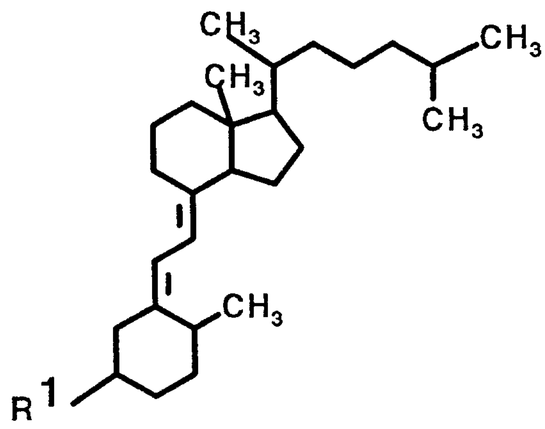

(IV)

wobei das Radikal $R^1$ in den Formeln (I) bis (IV) eine $C_1$ bis $C_{32}$-Alkylcarboxyl-, bzw. eine $C_2$- bis $C_{32}$-Alkenylcarboxyl- oder Alkapolyen-carboxylkette oder eine Gruppe der Formeln (V) oder (VI) :

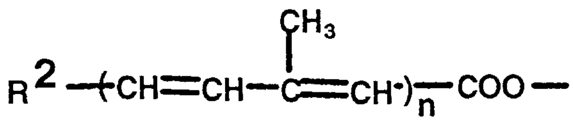

(V)

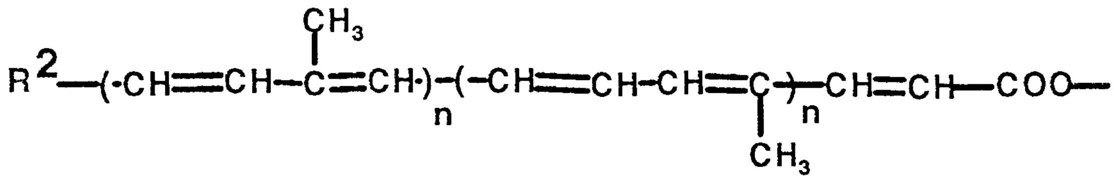

(VI)

darstellt, worin n die Zahlen 1, 2, 3, 4 oder 5 bezeichnet und $R^2$ für ein Radikal der Formeln

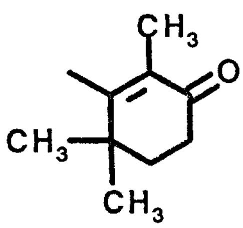
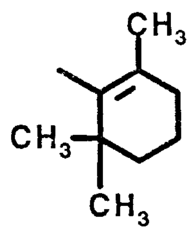
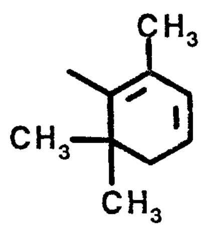
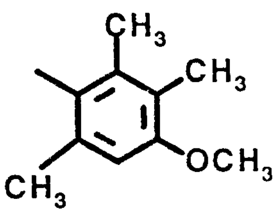
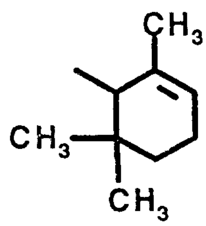
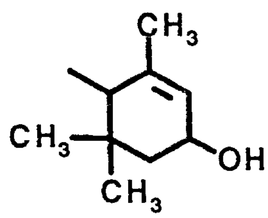

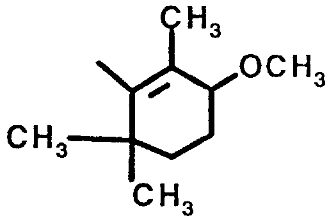

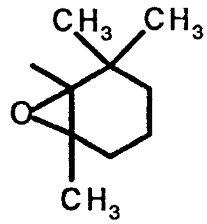

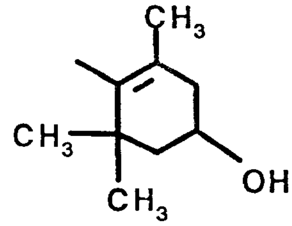

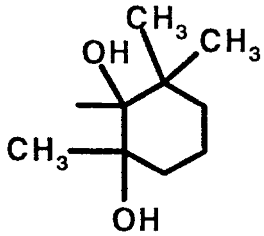

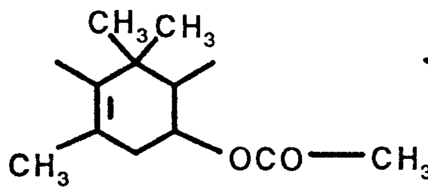

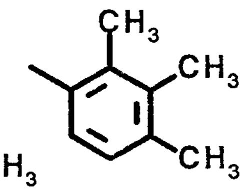

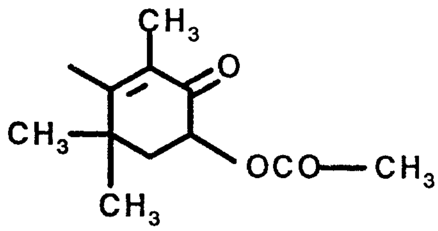

oder

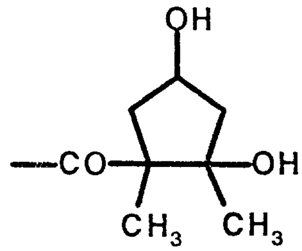

steht.

Die Gruppen der Formeln (V) und (VI) können in verschiedenen stereoisomeren oder Drehformen vorliegen.

Unter Vitamin-D-Verbindungen sind folgende Verbindungen zu verstehen:

Cholecalciferol: (5Z, 7E)-(3S)-9,10-seco-5,7,10 (19)-cholestatrien-3-ol

25-Hydroxycholecalciferol [Calcidiol]:

(5Z, 7E)-(3S)-9,10-seco-5,7,10 (19)-cholestatrien-3,25-diol

1α-Dihydroxy-cholecalciferol [Calcitriol] :

(5Z, 7E)-(1S, 3R)-9,10-seco-5,7,10 (19)-cholestatrien-1,3,25-triol

Ergocalciferol [Calciol] :

(5Z, 7E, 22E)-(3S)-9,10-seco-5,7,10 (19),22-Ergostatetraen-3-ol

Tachysterol: (6E)-(3S)-9,10-seco-5, (10)-6,8-cholestatrien-3-ol

Dihydrotachysterol: (5E, 7E)-(3S, 10S)-9,10-seco-5,7-cholestatrien-3-ol

Die Alkylcarboxyl-, Alkenylcarboxyl oder Alkapolyencarboxylgruppen bei $R^1$ können geradkettig oder verzweigt sein. Unter Alkapolyen sind die entsprechenden Alkadiene, Alkatriene, Alkatetraene, Alkapentaene, Alkahexaene und Alkaheptaene begriffen.

Wichtige Gruppen der Formel (V) sind gekennzeichnet durch die Formeln (VII), (VIII), bzw. (IX) :

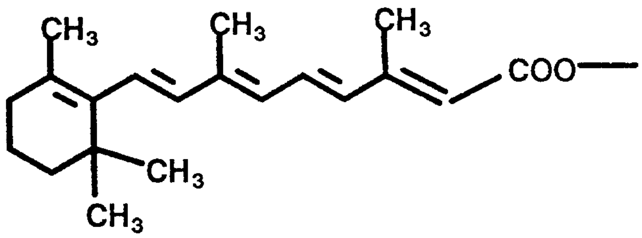

(VII)

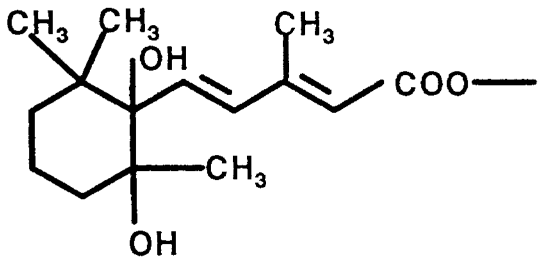

(VIII)

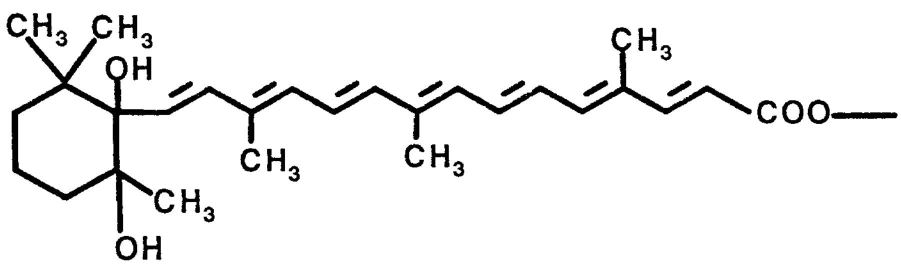

(IX)

Die Gruppe der Formel (VII) liegt in verschiedenen stereoisomeren Formen, so z.B. als all-trans, als 9-cis oder als 13-cis Form vor.

Beispiele von erfindungsgemäss zu verwendenden Estern mit Vitamin-D-Verbindungensind u.a.:

Cholecalciferyl-caproylat
Ergocalciferyl-caproylat
Cholecalciferyl-10-undecenoat
Ergocalciferyl-10-undecenoat
Cholecalciferyl-laurat
Ergocalciferyl-laurat
Cholecalciferyl-palmitat
Ergocalciferyl-palmitat
Cholecalciferyl-linolat
Ergocalciferyl-linolat
Cholecalciferyl-linolenat
Ergocalciferyl-linolenat
Cholecalciferyl-all trans-retinat
Ergocalciferyl-all trans-retinat

Die Ester mit Vitamin-D-Verbindungen der Formeln (I) bis (IV) lassen sich allgemein nach folgenden, an sich bekannten Verfahren herstellen:

a) Umsetzung einer Verbindung der Formeln (X) und (XI):

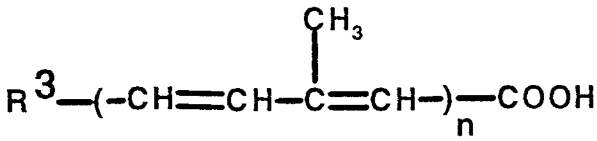

(X)

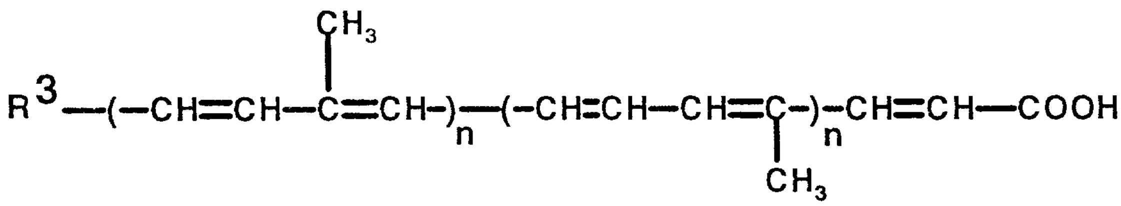

(XI)

worin n in den Zahlen 1, 2, 3, 4 oder 5 ist und $R^3$ für ein Radikal der Formeln:

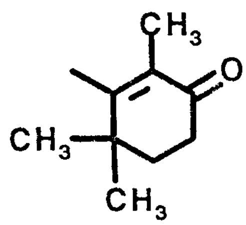
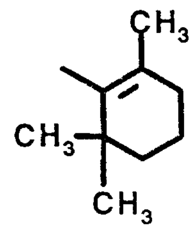
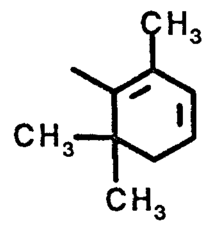

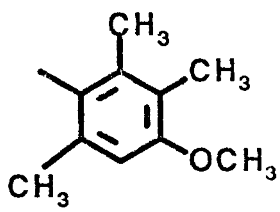
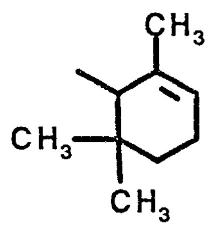
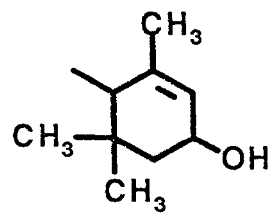

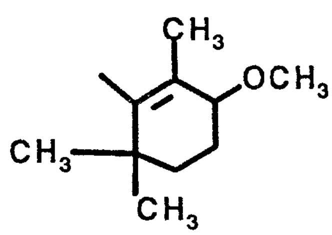
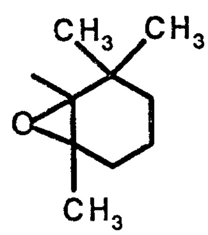
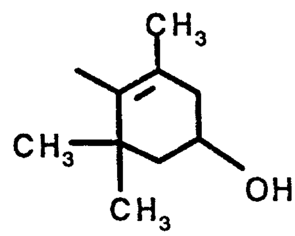

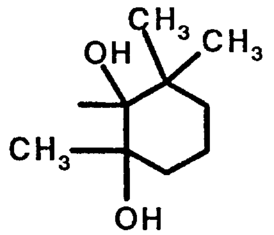

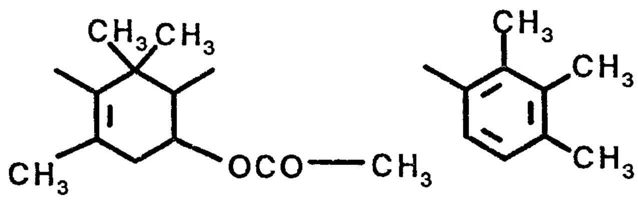

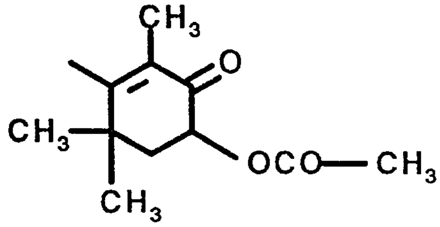 oder 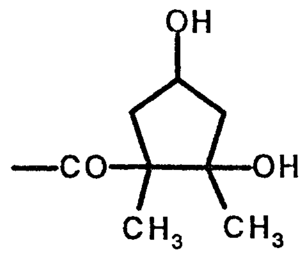

steht, mit N,N'-Carbonyldiimidazol bei 25 bis 70 °C in einem indifferenten Lösungsmittel, wie z.B. Tetrahydrofuran, Benzol, Toluol oder Chloroform und anschliessender Alkoholyse der gebildeten Imidazolide unter Zusatz einer katalytischen Menge eines Alkoholates mit einer Vitamin-D-Verbindung.

b) Bildung des Chlorides, bzw. des Bromides von einer Verbindung der Formeln (X), (XI) oder (XII) :

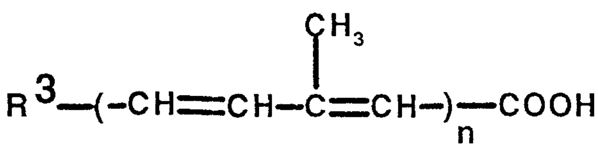

(X)

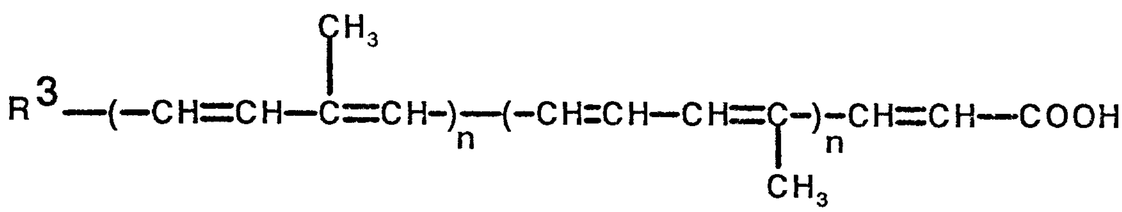

(XI)

$R^4$—COOH (XII )

worin $R^4$ eine $C_1$- bis $C_{32}$-Alkyl- oder eine $C_2$- bis $C_{32}$-Alkenyl-, bzw. $C_2$- bis $C_{32}$-Alkapolyengruppe darstellt, mit einem Chlorierungsmittel, bzw. Bromierungsmittel wie z.B. Thionylchlorid, Oxalylchlorid oder Oxalylbromid und anschliessender Reaktion der entstandenen Chloride, bzw. Bromide mit einer Vitamin-D-Verbindung bei einer Temperatur von 40 bis 120 °C in einem indifferenten Lösungsmittel und in Gegenwart eines Katalyten.

Die *Ester* von Vitamin-D-Verbindungen haben überraschenderweise *eine ausgezeichnete antitumorale Wirkung, vor allem dann, wenn sie in spontan dispergierbare Konzentrate eingearbeitet worden sind*. Gegenstand der vorliegenden Erfindung sind deshalb auch *spontan dispergierbare Konzentrate mit Estern* von Vitamin-D-Verbindungen der Formeln (I) bis (IV). Solche Konzentrate ergeben, mit Wasser versetzt, Mikroemulsionen von hervorragender Phasenstabilität und von überaus deutlich erhöhtem Permeationsvermögen. Sie finden Verwendung als Arzneimittel, sowohl zur prophylaktischen, wie auch zur therapeutischen Verabreichung.

Die erfindungsgemässen, spontan dispergierbaren Konzentrate enthalten:

0,001 bis 15 Gewichts-% einzelner Ester von Vitamin-D-Verbindungen der Formeln (I) bis (IV), bzw. Kombinationen solcher Komponenten, sowie

0 bis 40 Gewichts-% eines als Hydrotrop, bzw. Co-Emulgator dienenden, pharmaverträglichen Lösungsmittels oder Lösungsmittelgemisches

0.001 bis 90 Gewichts-% eines pharmaverträglichen Tensides oder Tensidgemisches
0 bis 10 Gewichts-% eines Vitamins oder Provitamins
0 bis 10 Gewichts-% einer freien Fettsäure und gegebenenfalls übliche Trägerstoffe und/oder Verdünnungsmittel.

Die erfindungsgemäss einzusetzenden Tenside oder Tensidgemische können anionaktiv, kationaktiv, amphoter oder nicht-ionogen sein. Am besten sind sie *nicht-ionogen* und haben ein HLB-Verhältnis (d.h. eine "hydrophiliclipophilic-balance") zwischen 2 und 18; bevorzugt liegt er zwischen 2 und 6 einerseits und 10 und 15 anderseits. HLB-Werte geben Auskunft über die hydrophilen und lipophilen Eigenschaften eines Emulgators. Vgl. dazu "Hydrophile-Lipophile Balance: History and recent Developments" von Paul Becher im Journal of Dispersion Science and Technology. 5 (1), 81-96 (1984).

Geeignete anionische Tenside können sowohl sog. wasserlösliche Seiten als auch wasserlösliche synthetische Verbindungen sein.
Als Seiten eignen sich die Alkali-, Erdalkali- oder gegebenenfalls substituierten Ammoniumsalze von höheren Fettsäuren ($C_{10}$ bis $C_{22}$), wie z.B. die natürlichen Na- oder K-Salze der Oel- oder Stearinsäure, oder von natürlichen Fettsäuregemischen, welche sich u.a. aus Kokosnuss- oder Talgöl gewinnen lassen. Ferner sind als Tenside auch die Fettsäure-Methyltaurinsalze, sowie modifizierte und nicht-modifizierte Phospholipide zu erwähnen.
Häufiger werden jedoch sogenannte synthetische Tenside verwendet, insbesondere Fettsulfonate, Fettsulfate, sulfonierte Benzimidazol-Derivate oder Alkylarylsulfonate.
Die Fettsulfonate und -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst. Beispiele hiefür sind das Na- oder Ca-Salz der Ligninsulfosäure, des Dodecylschwefelsäureesters und Sulfonsäuren von Fettalkohol-Äthylenoxyd-Addukten. Die sulfonierten Benzimidazol-Derivate enthalten vorzugsweise zwei Sulfonsäuregruppen und einen Fettsäurerest mit etwa 8 bis 22 C-Atomen. Alkylaryl-Sulfonate sind z.B. die Na-, Ca- oder Triäthanolaminsalze der Dodecylbenzolsulfonsäure, der Dibutylnaphthalinsulfonsäureoder eines Naphthalinsulfonsäure-Formaldehyd-Kondensationsproduktes.
Als nichtionische Tenside stehen in erster Linie zur Wahl die Polyglykolätherderivate von aliphatischen oder cycloaliphatischen Alkoholen, gesättigten oder ungesättigten Fettsäuren und Alkylphenolen, welche 3 bis 30 Glykoläthergruppen und 8 bis 20 C-Atome im (aliphatischen) Kohlenwasserstoffrest und 6 bis 18 C-Atome im Alkylrest enthalten können. Weiterhin kommen als nichtionische Tenside in Frage die wasserlöslichen, 20 bis 250 Äthylen-glykoläthergruppen und 10 bis 100 Propylenäthergruppen enthaltenden Polyäthylenoxydaddukte an Polypropylenglykol und Alkylpolypropylenglykol mit 1 bis 10 C-Atomen in der Alkylkette. Die genannten Verbindungen enthalten üblicherweise pro Propylenglykoleinheit 1 bis 5 Äthylenglykoleinheiten.
Als Beispiele nicht-ionischer Tenside seien erwähnt:
Nonylphenolpolyäthoxyäthanole, Ricinusölpolyglykoläther, Polypropylenpolyäthylenoxyd-Addukte, Tributylphenoxypolyäthoxyäthanol, Polyäthylenglykol und Octylphenoxypolyäthoxyäthanol. Ueberdies kommen auch Fettsäureester von Polyoxyäthylensorbitan, wie das Polyoxyäthylensorbitan-Trioleat, in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorab als Halogenide, Methylsulfate oder Äthylsulfate vor, z.B. das Stearyltrimethylammoniumchlorid oder das Benzyl-di(2-chloräthyl)äthylammoniumbromid.

In hohem Masse bevorzugt zur Herstellung von erfindungsgemässen, spontan dispergierbaren Konzentraten sind *Phosphorsäureestertenside*, wie z.B. :
Tristyrylphenolpolyoxyäthylen-18-mono/dimethylphosphorsäureester (Soprophor® FL, Rhône-Poulenc);
Nonylphenol-10-polyoxyäthylen-mono/dimethylphosphorsäureester (Diphasol® 3873, CIBA-GEIGY);

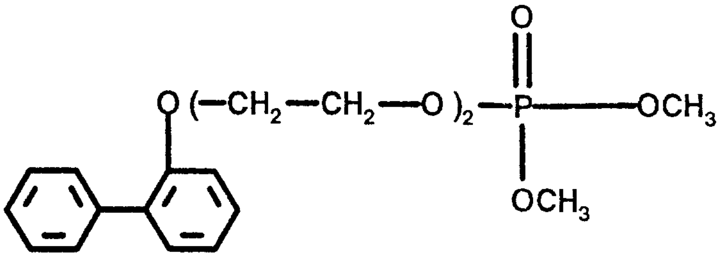

(Tensid 508, CIBA-GEIGY);
Tinovetin® JU (CIBA-GEIGY), ein Hydroxybiphenyl-10-Äthoxy-Phosphorsäureester;
Butyl-mono-4-Äthoxy-Phosphorsäureester (Zerostat® AT, CIBA-GEIGY), bzw.

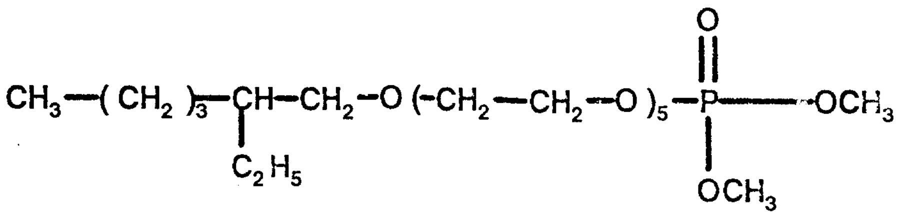

(Zerostat ® AN , CIBA-GEIGY)

Als *Hydrotrop*, bzw. *Co–Emulgator* dienende, pharmaverträgliche Lösungsmittel lassen sich einsetzen, z.B.:
Ester eines aliphatischen Alkohols ($C_3$ bis $C_{18}$) mit einer aliphatischen Carbonsäure ($C_{10}$ bis $C_{22}$), wie etwa Isopropyllaurat, Hexyllaurat, Decyllaurat, Isopropylmyristat, Isopropylpalmitat und Laurylmyristat; Kohlenwasserstoffe mit einer geraden Kohlenstoffkette ($C_{12}$ bis $C_{32}$), welche mit 6 bis 16 Methylgruppen substituiert ist und 1 bis 6 Doppelbindungen aufweisen kann, wofür Terpene wie Polymethylbutane und Polymethylbutene als Beispiele dienen mögen.

Mono-Ester aus Äthylenglykol oder Propylenglykol mit einer aliphatischen Carbonsäure ($C_6$ bis $C_{22}$), wie etwa Propylenglykolmonolaurat und Propylenglykolmonomyristat.
Ester aus einem aliphatischen Alkohol ($C_{12}$ bis $C_{22}$) mit Milchsäure, wie z.B. Myristyl- oder vorzugsweise Lauryl-Lactat; Mono-, Di- oder Triester des Glycerins mit einer aliphatischen Carbonsäure ($C_6$ bis $C_{22}$), wie z.B. Glyceryl-Caprylat, oder Miglyol® 812 Neutralöl (Oleum neutrale).
Ester aus einem Poly(2-7)äthylenglykolglyzerinäther mit mindestens einer freien Hydroxylgruppe mit einer aliphatischen Carbonsäure ($C_6$ bis $C_{22}$), wie z.B. aliphatische Alkohole ($C_{12}$ bis $C_{22}$), somit u.a. Dodecanol, Tetradecanol, Oleylalkohol, 2-Hexyldecanol und 2-Octyldecanol.
Ester mit mindestens einer freien Hydroxylgruppe, aus Poly-(2-10)glykol mit einer aliphatischen Carbonsäure ($C_6$ bis $C_{22}$), Monoäther aus einem Polyäthylenglykol mit einem aliphatischen Alkohol ($C_{12}$ bis $C_{18}$), wie z.B. Polyoxyäthylen ($C_{10}$)-octyläther.
Heterocyclische Verbindungen, wie z.B. 1-Methyl-2-Pyrrolidon.

Alle technischen Tenside wurden vor dem Eintrag in die spontan dispergierbaren Konzentrate mittels Filtration, bzw. Chromatographie über neutralem Aluminiumoxyd mit einem inerten Lösungsmittel wie z.B. Tetrahydrofuran, Äthylalkohol oder Methylenchlorid gereinigt.

Als Zusätze in die erfindungsgemässen spontan dispergierbaren Konzentrate eignen sich Vitamine und Provitamine (wie z.B. Vitamin A, Retinol, Carotine, Tocopherole), sowie auch freie Fettsäuren wie etwa:
Valeriansäure, Isovaleriansäure, Sorbinsäure, Isocapronsäure, Pelargonsäure, Laurinsäure, Myristinsäure, Palmitinsäure, Stearinsäure, Arachidsäure, Behensäure, Hexacosansäure, Octacosansäure, Pentadecansäure, Decenylsäure, Undecenylsäure, Dodecenylsäure, Oleinsäure, Linolsäure, Linolensäure, Arachidonsäure, Erucasäure, etc.

Die zur pharmazeutischen Anwendung erforderliche Tagesdosis beträgt 0,001 bis 25 mg/kg Körpergewicht, wenn möglich verteilt auf 2 bis 3 Einzeldosen. Hiefür lassen sich die biotensiden Ester mit Vitamin-D-Verbindungen oder die damit gebildeten, spontan dispergierbaren Konzentrate in die gängigen pharmazeutischen Zubereitungen und Darreichungsformen wie Dragées, Tabletten, Kapseln, Pulver, Granulat, Pellets, Lösungen, Ampullen, Emulsionen, Crèmes oder Zäpfchen zusammen mit den üblichen Trägerstoffen und/oder Verdünnungs- und Stabilisierungsmitteln einarbeiten.

Die Gegenstand der Erfindung bildenden Wirkstoffe oder Wirkstoffmischungen, sowie die spontan dispergierbaren Konzentrate, welche diese Wirkstoffe oder Wirkstoffmischungen enthalten, können dem Menschen oral, durch Injektion (intravenös, subkutan oder intramuskulär) oder in anderer Weise verabreicht werden. Wenn sie als feste Darreichungsformen für die orale Verwendung aufbereitet werden, kann dies in Form von Tabletten, Granulaten, Pellets, Pulvern oder Kapseln, usw. geschehen. Die Aufbereitungen können Zusatzstoffe enthalten, z.B. einen Arzneimittelträger wie eine Saccharid- oder Cellulose-Grundlage, ein Bindemittel wie Stärkepaste oder Methyl-Cellulose, ein Füllmittel oder ein Desintegriermittel, usw. - wobei Zusatzstoffe eingesetzt werden, wie sie üblicherweise bei der Herstellung medizinischer oder paramedizinischer Formulierungen verwendet werden. Wenn die erfindungsgetreuen Wirkstoffe oder Wirkstoffmischungen als flüssige Darreichungsformen zu oraler Verabreichung gelangen, so können sie irgend

eine aus wässrigen Zubereitungen für innere Verwendung, aus Suspensionen, Emulsionen und Sirups usw. ausgewählte Form haben, und sie können ausserdem in der Form getrockneter Präparate vorliegen, welche vor ihrer Verwendung in Lösung oder Emulsion gebracht werden.
Wenn die erfindungsgemässen Wirkstoffe oder Wirkstoffmischungen in der Form wässriger Lösungen, Suspensionen oder öliger, bzw. wässriger Emulsionen, vorzugsweise als Mikroemulsionen aus den erfindungskonformen, spontan dispergierbaren Konzentraten aufbereitet sind, können sie auch injiziert werden. Die Injektionslösungen werden jedoch üblicherweise kurz vor der Anwendung hergestellt, indem man die Extrakte oder Konzentrate in wässrigen, flüssigen Medien wie sterilem Wasser, Glucoselösung oder physiologischer Kochsalzlösung auflöst oder suspendiert und entgast.

Falls erforderlich, können zu einem Injektionspräparat üblicherweise verwendete Lösungsmittel, Stabilisierungsmittel, Konservierungsmittel und Zusätze für die Herstellung isotonischer Lösungen hinzugegeben werden. Die auf diese Weise erhaltenen Injektions-Präparate werden intravenös, intramuskulär, subkutan oder in einer anderen geeigneten Weise verabreicht.

Die vorliegende Erfindung betrifft ebenfalls ***pharmazeutische Präparate***, welche die Wirkstoffe, bzw. Wirkstoffgemische, und/oder die beschriebenen, spontan dispergierbaren Konzentrate zur Bekämpfung des Wachstums von Tumorzellen enthalten. Bei den der Erfindung entsprechenden pharmazeutischen Präparaten handelt es sich um solche zur enteralen (wie oralen oder rektalen), sowie zur parenteralen oder topischen Verabreichung an Warmblüter, welche das spontan dispergierbare Konzentrat allein oder zusammen mit einem pharmazeutisch anwendbaren Trägermaterial enthalten.

Die Dosierung der erfindungsgemässen Konzentrate hangt von der Warmblüterspezies, dem Alter und dem individuellen Zustand, sowie von der Verabreichungsart ab. So werden z.B. zur Erzielung eines Abtötungseffektes von Tumorzellen an Warmblütern mit geringem Körpergewicht, wie z.B. Mäusen, Ratten und Hamstern, bei sukutaner Verabreichung Dosen im Bereich von ca. 0,1 bis 50 mg/kg Körpergewicht, und bei intraperitonealer Verabreichung Dosen im Bereich von 0,05 bis 5 mg/kg Körpergewicht angewandt.

Die oralen und rektalen Formen der neuen pharmazeutischen Präparate enthalten zwischen 1 bis 95 %, vorzugsweise zwischen 10 bis 95 %, insbesondere zwischen 20 bis 95 % des erfindungsgemässen, spontan dispergierbaren Konzentrates. Sie können z.B. in Dosis-Einheitsform vorliegen, also als Dragees, Micropellets, Tabletten, Suppositorien oder Ampullen und vor allem als Kapseln.

Geeignete pharmazeutisch anwendbare Trägerstoffe für die oralen Formen sind hauptsächlich Füllstoffe, wie Zucker (z.B. Lactose, Saccharose, Mannit oder Sorbit), Cellulosepräparate und/oder Calciumphosphate (z.B. Tricalcium- oder Calciumhydrogenphosphat), ferner Bindemittel wie Stärke-kleister unter Verwendung von u.a. Mais-, Weizen-, Reis- oder Kartoffel-stärke, Gelatine, Traganth, Methylcellulose, Hydroxyl-Methylcellulose, Hydroxypropyl-Methylcellulose, Natriumcarboxymethylcellulose und/oder Polyvinylpyrrolidon und/oder Sprengmittel (wenn erwünscht), wie die obgenannten Stärken, ferner Carboxymethylstärke, quervernetztes Polyvinylpyrrolidon, Agar, Alginsäure oder ein Salz davon, wie z.B. Natriumalginat.

Als Fliessreguliermittel sind z.B. die Polyäthylenglykole Nr. 200 bis 600 und höher geeignet.

Die beim Menschen als Darreichungsform bevorzugten Gelatinekapseln werden mit geeigneten Ueberzügen versehen, wobei man u.a. konzentrierte Zuckerlösungen [welche gegebenenfalls arabischen Gummi, Talk, Polyvinylpyrrolidon, Polyäthylenglykol und/oder Titandioxid enthalten] Lacklösungen (wässrige oder solche, die unter Verwendung organischer Lösungsmittel aufbereitet worden sind), oder magensaftresistente Ueberzüge aus Lösungen von geeigneten Cellulosepräparaten, wie mikrokristalliner Cellulose (Avicel®), Acetylcellulosephthalat, Hydroxymethylcellulosephthalat (Metolose™), Hydroxypropylmethylcellulose-Acetatsuccinat (AQOAT™) oder einem Copolymerisat wie Eudragit® L 30 D verwendet.

Als erfindungsgemäss besonders geeignete, oral anwendbare pharmazeutische Darreichungsform eignen sich Steckkapseln aus Gelatine und einem Weichmacher, wie Glyzerin oder Sorbitol. Die Weich- bzw. Hartgelatine-Kapseln, sowie solche aus AQOAT™ (Hydroxypropyl-Methylcellulose-Acetat-Succinat) können das der Erfindung entsprechende, spontan dispergierbare Konzentrat im Gemisch mit Füllstoffen, wie Laktose, Bindemitteln wie Stärke und/oder Gleitmitteln wie Talk oder Magnesium-Stearat und gegebenenfalls mit Stabilisatoren und Antioxydantien wie z.B. $\alpha$-, $\beta$- oder $\gamma$-Tocopherol enthalten. Der Einsatz von geeigneten Flüssigkeiten wie flüssigen Polyäthylenglykolen No. 200 bis 600 als Verdünnungsmittel kann sich empfehlen, wobei sich ebenfalls Stabilisatoren und Antioxydantien zufügen lassen.

Zur parenteralen Verabreichung werden die erfindungsgemässen Konzentrate mit destilliertem Wasser versetzt. Der entstehenden wässrigen Injektions-Mikroemulsion können viskositätserhöhende Stoffe, wie z.B. Na-Carboxymethylcellulose, Sorbit, Mannit und/oder Dextran, und gegebenenfalls auch Stabilisatoren und Antioxydantien zugefügt werden.

Die pharmazeutischen Präparate für die parenterale Anwendung enthalten vorzugsweise zwischen 0,1 bis 60 %, und hauptsächlich zwischen 1 bis 40 % des erfindungskonformen, spontan dispergierbaren Konzentrates.

Als topisch anwendbare Präparate, welche sich vornehmlich zur Prophylaxe und Therapie von Hautkrebsarten eignen, kommen z.B. Crèmen, Salben, Pasten, Pomaden, Schäume, Tinkturen und Lösungen in Betracht, welche zwischen 0,01 bis 70 % des erfindungsgemässen Konzentrates enthalten.
Für Crèmen und Oel-in-Wasser-Emulsionen, welche mehr als 50 % Wasser aufweisen, verwendet man als oelige Grundlage in erster Linie Fettalkohole, z.B. Lauryl-, Cetyl- oder Stearylalkohol, flüssige bis feste Wachse, z.B. Isopropylmyristat, Woll- oder Bienenwachs und/oder Kohlenwasserstoffe wie z.B. Vaseline (Petrolatum) oder Paraffinoel. Zur Emulgierung dieser öligen Grundlagen eignen sich in erster Linie oberflächenaktive, pharmaverträgliche Substanzen mit vorwiegend hydrophilen Eigenschaften, wie z.B. nicht-ionogene Emulgatoren, vorab Fettsäureester von Polyalkoholen oder Äthylenoxydaddukten (etwa Polyglycerinfettsäureester oder Polyäthylensorbitan-Fettsäureester) mit einem HLB-Wert von unter 8. Zusätze zur Wasserphase sind u.a. Mittel, welche die Austrocknung der Crèmen vermindern, z.B. Polyalkohole wie Glyzerin, Sorbit, Propylenglykol und/oder Polyäthylenglykole No. 200 bis No. 600, ferner Konservierungsmittel, Riechstoffe, etc.

Salben sind Wasser-in-Oel Emulsionen, die bis zu 70 %, vorzugsweise jedoch zwischen 20 und 50 % Wasser oder wässrige Phasen enthalten.
Als Fettphase kommen in erster Linie Kohlenwasserstoffe, z.B. Vaselin, Paraffinoel und/oder Hartparaffine in Frage, welche zur Verbesserung des Wasserbindungsvermögens geeignete Hydroxydverbindungen, wie z.B. Fettalkohole oder Ester, davon etwa Cetylalkohol oder Wollwachsalkohole, enthalten.
Fallweise werden noch Emulgatoren mit einem HLB-Wert von 8 bis 16, wie z.B. Sorbitan-Fettsäureester (etwa Sorbitanisostearol) zugesetzt. Zusätze zur Wasserphase sind u.a. Feuchthaltungsmittel, wie Polyalkohole (Glycerin, Propylenglykol, Sorbit und/oder Polyäthylenglykole No. 200, 400, 600); ferner Konservierungsmittel, Riechstoffe, etc.

Fettsalben sind wasserfrei und enthalten als Grundlage vornehmlich Kohlenwasserstoffe, z.B. Paraffin, Vaselin und/oder flüssige Paraffine; überdies natürliche oder partial synthetische Fette wie z.B. Kokosfettsäuretriglycerid, ferner: Fettsäurepartialester des Glycerins, wie z.B. die im Zusammenhang mit den Salben erwähnten, die Wasser-Aufnahmefähigkeit steigernden Fettalkohole, Emulgatoren und/oder Zusätze.
Pasten sind Crèmen und Salben mit sekretabsorbierenden Puderbestandteilen, wie beispielsweise Metalloxide (etwa Titanoxid oder Zinkoxid), ferner Talk und/oder Aluminiumsilikate, welche die Aufgabe haben, vorhandene Feuchtigkeit oder Sekrete zu binden.
Schäume werden aus Druckbehältern verabreicht und sind in Aerosolform vorliegende Oel-in-Wasser Emulsionen der erfindungsgemässen, spontan dispergierbaren Konzentrate, wobei halogenierte Kohlenwasserstoffe (wie z.B. Chlorfluorniederalkane: etwa Dichlordifluormethan und Dichlortetrafluoräthan) als Treibmittel verwendet werden. Dazu kommen gegebenenfalls die üblichen Zusätze wie Konservierungsmittel, usw.

*Verfahrensbeispiele zur Herstellung der erfindungsgemäss zu verwendenden Ester mit Vitamin–D–Verbindungen.*

*1. Herstellung von Cholecalciferyl–palmitat*

Zu 450 mg Cholecalciferol (Vitamin $D_3$) und 50 mg Dimethylformamid in 30 ml Toluol werden bei 20 °C 350 mg Palmitoylchlorid (ca. 20 % Überschuss) in 20 ml Toluol zugetropft.
Die Reaktionslösung wird am Rückfluss während drei Stunden auf 80 °C erhitzt. Anschliessend wird das Lösungsmittel durch Vakuumdestillation entfernt und der Rückstand in Acetonitril umkristallisiert. Man erhält das reine Cholecalciferylpalmitat mit einem Brechungsindex $n_D^{20}$ (BI) von 1,51418.
Cholecalciferylpalmitat

| | |
|---|---|
| IR | 2928 $cm^{-1}$ $\nu$ (CH) |
| | 2855 $cm^{-1}$ $\nu$ (CH) |
| | 1723 $cm^{-1}$ $\nu$ (C=O) Ester |
| | 1467 $cm^{-1}$ $\delta$ (CH) |
| | 1370 $cm^{-1}$ $\delta$ ($CH_3$) |
| | 1184 $cm^{-1}$ $\nu$ (C-O) |
| | 959 $cm^{-1}$ $\delta$ (CH) olefin. CH |

Auf gleiche Weise werden auch folgende Verbindungen hergestellt:
Ergocalciferylpivaloylat
BI 1.52148
Ergocalciferylvalerat
BI 1.53924

Ergocalciferylcaproylat
UV λmax. 302,4 290,4 nm
BI 1.53010
Ergocalciferyllaurat
UV λmax. 301.6 289,0 nm
Ergocalciferylpalmitat
BI 1.50316
Ergocalciferylcrotonat
UV λmax. 300,4 288,6 nm
BI 1.55342
Ergocalciferyl-10-undecenoat
UV λmax. 302,4 292.4 nm
BI 1.50360
Ergocalciferyloleat
BI 1.52620
Cholecalciferylpivaloylat
BI 1.51292
Cholecalciferylvalerat
BI 1.52714
Cholecalciferylcaproylat
BI 1,51756
Cholecalciferylcaproylat
IR 2931 $cm^{-1}$ ν (CH)
2870 $cm^{-1}$ ν (CH)
1722 $cm^{-1}$ ν (C=O) Ester
1467 $cm^{-1}$ δ (CH)
1370 $cm^{-1}$ δ ($CH_3$)
1171 $cm^{-1}$ ν (C-O)
959 $cm^{-1}$ δ (CH) Vinyliden
Cholecalciferylcrotonat
BI 1,55444
IR 2955 $cm^{-1}$ ν (CH)
1717 $cm^{-1}$ ν (C=O)
1467 $cm^{-1}$ δ (CH)
1369 $cm^{-1}$ δ ($CH_3$)
1189 $cm^{-1}$ ν (C-O)
959 $cm^{-1}$ δ (CH) Olefinic CH
Cholecalciferyl-10-undecenoat
BI 1,51796
IR 2931 $cm^{-1}$ ν(CH)
1725 $cm^{-1}$ ν(C=O) Ester
1458 $cm^{-1}$ δ(CH)
1371 $cm^{-1}$ δ(CH3)
1179 $cm^{-1}$ ν(C-O)
δ(CH) } 974 $cm^{-1}$ { δ(CH)
trans } 913 $cm^{-1}$ { δ(CH) of CH=CH2
Cholecalciferyloleat
BI 1.52092
IR 2931 $cm^{-1}$ δ (CH)
1723 $cm^{-1}$ ν (C=O)
1458 $cm^{-1}$ δ (CH)
1372 $cm^{-1}$ δ ($CH_3$)
1187 $cm^{-1}$ ν (C-O)
973 $cm^{-1}$ δ (CH) trans
Cholecalciferyl-all trans-retinat
NIR 1627 $cm^{-1}$ (C=C) [$D_3$]
1583 $cm^{-1}$ (C=C) [Retinat]
Cholecalciferylcrotonat

BI 1,55444

Cholecalciferyl-10-undecenoat

BI 1,55796

Cholecalciferyloleat

BI 1.52092

*2. Herstellung von Ergocalciferyl–Linolenat*

Zu 300 mg Linolensäure in 20 ml Toluol tropft man bei 10 °C 200 mg Oxalylchlorid in 30 ml Toluol. Die Reaktionslösung wird bei 20 °C 24 Stunden stehen gelassen.

Anschliessend werden unter Vakuum 10 ml Toluol abdestilliert und dann 400 mg Ergocalciferol sowie 50 mg Dimethylformamid zugesetzt. Nach 2-stündiger Erhitzung bei 90 °C wird das Lösungsmittel durch Vakuumdestillation entfernt. Der Rückstand wird auf einer Silicagelsäule mit Cyclohexan/Essigester (80:20) als Eluiermittel chromatographiert. Man erhält das Ergocalciferyllinolenat, mit einem Brechungsindex $n_D^{20}$ (BI, englisch "Refractive Index" RI) von 1,48452 *)Ergocalciferyl-Linolenat

IR 2931 $cm^{-1}$ δ (CH)
1723 $cm^{-1}$ ν (C=O)
1458 $cm^{-1}$ δ (CH)
1372 $cm^{-1}$ δ ($CH_3$)
1187 $cm^{-1}$ ν (C-O)
973 $cm^{-1}$ δ (CH) trans

Auf entsprechende Weise werden auch folgende Verbindungen hergestellt:

Cholecalciferyl-Linolenat

BI 1.54030

Ergocalciferyl-Linoleat

BI 1.53274

Cholecalciferyl-Linoleat

BI 1.52138

Ergocalciferyl-all trans-retinat

UV λmax. 292,8
BI 1,51670
IR 2958 $cm^{-1}$ ν (CH)
2871 $cm^{-1}$ ν (CH)
1701 $cm^{-1}$ ν (C=O)
1608 $cm^{-1}$ ν (C=C)
1583 $cm^{-1}$ ν (C=C)
1457 $cm^{-1}$ δ (CH)
1239 $cm^{-1}$ ν (C=O)
1153 $cm^{-1}$ ν (C=O)
1016 $cm^{-1}$ ν (C=O)
970 $cm^{-1}$ trans C=C
δ (CH)

Cholecalciferyl-all trans-retinat

BI 1,48774
IR 2958 $cm^{-1}$ ν (CH)
2871 $cm^{-1}$ ν (CH)
1701 $cm^{-1}$ ν (C=O)
1608 $cm^{-1}$ ν (C=C)
1583 $cm^{-1}$ ν (C=C)
1457 $cm^{-1}$ δ (CH)
1239 $cm^{-1}$ ν (C=O)
1153 $cm^{-1}$ ν (C=O)

*) N.B.: BI = Brechungsindex, Refractive Index (RI), gemessen im DUR Refractometer Schmidt + Haensch, Berlin.
IR = Infrarot Spektren gemessen am Spektrophotometer Perkin-Elmer 683G
NIR = Near Infrared (FT RAMAN) Spektren, gemessen am Spektrometer BRUKER IFS 88 mit RAMAN Modul FRA 106 und NIR Neodym-YAG Laser, Anregung bei 1064 nm
UV-Spektren gemessen am Spektrophotometer Shimadzu UV-160A

1016 $cm^{-1}$ $\nu$ (C=O)
970 $cm^{-1}$ trans C=C
$\delta$ (CH)
NIR 1627 $cm^{-1}$ (C=C) [$D_3$]
1583 $cm^{-1}$ (C=C) [Retinat]

Ergocalciferyl-13 cis-retinat
NIR 1627 $cm^{-1}$ (C=C) [$D_2$]
1585 $cm^{-1}$ (C=C) [Retinat]

Cholecalciferyl-13 cis-retinat
NIR 1627 $cm^{-1}$ (C=C) [$D_3$]
1583 $cm^{-1}$ (C=C) [Retinat]

*3. Herstellung von Ergocalciferyl–Azafrinat*

Zu 80 mg Azafrinsäure [Herstellung vgl. Helv.Chim.Acta 58 (1975) 1722-1727 oder Helv.Chim.Acta 65 - (1982) 353-354] in 50 ml Chloroform werden bei 20 °C 65 mg N,N'-Carbonyldiimidazol zugesetzt. Man lässt die Reaktionslösung während 12 Stunden bei 20 °C stehen und setzt dann 40 mg Ergocalciferol zu. Nach weiteren 12 Stunden bei 30 °C wird das Lösungsmittel abdestilliert und der Rückstand in 30 ml Essigester aufgenommen. Diese Lösung schüttelt man einmal mit 1/10N Salzsäure und einmal mit 1/10N Natronlauge aus. Nach dem Abdestillieren des Lösungsmittels wird der Rückstand auf einer Silicagelsäule mit Hexan/Essigester (80:20) als Eluiermittel chromatographiert.
Man erhält das Ergocalciferyl-Azafrinat mit einem Schmelzbereich von 171 bis 173 °C, UV Absorption $\lambda$max. 429,5 nm.

Rf - **WERTE** von erfindungsgemässen Verbindungen, welche nach den Verfahrenbeispielen 1 bis 3 hergestellt worden sind:

1%-Lösung in $CH_2Cl_2$, bandförmig aufgetragen cm/2$\mu$l
LINOMAT III CAMAG 10 cm run
UV 366 nach 1:1 $H_2SO_4$/MeOH 2 min 120° C

| SYSTEM<br>VERBINDUNG | 1 | 2 | 3 | 4 | 5 | 6 | 7 |
|---|---|---|---|---|---|---|---|
| $D_2$ - C 16:0 | 0.25 | 0.13 | 0.35 | 0.89 | 1.0 | 1.0 | 0.88 |
| $D_2$ - trans-RET. | 0.21 | 0.1 | 0.33 | 0.85 | 0.97 | 1.0 | 0.83 |
| | | | | | | | |
| $D_3$ - C 11:1 | 0.22 | 0.13 | 0.42 | 0.87 | 0.88 | 0.92 | 0.75 |
| $D_3$ - trans-RET | 0.15 | 0.14 | 0.41 | 0.89 | 1.0 | 0.94 | 0.79 |

| *Erklärung:* | | | |
|---|---|---|---|
| System 1 | Platte Merck Art. 5715 | Petroläther/Diäthyläther | 97:3 |
| System 2 | do. | do. | 98:2 |
| System 3 | do. | Cyclohexan/Äthylacetat | 97:3 |
| System 4 | Platte Macherey Nagel RP 18w Art. 811'071 | Petroläther/Diäthyläther | 95:5 |
| System 5 | do. | n-Hexan/t.Butylmethyläther/Aceton | 90 : 5 : 5 |
| System 6 | do. | Petroläther/Cyclohexan/Äthylacetat/$H_2O$ | 48:48:3:1 |
| System 7 | do. | Petroläther/Diäthyläther | 97:3 |

*Zusammensetzungsbeispiele von erfindungsgemässen, spontan dispergierbaren Mitteln zur Verwendung als Arzneimittel, welche als antitumorale Wirkstoffe Sterolester gemäss den Formeln (I) bis (IV) enthalten*

a) 7,5 bis 15 Gewichts-% eines oder mehrerer Ester mit Vitamin-D-Verbindungen der Formeln (I) bis (IV)
1 bis 40 Gewichts-% Isopropylmyristat, Isopropylpalmitat oder Miglyol® 812 (Dynamit Nobel)
20 bis 45 Gewichts-% Diphasol® 3873 (CIBA-GEIGY)
20 bis 45 Gewichts-% Invadin® JFC 800 % (CIBA-GEIGY)

b) 7,5 bis 15 Gewichts-% eines oder mehrerer Ester mit Vitamin-D-Verbindungen der Formeln (I) bis (IV)
1 bis 40 Gewichts-% Isopropylmyristat, Isopropylpalmitat oder Miglyol® 812 (Dynamit Nobel)
20 bis 45 Gewichts-% Invadin® JFC 800% (CIBA-GEIGY)
20 bis 45 Gewichts-% Soprophor® FL (Rhône-Poulenc)

Miglyol® 812-Neutralöl ist ein *Neutralöl* (Oleum neutrale) der Firma Dynamit Nobel, das einem gemischtsäurigen Triglycerid der fraktionierten Kokosfettsäuren $C_8$ bis $C_{10}$ entspricht.

Diphasol® 3873 ist ein Mischemulgator, bestehend aus je 50 % der beiden Verbindungen mit den Formeln:

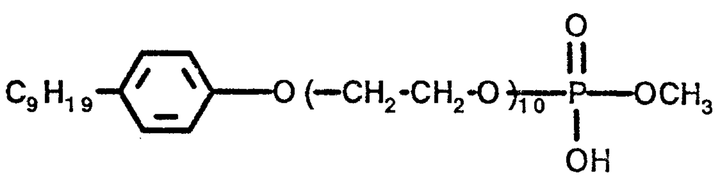

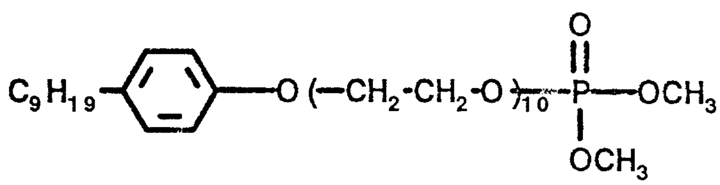

(DIPHASOL® 3873 CIBA-GEIGY)

Invadin® JFC 800% (CIBA-GEIGY) ist ein tert. Octylphenylpolyoxyäthylenaether mit 9 bis 10 Oxyäthylengruppen.

Soprophor® FL (Rhône-Poulenc) ist ein Tristyrylphenolpolyoxyäthylen-18-mono/dimethyl-Phosphorsäureester:

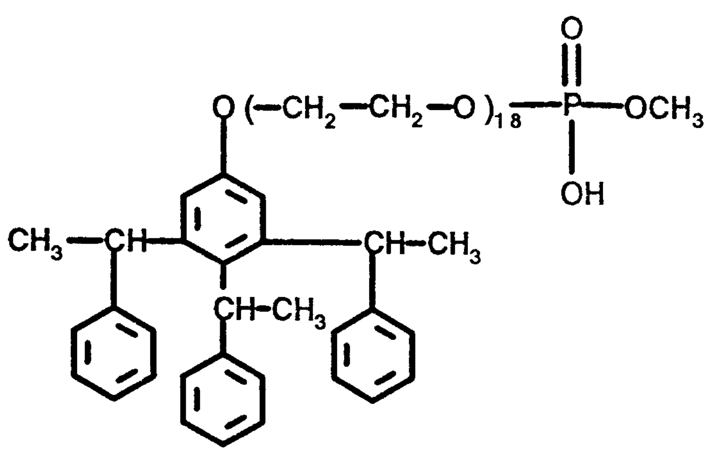

*Experimenteller Nachweis des Spreitungs– und Wanderungsvermögens der erfindungsgemässen Konzentrate und Microemulsionen.*

Methode: DC-Platte 0,25 mm Kieselgel 60F254, Merck Art. No. 11'798 mit Konzentrationszone
Laufmittel: PBS Dulbecco's ohne Ca und Mg (= Ringerlösung, bzw. Kochsalzlösung, gepuffert)
Entwicklungszeit: 15 Minuten

**ERGEBNIS** : Rf.-Werte

| | 0,1 % Substanz, gelöst in Methylenchlorid | Microemulsion mit 1000 ppm. Konzentrat |
|---|---|---|
| | Rf.-Werte: | |
| C 8:0-Ergocalciferyl-Ester | 0 | 21,21 |
| Ergocalciferyl-all trans Retinat | 0 | 48,39 |
| Ergocalciferyl-all trans Retinat : (mit DL-α-Tocopheryl-Acetat als Co-Emulgator) | 0 | 45,45 |
| *P.S.*: Mikroemulsionen 1:1'000 = 1'000 ppm Konzentrat = 1 mg/ml. | | |

Die oben angeführten Rf.-Werte vermitteln wichtige Hinweise auf das Verhalten der erfindungsgemässen, spontan dispergierbaren Konzentrate, welche mit den neuen Verbindungen der Formeln (I) bis (IV) gebildet und mit Wasser verdünnt werden, in den Zellverbänden und insbesondere an den Zellmembranen. Durch die geringe Oberflächenspannung für diese Konzentrate mit Werten von 30 bis 32 $mN/m^{-1}$, den kleinen Teilchenradius der sich ausbildenden Mikromizellen, sowie die niedrige Viskosität der Emulsionen werden vorab das Diffusionsvermögen und die Spreitung der neuen, antitumoralen Verbindungen der Formeln (I) bis (IV) in ausnehmend günstiger Weise beeinflusst. Kontrollmessungen, ausgeführt am Institut für Polymere der E.T.H., Zürich, haben ergeben, dass die hydrodynamischen Radien für die Mizellen um 1 nm betragen. (Prof. Dr. Pier Luigi LUISI und Prof. Dr. Peter SCHURTENBERGER).

*Beispiel für die pharmazeutische Herstellung eines Systempräparates mit erfindungsgemässen Konzentraten in der Form von "multiple units".*

a) Granulierung

| | |
|---|---|
| Metolose® 90 SH-4000 (Shin-Etsu Chemical) | 90.0 g |
| Avicel® PH-101 | 80.3 g |
| Erfindungsgemässes KONZENTRAT | 139.4 g |
| Aerosil® 200 | 80.3 g |
| | Σ 390.0 g |

Granulieren/formen im Schnellmixer oder im Rotationsbett unter Zusatz von 110 g Äthanol, brechen, sieben 18 bis 42 mesh, trocknen 24 h bei 40 °C.
b) MSR- und RETARD-Ausrüstung
im Rotationsbett mit AQOAT® AS-HG (Shin-Etsu Chemical) und Talk
c) Zusammensetzung fertiges Granulat/bzw. Micropellets

| | |
|---|---|
| Kernmaterial | 44 % |
| Erfindungsgemässes KONZENTRAT | 25 % |
| MSR-Beschichtung | 31 % |
| | Σ 100 % |

N.B.: MSR = Magensaft-Resistenz. Die Pellets/Granulate gemäss a) können auch ohne Befilmung unmittelbar in Kapseln abgefüllt werden, welche aus AQOAT™ (HPMC-AS-M oder HPMC-AS-N) hergestellt sind, mit Aceton/Ethanol 1:1 verschlossen werden und so die Funktionen der MSR und der verzögerten Abgabe (Retard) angemessen steuern.

*Biologische Prüfungen*

Die antitumorale Wirkung von spontan dispergierbaren Konzentraten mit Wirkstoffen gemäss den Aufarbeitungsbeispielen No. 1 bis 4 wird anhand folgender Prüfungsergebnisse bestätigt:

*1. In vitro–Tests mit geeigneten Tumorzell–Linien*

Es wurde ein biologisches Assay-System entwickelt, das mit Mikrotiterplatten und Verdünnungsreihen arbeitet. Angesetzt werden je $10^4$/ml Tumorzellen in Kulturmedium RPMI 1640 mit 10 % fötalem Kalbserum inaktiviert (GIBCO); sie werden so undicht ausgesät, dass sie während des Assays wachsen können, in sog. nichtkonfluenten Monolayers. Die Probenzugabe erfolgt nach 6 bis 24 Stunden, mit 100 µl pro Reihe, die man im 1. Loch mit 100 µl Medium versetzt. Davon wird die Hälfte entnommen und in das folgende Loch eingebracht, wieder mit 100 µl Medium versetzt, usf. Es entsteht eine geometrische Verdünnungsreihe $n\frac{1}{2}$.

Die Proben werden im Plaque Assay während 3 bis 5 Tagen bei 37° C mit $3\frac{1}{2}$ % $CO_2$ inkubiert. Anschliessend färben/fixieren mit 0,1 % Kristallviolett (Fluka, Buchs) in einer Lösung von 70 % Methanol, 1 % Formaldehyd, 29 % Wasser. Die Auswertung wird am Mikroskop vorgenommen, Vergrösserung 300-fach. Man bestimmt die grösste cytotoxische Verdünnung. Die quantitative Auswertung lässt sich auch mittels Scanning und Absorptionsmessung am Spektrophotometer vornehmen.

*AUSWERTUNG DER ERGEBNISSE*

a) TS/A-Assay: grösste zelltoxische Verdünnung

| TUMORLINIE / PRÄPARAT | TS/A Murines Adenokarzinom In Verdünnung wirksam bis 1: | |
|---|---|---|
| EXPOSITION | 24 h | 72 h |
| $D_2$ - C 4 : 1 | 20'480'000 | -- |
| $D_2$ - C 8 : 0 | 2'000'000 | 4'000'000 |
| $D_2$ - C 11 : 1 | 40'960'000 | 163'840'000 |
| $D_2$ -all trans-RET. | 16'000'000 | 64'000'000 |

TS/A :murines Adenokarzinom (spontanes Mammakarzinom)
Prof.Dott. Guido FORNI, Istituto di Microbiologia, Università degli Studi di TORINO, Scuola di Medicina

b) Py 6-ASSAY: grösste zelltoxische Verdünnung

| TUMORLINIE / PRÄPARAT | Py 6 (Polyoma transformed 3T3 mouse cells, Fibroblasten) In Verdünnung wirksam bis 1 : | |
|---|---|---|
| EXPOSITION | 20 h | 60 h |
| $D_2$ - C 4:1 | 10'240'000 | -- |
| $D_2$ - C 11:1 | 5'120'000 | -- |
| $D_2$ -all trans-RETINAT | 16'000'000 | 32'000'000 |
| $D_2$ - C 18:3 | 6'025'000 | 96'375'000 |
| $D_3$ - C 18:3 | 6'000'000 | 24'000'000 |

Py 6 : Polyoma-Virus-transformierte 3T3 Zellen der Maus (Fibroblasten)

c) Andere Tumor-Zell-Linien

| TUMORLINIENEN<br>PRÄPARAT | Y T 43<br>In Verdünnung wirksam bis 1 : | P 815<br>In Verdünnung wirksam bis 1 : | L 929<br>In Verdünnung wirksam bis 1 : |
|---|---|---|---|
| Exposition | 4 Tg | 4 Tg | 2 Tg |
| CALCIOL - C 4 : 1 | 48'000'000 | 16'000'000 | 64'000'000 |
| CALCIOL - C 8 : 0 | 32'000'000 | 32'000'000 | 64'000'000 |
| CALCIOL - C 11:1 | 32'000'000 | 16'000'000 | 32'000'000 |
| CALC.-all trans-RETINAT | 128'000'000 | 64'000'000 | 128'000'000 |

Y T 43 : ibridoma/anticorpi monoclonali (Hybridom-Zellen)
P 815 : mastocitoma
L 929 : fibroblastoma

Prof. Guido Forni, Istituto di Microbiologia, Università degli Studi di Torino, Scuola di Medicina.

d) Zelltoxizität auf humanen Tumorzell-Linien

Ueberlebensquote in % (MTT-Methode mit Tetrazolblau)

VERDUENNUNG 1 : 1'000'000

| ZELL-LINIE<br>PRÄPARAT | ST-4 | RAJI | K 562 | H L 60 |
|---|---|---|---|---|
| CALCIOL-CROTONAT | -- | 8 | 3 | 9 |
| CALCIOL-CAPROYLAT<br>CALCIOL- C 11:1 | 1 | 7 | 8 | 7 |
| CALCIOL-all tr.-RET. | -- | 9 | 3 | 8 |
| | 1 | 2 | 0 | 0 |

VERDUENNUNG 1 : 10'000'000

| ZELL-LINIEN PRÄPARAT | ST-4 | RAJI | K 562 | H L 60 |
|---|---|---|---|---|
| CALCIOL-CROTONAT | 57 | 83 | 63 | 93 |
| CALCIOL-CAPROYLAT CALCIOL - C 11:1 | 39 | 88 | 71 | 35 |
| CALCIOL-all tr.-RET. | 56 | 91 | 56 | 88 |
| | 64 | 36 | 85 | 13 |

ST-4 Linfoma convoluto T — K 562 Eritroleucemia
RAJI Leucemia linfoide B — H L 60 Leucemia mieloide

e) Konzentrat-Mischung mit $D_2$- und $D_3$-Komponenten
Antitumorale Wirkung an humanen Zell-Linien Abtötung in %

| ZELL-LINIEN KONZENTRATION | ST-4 | H L 60 | K 562 |
|---|---|---|---|
| 1 : 10'000'000 | 100 | 100 | 100 |
| 1 : 100'000'000 | 73 | 68 | 70 |
| 1 : 1'000'000'000 | 47 | 6 | 54 |

*Humane Tumorzell-linien*:
ST-4 Linfoma convoluto T
HL-60 Leucemia mieloide
K 562 Eritroleucemia

*KONZENTRATIONEN*:
bezogen auf den Gesamtgehalt an Calciol- und Cholecalciolestern

Prüfungen ausgeführt am Ospedale maggiore San Giovanni Battista, LE MOLINETTE, Torino. Leitung: Prof.Dott. Giorgio RIVARA.

Weitere *Prüfungen mit humanen Tumorzell-Linien*

Das BATTELLE INSTITUT, Frankfurt, (Dr. Matthias GIESE) hat die zytotoxische Wirkung bei verschiedenen soliden, eher langsam wachsenden Tumorarten geprüft. Eingesetzt wurden 2 %-Konzentrate (gewichtsmässig) von:

A CALCIOL-CHOLECALCIOL-ESTER-MISCHUNG
B CALCIOL-all trans-RETINAT
C DL-$\alpha$-TOCOPHERYLESTER-MISCHUNG

mit folgenden humanen Tumorzell-Linien (DKFZ, Heidelberg, BRD):

1 EJ 28 : Blasen-Karzinom
2 LX-1 : LungenKarzinom

Als Kontroll-Substanzen wurden biologische "response modifiers" (BRM) verwandt:

a rhu Interferon-Gamma (Biozol, BRD)
b rhu Tumor-Nekrose Faktor Alpha (Biozol, BRD)

Ergebnis-Ausweis mittels Verdünnungsreihen und in der Kurzform der $IC_{50}$. (= Inhibitory Concentration; bzw. LC = Lethal Concentration) Inkubierung der Zellen über 24 h, bzw. 72 h bei 37 °C; Vitalitäts-Färbetest nach 3, bzw. 14 Tagen. Testbereich $1{:}10^5$ bis $1{:}10^9$.

Es konnten kieinere Unterschiede zwischen den Zell-Linien, wie auch zwischen den drei Wirkstoff-Konzentraten festgestellt werden. Die $LC_{50}$ lag im Verdünnungsbereich $1{:}10^6$ bis $1{:}10^7$.

Der Hauptbefund der Tests lautet: ***es liegt eine zytotoxische und keine zytostatische Wirkung vor.***

Der stärkste Zytotoxizitäts-Effekt liess sich für die CALCIOL/CHOLECALCIOLESTER-MISCHUNG ermitteln. Die Kontroll-Substanzen BRM zeigten keine messbaren Wirkungen auf die 2 geprüften Zell-Linien.

Die nachstehend als Beilage 1/4 Abbildung 1 und 2 wiedergegebenen graphischen Darstellungen fassen die Ergebnisse für die drei Konzentrate A, B und C zusammen. Vgl. die techn. Beilage Abbildungen 1 + 2.

*Prüfungen beim National Cancer Institute, Bethesda*

Im breit ausgebauten "in-vitro assay" des NCI werden gegenwärtig standardmässig 60 verschiedene humane Tumorarten als Zell-Linien eingesetzt, gruppiert in die *8 Panels* (statistischen Klassen):

Leukämie
Lungentumore, nicht-kleinzellig
Lungentumore, kleinzellig
Dickdarmtumore
ZNS-Tumore
Melanome
Ovarienkarzinome
Nierenkarzinome

Als Messgrössen werden ausgewiesen

PG (Percentage Growth, Wachstumsraten in %)
Dose-Response-Curves (Dosis-Wirkungs-Beziehung)
Mean Graphs (Mittelwert-Graphen)

*Percentage Growth* (Prozent-Wachstum) ist eine optische Dichtemessung mithilfe des Sulforhodamin B Färbe- und Vitalitätstests; Exposition der Zellen 48 h, in 5 verschiedenen Konzentrationen.

*Dose–Response–Curves*: Konzentrations-Abhängigkeit der Antitumorwirkung, dargestellt in Kurvenform als Dosis-Wirkung-Beziehung.

Die PG-Werte werden gegen $\log_{10}$ der entsprechenden Konzentration jeder einzelnen Zell-Linie aufgetragen. Ausgewiesen werden die PG-Werte für +50, 0, und -50. An den Schnittpunkten lassen sich die Werte für $G^{150}$, TG1 und $LC_{50}$ ablesen. Die ganze Matrix zeigt interpolierte Response Parameter für die Sensibilität der einzelnen Zell-Linie gegenüber der Prüfsubstanz, bzw. dem Prüfkonzentrat.

*Mean Graphs*: Mittelwert-Graphen, abgeleitet von den IC-Werten. Dargestellt werden das arithmetische Mittel des Logarithmus der IC-Werte für alle Zell-Linien-Messwerte für eine gegebene Prüfsubstanz, bzw. ein gegebenes Prüfsubstanzkonzentrat, sowie die Abweichungen davon als Mass der relativen Sensitivität der einzelnen Zell-Linien. Die Abweichungen zeigen, ob die Empfindlichkeit der einzelnen Zell-Linien grösser oder kleiner als der Durchschnitt ist. Es entsteht ein "Fingerprint Muster".

Die in den Beilagen 2/4 bis 4/4 gemachten Angaben enthalten anhand der Auswertungsprotokolle des National Cancer Institute, Bethesda, die zusammenfassenden Ergebnisse in graphischer Darstellung für:

CALCIOL-10-UNDECENOAT-KONZENTRAT
CALCIOL-all trans-RETINAT-KONZENTRAT und für
CALCIOL-LINOLENAT-KONZENTRAT

Vgl. die technische Beilage. Abbildungen 3 - 27.

Als *Hauptergebnis resultiert*, wie bei den Prüfungen am Battelle Institut, dass nicht nur *ein Antiproliferationseffekt, d.h. eine Wachstumshemmung* vorliegt, sondern *eine echte* Zytotoxizität, insbesondere auch bei jenen Zell-Linien, welche eine kurze Verdoppelungszeit aufweisen und deshalb vergleichsweise gegenüber langsamer wachsenden Linien als überempfindlich erscheinen.

Vgl. im übrigen: Michael R. Boyd: "Status of the NCI Preclinical Antitumor Drug Discovery Screen", PPO updates, Vol. 3, Oct. 1989, Number 10.

Anne Monks et al.: "Feasibility of a high-flux Anticancer Drug Screen using a diverse Panel of cultured human Tumor Cell Lines". Journal of the National Cancer Institute, Vol. 83, No. 11, June 5, 1991.

Übersicht der graphischen Darstellungen:

1/4 Abbildungen 1 und 2 : BATTELLE INSTITUT, FRANKFURT
2/4 bis 4/4 Abbildungen 1 bis 27 : NATIONAL CANCER INSTITUTE, BETHESDA

Abb. 1 - 9 : Calciol-10-Undecenoat (C 11:1-$D_2$)
Abb. 10 - 18 : Calciol-all trans-Retinat ($D_2$-Retinylester)

Abb. 19 - 27 : Calciol-Linolenat (C 18:3-$D_2$)

*Nachweis der Membran–Penetration an der Tumorzelle*

Am Lichtmikroskop wie auch mithilfe der "Laser scanning microscopy" lässt sich aufzeigen, dass wenige Stunden nach der Inkubation (Beispiel Py6 - Zellen der 3T3-Maus; dünn ausgesät, mittlere Verdünnung der Wirkstoff-Konzentrate) sich ein Kranz von Vakuolen um den Zellkern herum ausbildet.

Der analytische Nachweis, dass diese Vakuolen die Sterolester-Wirksubstanzen enthalten, kann sehr deutlich dadurch erbracht werden, dass man den gereinigten inkubierten Tumorzellen das Zellplasma entnimmt, es zentrifugiert und mit einer 0,05 %-Lösung von Uvitex® CF conc. (CIBA-GEIGY) in Aceton/Wasser (85:15) oder von Uvitex® EBF oder von Tinopal® GS versetzt.

Die eingesetzten erfindungsgetreuen Sterolester löschen die durch den Marker Uvitex® CF conc. hervorgerufene Fluoreszenz im langwelligen UV-Lichtbereich aus; auf der Dünnschicht-Platte entsteht eine blaue Färbung. UV-Scanning bei 366 nm. Microdialyse mithilfe von mizellarer Kapillar-Zonen-Elektrophorese, (Beckman Instruments, P/ACE System 2000, Version 1.50).

**Patentansprüche**

1. Spontan dispergierbares Konzentrat, welches mit Wasser versetzt Mikroemulsionen von hervorragender Phasenstabilität und von überaus deutlich erhöhtem Permeationsvermögen ergibt, dadurch gekennzeichnet, dass es als antitumorale Komponente
   0,001 bis 15 Gewichts-% einer Verbindung der Formeln (I) bis (IV) :

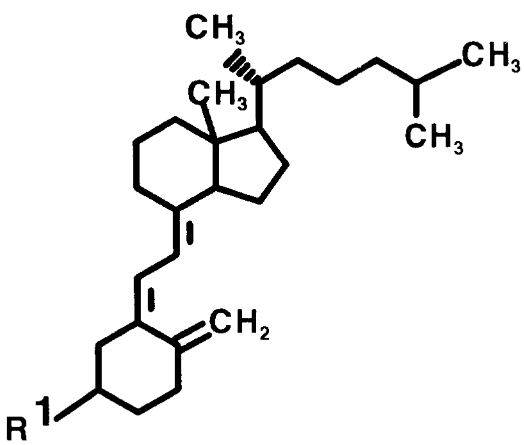

(I)

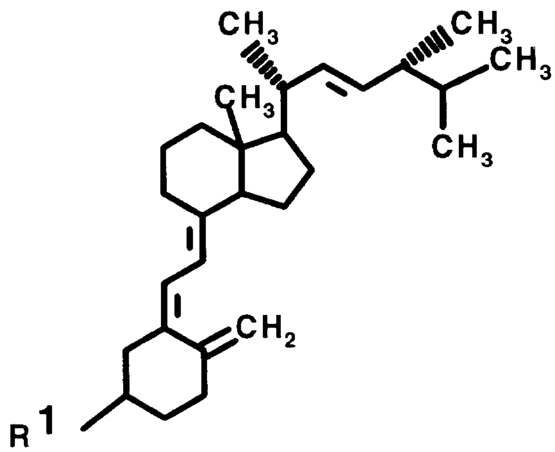

(II)

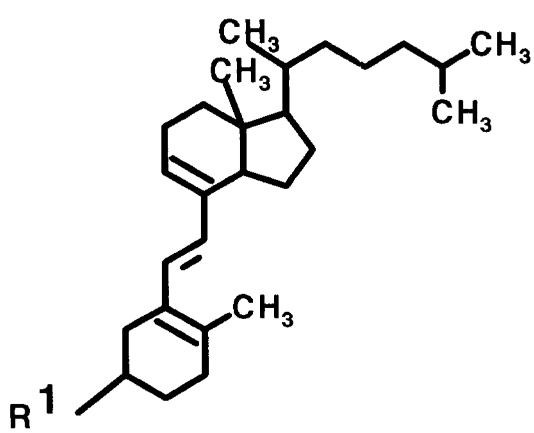

(III)

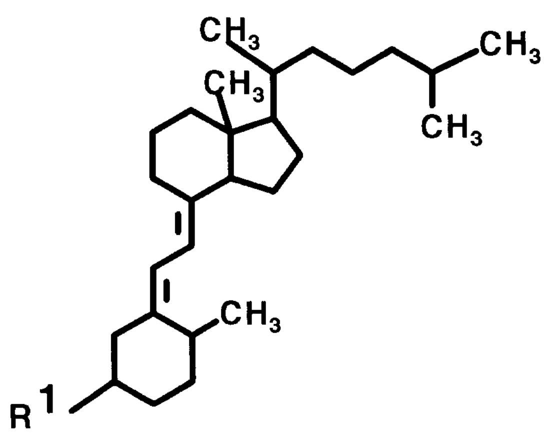

(IV)

wobei das Radikal $R^1$ in den Formeln (I) bis (IV) eine $C_1$ bis $C_{32}$-Alkylcarboxyl-, bzw. eine $C_2$- bis $C_{32}$-Alkenylcarboxyl- oder Alkapolyencarboxylkette oder eine Gruppe der Formeln (V) oder (VI) :

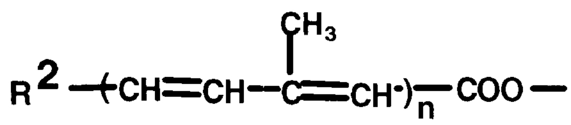

(V)

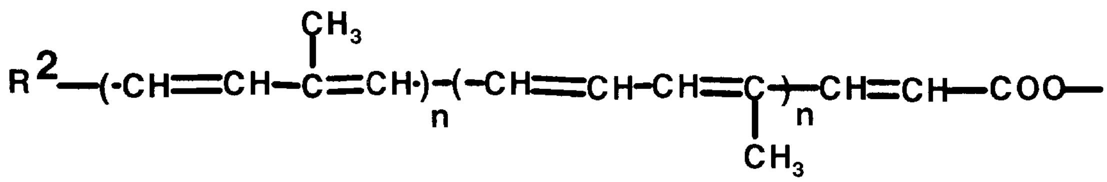

(VI)

darstellt, worin n die Zahlen 1, 2, 3, 4 oder 5 bezeichnet und $R^2$ für ein Radikal der Formeln

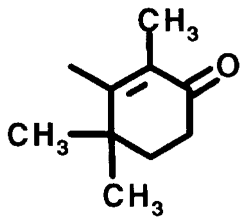
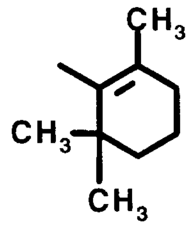
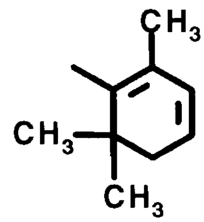
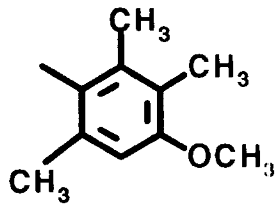
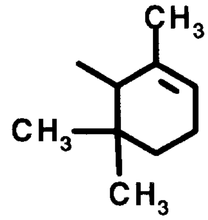
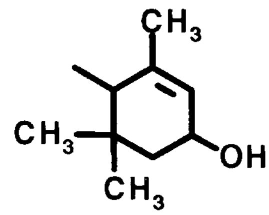
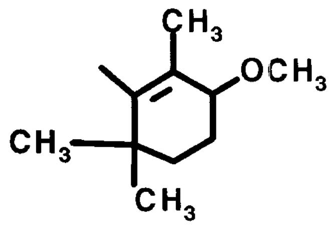
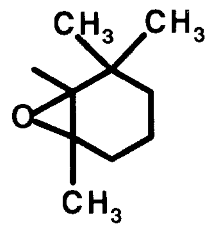
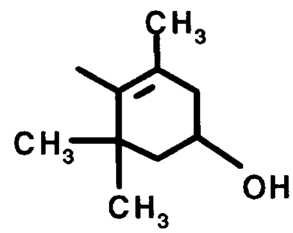
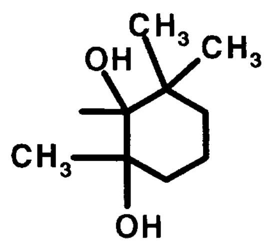
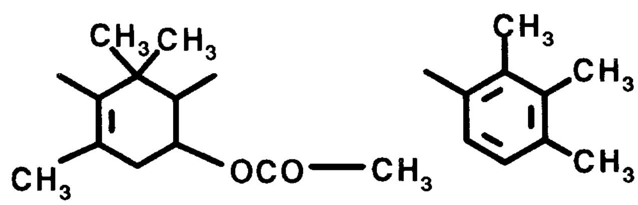

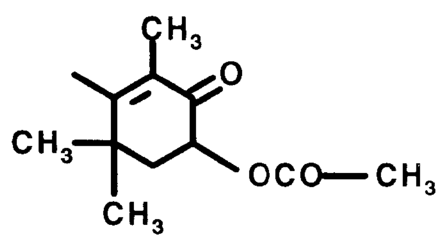 oder 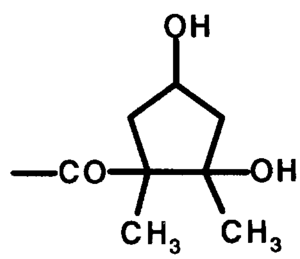

steht, bzw. einer Kombination solcher Wirkstoffe, sowie
0 bis 40 Gewichts-% eines als Hydrotrop, bzw. Co-Emulgator dienenden, pharmaverträglichen Lösungsmittels oder Lösungsmittelgemisches
0,001 bis 90 Gewichts-% eines pharmaverträglichen Tensides oder Tensidgemisches
0 bis 10 Gewichts-% eines Vitamins oder Provitamins
0 bis 10 Gewichts-% einer freien Säure, pharmaübliche Trägerstoffe und/oder Verdünnungsmittel enthält.

2. Spontan dispergierbares Konzentrat gemäss Anspruch 1, dadurch gekennzeichnet, dass es als Tensid ein nicht-ionogenes Tensid mit einem HLB-Wert zwischen 2 und 18, vorzugsweise zwischen 2 und 6 und/oder ein Phosphorsäureester-Tensid enthält.

3. Spontan dispergierbares Konzentrat gemäss Anspruch 2, dadurch gekennzeichnet, dass es
7,5 bis 15 Gewichts-% eines oder mehrerer Ester mit Vitamin-D-Verbindungen der Formeln (I) bis (IV)
0 bis 40 Gewichts-% Isopropylmyristat, Isopropylpalmitat oder Neutralöl
20 bis 45 Gewichts-% Nonylphenol-10-polyoxyäthylen-mono/dimethyl Phosphorsäureester und
20 bis 45 Gewichts-% tert. Octylphenylpolyoxyäthylenäther mit 9 bis 10 Oxyäthylengruppen, wasserfrei, enthält.

4. Spontan dispergierhares Konzentrat gemäss Anspruch 2, dadurch gekennzeichnet, dass es
7,5 bis 15 Gewichts-% eines oder mehrerer Ester mit Vitamin-D-Verbindungen der Formeln (I) bis (IV)
0 bis 40 Gewichts-% Isopropylmyristat, Isopropylpalmitat oder Neutralöl
20 bis 45 Gewichts-% Tristyrylphenolpolyoxyäthylen-18-mono/dimethyl-Phosphorsäureester und
20 bis 45 Gewichts-% tert. Octylphenylpolyoxyäthylenäther mit 9 bis 10 Oxyäthylengruppen, wasserfrei, enthält.

5. Ester mit Vitamin-D-Verbindungen der allgemeinen Formeln (I) bis (IV), zur Verwendung als Arzneimittel mit antitumoraler Wirksamkeit :

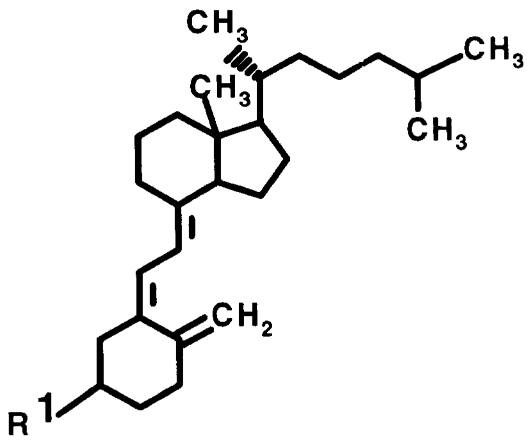

(I)

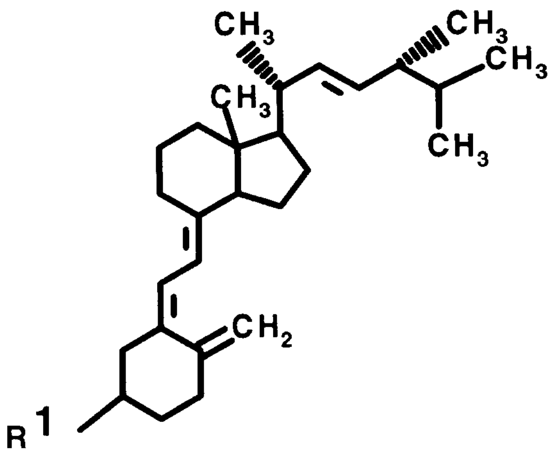

(II)

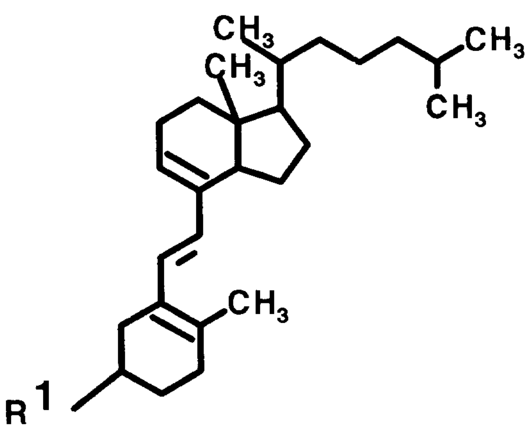

(III)

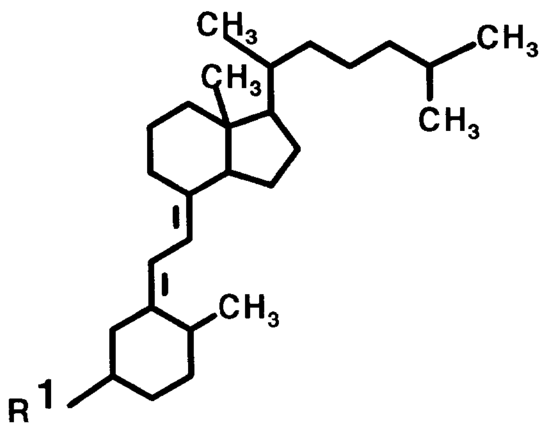

(IV)

wobei das Radikal $R^1$ in den Formeln (I) bis (IV) eine $C_1$ bis $C_{32}$-Alkylcarboxyl-, bzw. eine $C_2$- bis $C_{32}$-Alkenylcarboxyl- oder Alkapolyencarboxylkette oder eine Gruppe der Formeln (V) oder (VI) :

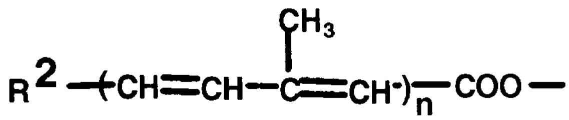

(V)

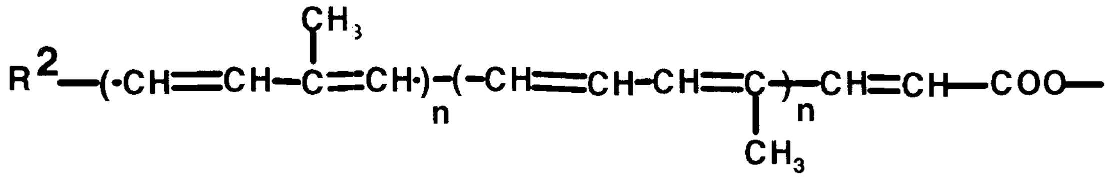

(VI)

darstellt, worin n die Zahlen 1, 2, 3, 4 oder 5 bezeichnet und $R^2$ für ein Radikal der Formeln
darstellt, worin n die Zahlen 1, 2, 3, 4 oder 5 bezeichnet und $R^2$ für ein Radikal der Formeln

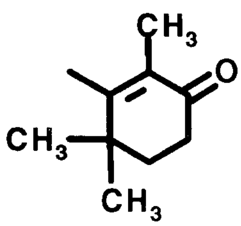
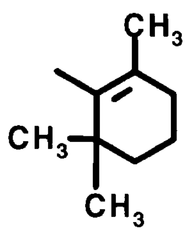
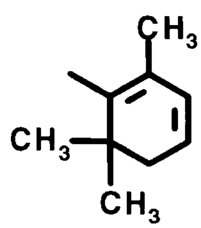

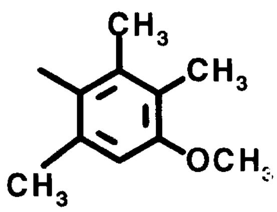
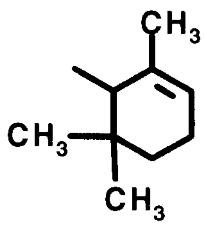
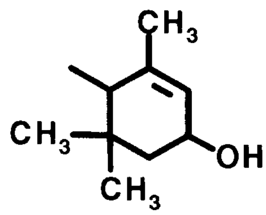

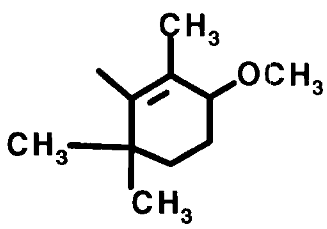
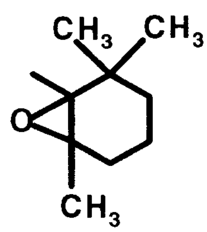
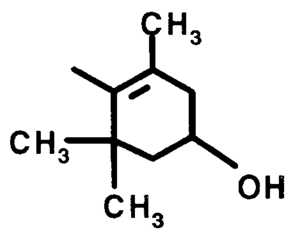

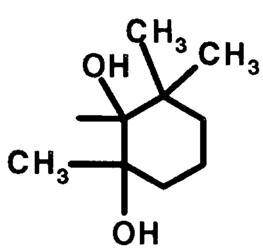
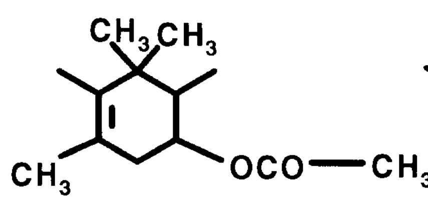
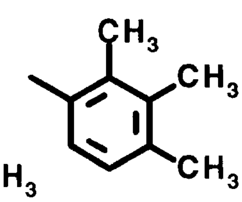

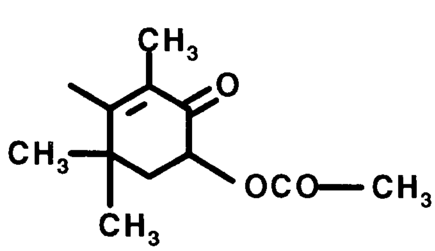 oder 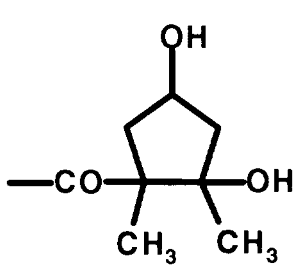

steht.

6. Ester mit Vitamin-D-Verbindungen gemäss Anspruch 5, dadurch gekennzeichnet, dass das Radikal $R^1$ in den Formeln (I) bis (IV) eine $C_4$- bis $C_{22}$-Alkylcarboxyl-, bzw. eine $C_4$- bis $C_{22}$-Alkenylcarboxyl- oder eine $C_4$- bis $C_{22}$-Alkapolyencarboxylgruppe bezeichnet oder für ein Radikal der Formeln (VII), (VIII) oder (IX) :

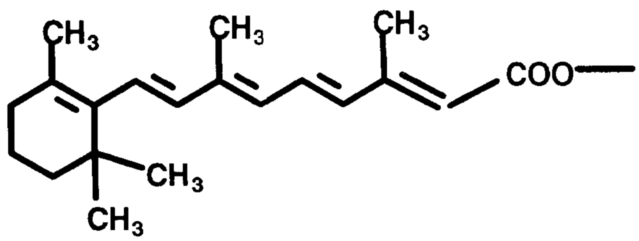

(VII)

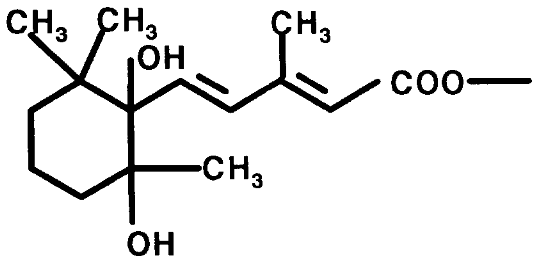

(VIII)

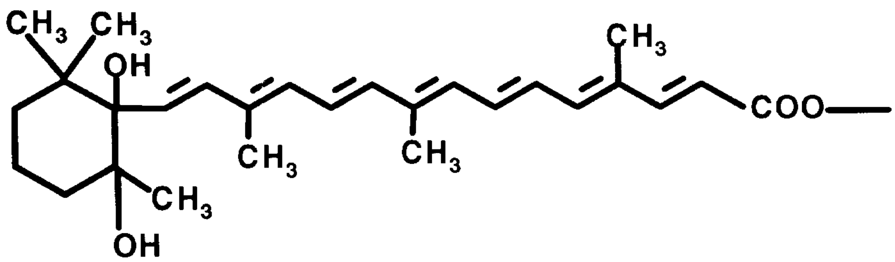

(IX)

steht.

7. Ester mit Vitamin-D-Verbindungen der allgemeinen Formeln (I) bis (IV)

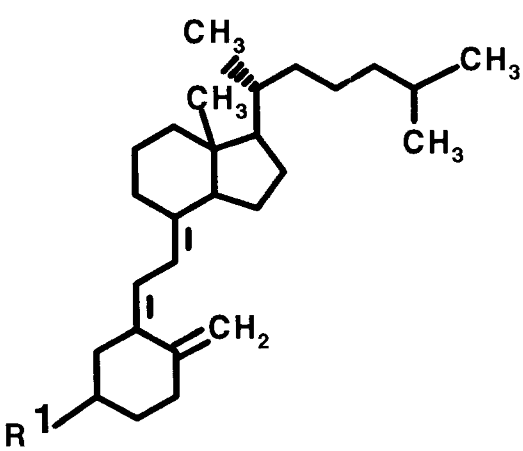

(I)

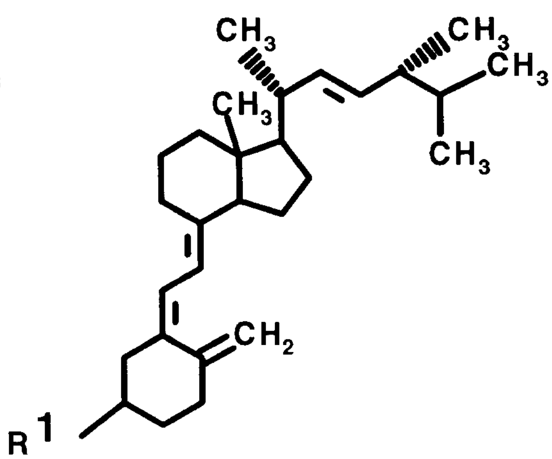

(II)

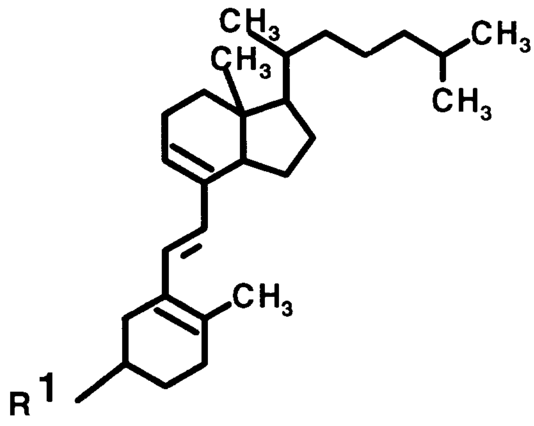

(III)

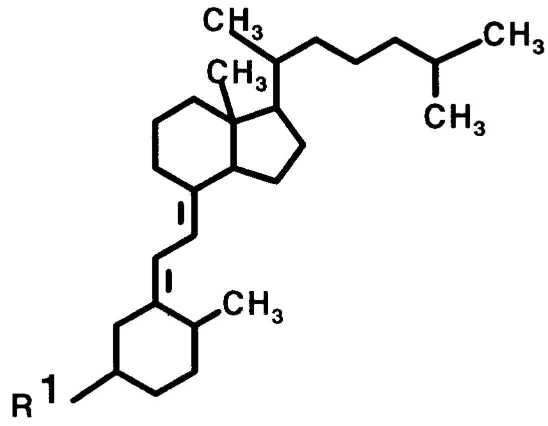

(IV)

wobei das Radikal $R^1$ in den Formeln (I) bis (IV) eine $C_1$ bis $C_{32}$-Alkylcarboxyl-, bzw. eine $C_2$- bis $C_{32}$-Alkenylcarboxyl- oder Alkapolyencarboxylkette oder eine Gruppe der Formeln (V) oder (VI) :

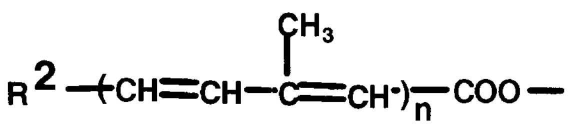

(V)

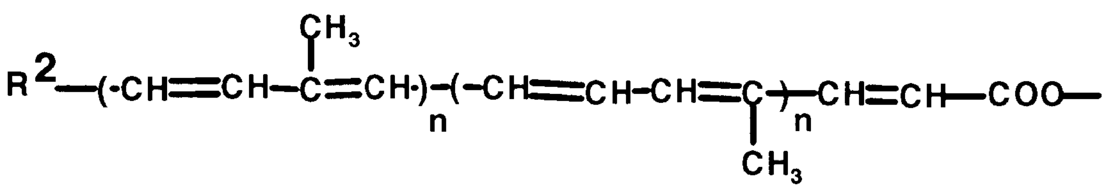

(VI)

darstellt, worin n die Zahlen 1, 2, 3, 4 oder 5 bezeichnet und $R^2$ für ein Radikal der Formeln

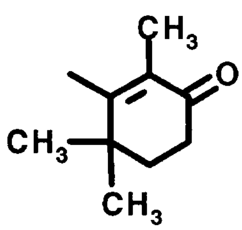

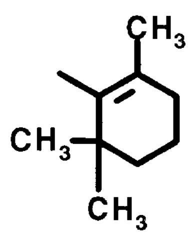

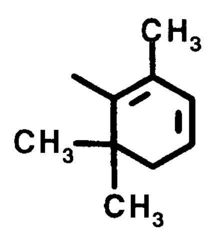

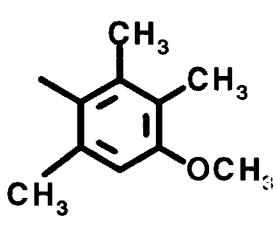

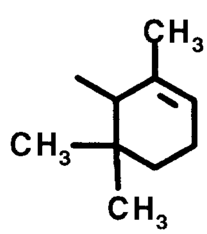

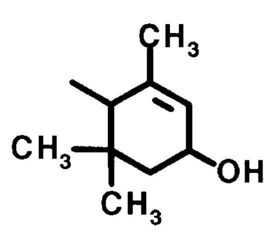

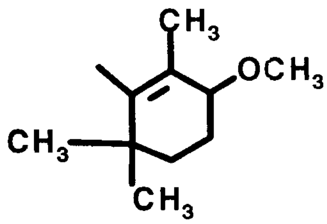

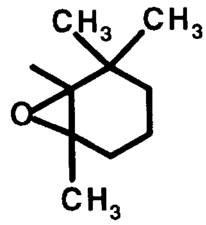

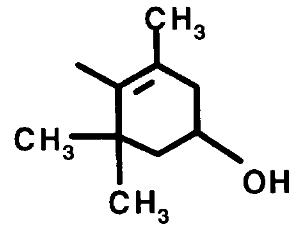

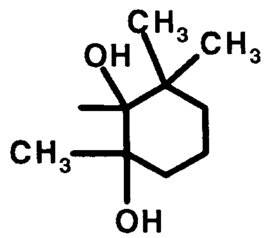

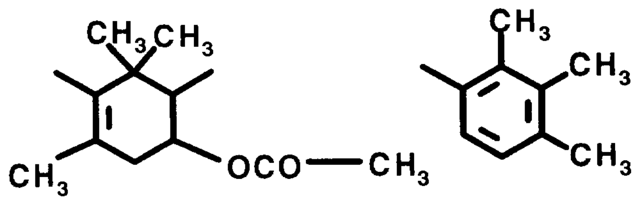

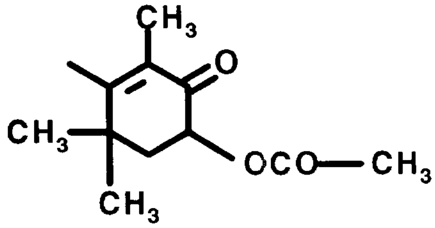

oder

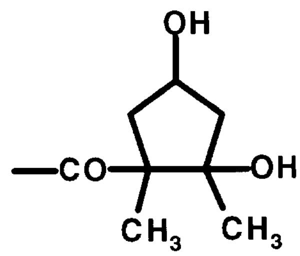

steht, mit der Massgabe, dass die Verbindungen Ergocalciferylacetat, -laurat, -myristat, -palmitat, -stearat, -oleat, -linolat, und linolenat, sowie die Cholecalciferylester mit Eicosa-5,8,11,14,17-pentaensäure und mit Docosa-4,7,10, 13,16,19-hexaensäure ausgenommen sind.

**8.** Ester mit Vitamin-D-Verbindungen gemäss Anspruch 7, wobei in den Formeln (I) bis (IV) das Radikal $R^1$ eine $C_4$- bis $C_{22}$-Alkylcarboxyl-, bzw. eine $C_4$- bis $C_{22}$-Alkenylcarboxyl- oder eine $C_4$- bis $C_{22}$-Alkapolyencarboxylgruppe bezeichnet oder für ein Radikal der Formeln (VII), (VIII) oder (IX) steht

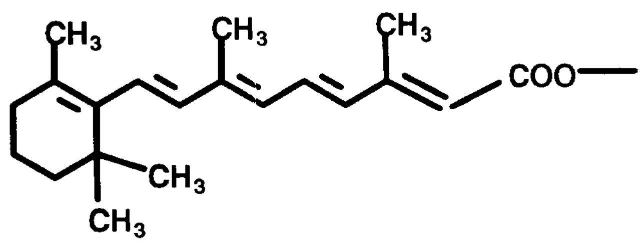

(VII)

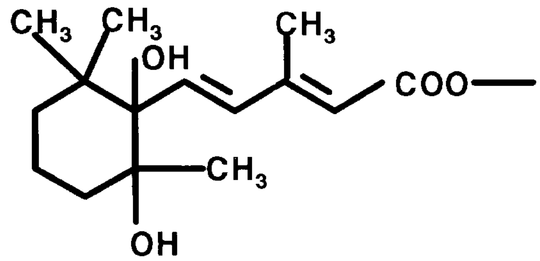

(VIII)

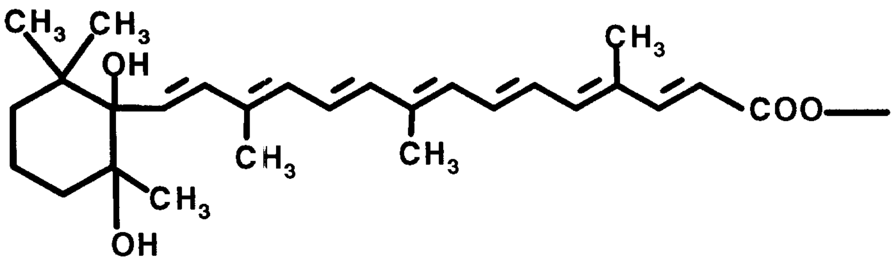

(IX)

mit der Massgabe, dass die Verbindungen Ergocalciferyllaurat, -myristat, palmitat, -stearat, -oleat, -linolat, und linolenat, sowie die Cholecalciferylester mit Eicosa-5,8,11,14,17-pentaensäure und mit Docosa-4,7,10,13,16,19-hexaensäure ausgenommen sind.

**9.** Das Cholecalciferyl-10-Undecenoat, das Cholecalciferyl-all trans-Retinat oder das Ergocalciferyl-all trans-Retinat gemäss einem der Ansprüche 5 oder 7.

**10.** Verfahren zur Herstellung von Verbindungen der Formeln (I) bis (IV) gemäss Anspruch 7, dadurch gekennzeichnet, dass man eine Verbindung der Formeln (X) oder (XI)

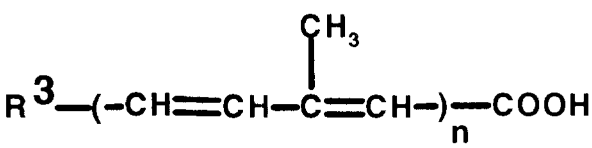

(X)

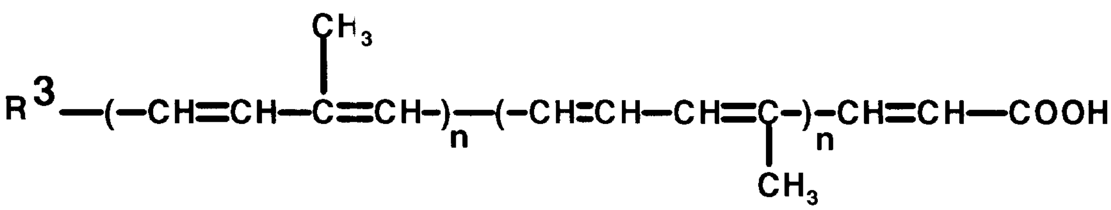

(XI)

in der n die Zahlen 1, 2, 3, 4 oder 5 bezeichnet und $R^3$ für ein Radikal der Formeln

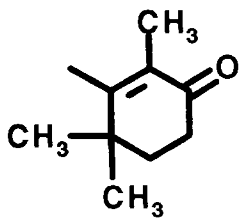
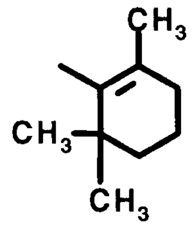
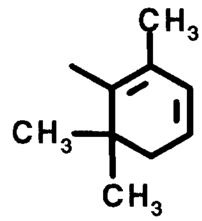

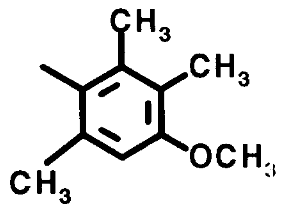
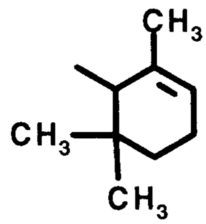
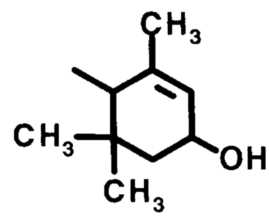

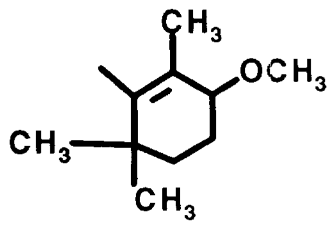
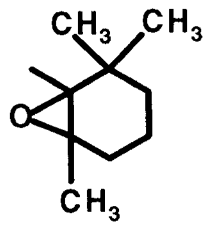
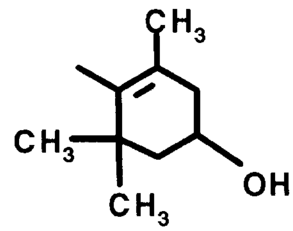

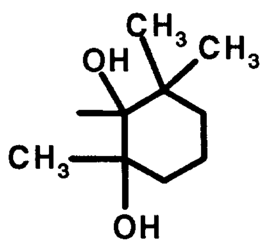
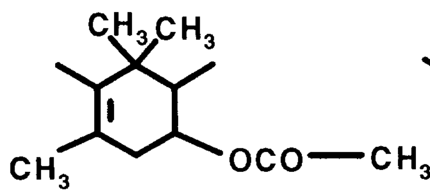
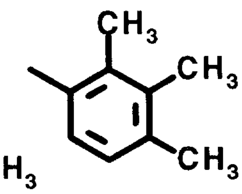

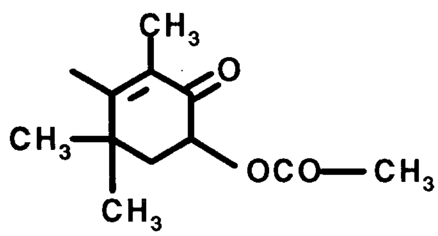 oder 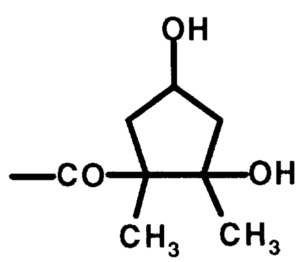

fungiert, mit N,N'-Carbonyldiimidazol bei 25 bis 70 °C unter Zusatz einer katalytischen Menge eine Alkoholates in einem indifferenten Lösungsmittel umsetzt und anschliessend eine Alkoholyse der gebildeten Imidazolide mit einer Vitamin-D-Verbindung durchführt.

**11.** Verfahren zur Herstellung von Verbindungen der Formeln (I) bis (IV) gemäss Anspruch 7, dadurch gekennzeichnet, dass man eine Verbindung der Formeln (X), (XI oder (XII) :

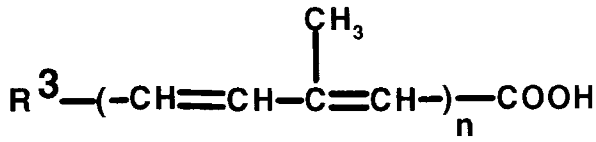

(X)

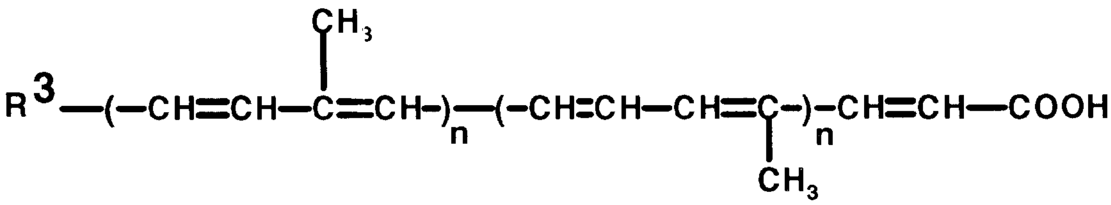

(XI)

$R^4$—COOH (XII)

worin $R^4$ eine $C_1$- bis $C_{32}$-Alkyl- oder eine $C_2$- bis $C_{32}$-Alkenyl-, bzw. $C_2$- bis $C_{32}$-Alkapolyengruppe darstellt, mit einem Chlorierungsmittel, bzw. Bromierungsmittel und anschliessender Reaktion der entstandenen Chloride, bzw. Bromide mit einer Vitamin-D-Verbindung bei einer Temperatur von 40-120 °C in einem indifferenten Lösungsmittel und in Gegenwart eines Katalysatoren umsetzt.

**12.** Therapeutisches Systempräparat, welches 1 bis 95 Gewichts-% des spontan dispergierbaren Konzentrates gemäss einem der Ansprüche 1 bis 4 enthält und welches in Dosis-Einheitsform als Micropellets, Granulat, Dragées, Suppositorien, Ampullen oder als Kapseln vorliegt.

**13.** Therapeutisches Systempräparat gemäss Anspruch 12, welches 44 Teile Kernmaterial für die Granulat-, bzw. Pellet-Bildung, 25 Teile eines erfindungsgemässen Konzentrates und 31 Teile magensaftresistente und retardierende Beschichtuncg mit Hydroxylpropyl-Methylcellulose-Acetat-Succinat enthält.

**14.** Therapeutisches Systempräparat gemäss Anspruch 12, welches 64 Teile Kernmaterial für die Granulat-, bzw. Pellet-Bildung und 36 Teile eines erfindungsgemässen Konzentrates enthält und in pharmazeutisch geeignete Kapseln aus Hydroxylpropyl-Methylcellulose-Acetat-Succinat abgefüllt wird.

## Claims

**1.** A spontaneously dispersible concentrate which after dilution with water produces a microemulsion having excellent phase stability and markedly enhanced permeation capability, consisting of
0,001 to 15 % by weight of a compound according to the formulae (I) to (IV)

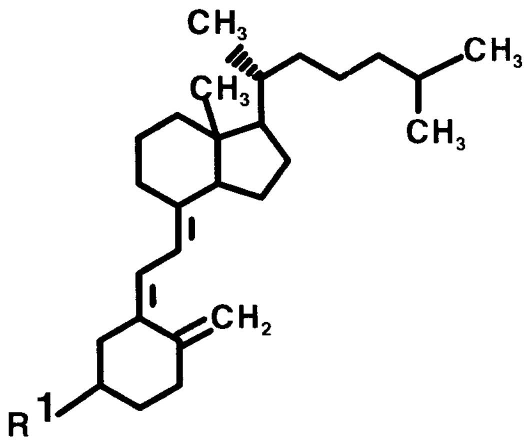

(I)

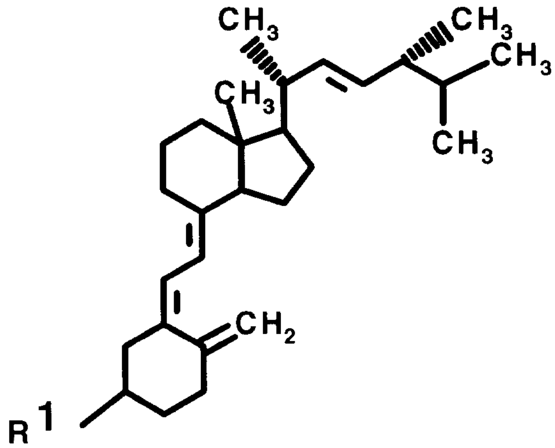

(II)

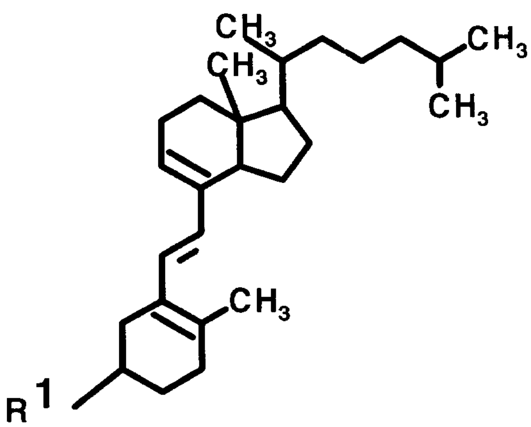

(III)

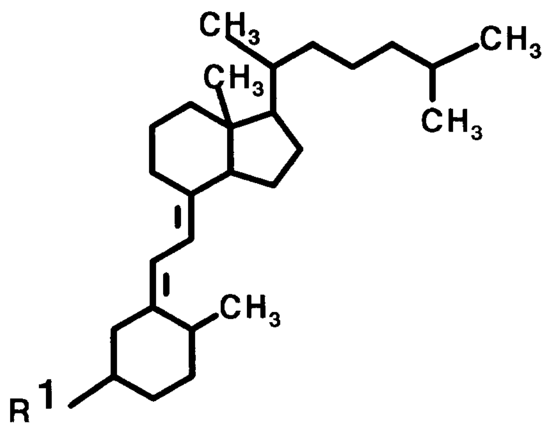

(IV)

whereby the radical $R^1$ in the formulae (I) to (IV) is a $C_1$ to $C_{32}$ alkyl carbonyloxy and a $C_2$ to $C_{32}$ alkenyl carbonyloxy or alkapolyene carbonyloxy chain, respectively, or a group according to the formulae (V) or (VI):

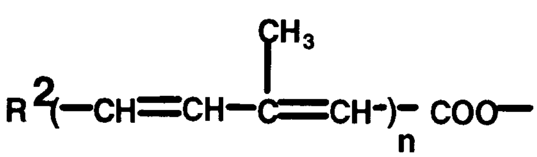

(V)

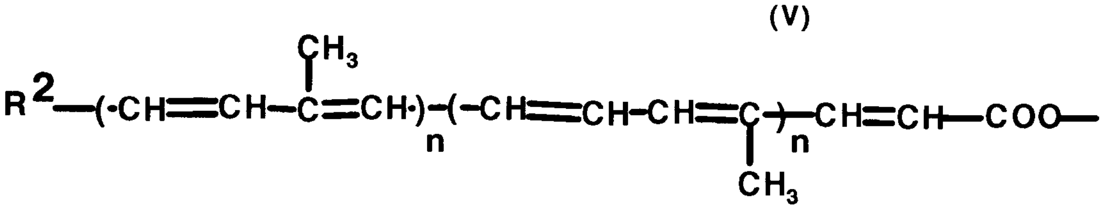

(VI)

in which the letter n designates the numbers 1, 2, 3, 4 or 5 and $R^2$ stands for one of the radicals according to the following formulae

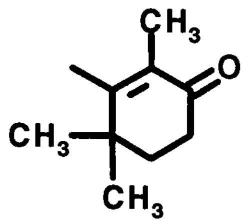 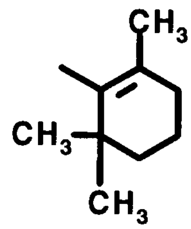 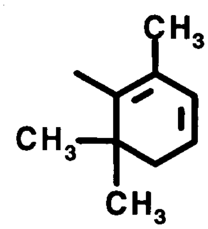

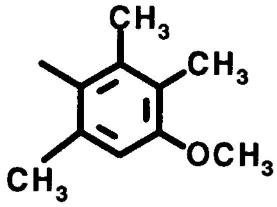 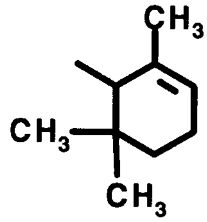 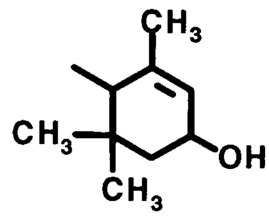

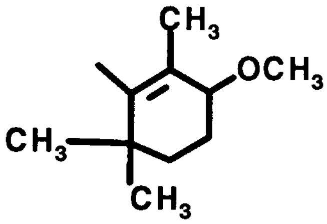 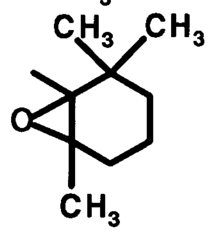 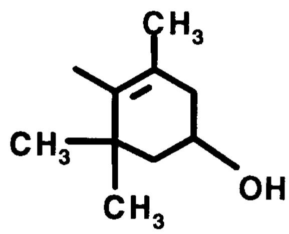

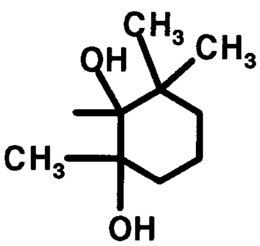 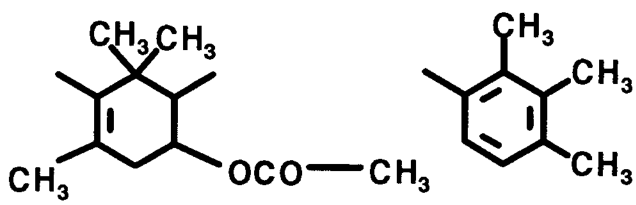

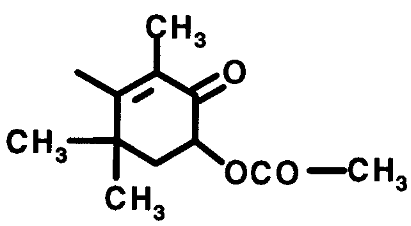 or 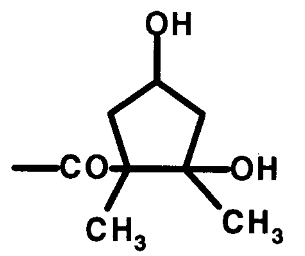

or a combination of such active principles, as well as
0 to 40 % by weight of a solvent or solvent mixture which is pharmaceutically acceptable and acts as a hydrotropic or coemulsifier,
0.001 to 90 % by weight of a pharmaceutically acceptable surfactant or surfactant mixture and, optionally,
up to 10 % by weight of a vitamin or provitamin
up to 10 % by weight of a free acid, pharmaceutical auxiliaries and/or solvents.

**2.** A spontaneously dispersible concentrate according to claim 1), containing as the surfactant a non-ionogenic tenside with a HLB-value between 2 and 18, preferably between 2 and 6 and/or a phosphoric acid ester tenside.

**3.** A spontaneously dispersible concentrate according to claim 2), consisting of
7,5 to 15 % by weight of one or several esters formed with vitamin-D compounds according to formulae (I) to (IV), as well as
0 to 40 % by weight of isopropylmyristate, isopropylpalmitate or neutral oil
20 to 45 % by weight of a 50:50 mixture of the two tensides with formulae

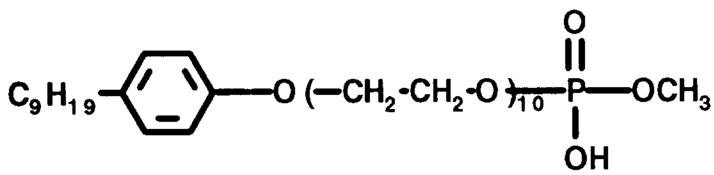

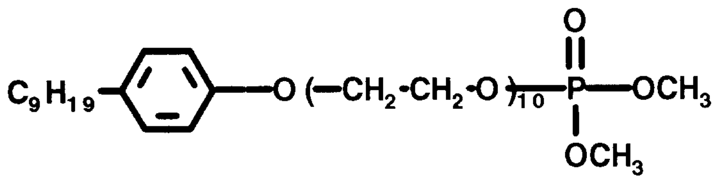

(Nonylphenol-10-polyoxyethylene-mono/dimethyl phosphoric acid ester)
and 20 to 45 % by weight of the waterfree compound tert. octylphenylpolyoxyethylene ether having 9-10 oxyethylene groups.

**4.** A spontaneously dispersible concentrate according to claim 2), consisting of
7,5 to 15 % by weight of one or several esters formed with vitamin-D compounds according to formulae (I) to (IV), as well as
0 to 40 % by weight of isopropylmyristate, isopropylpalmitate or neutral oil
20 to 45 % by weight of the tenside of the formula

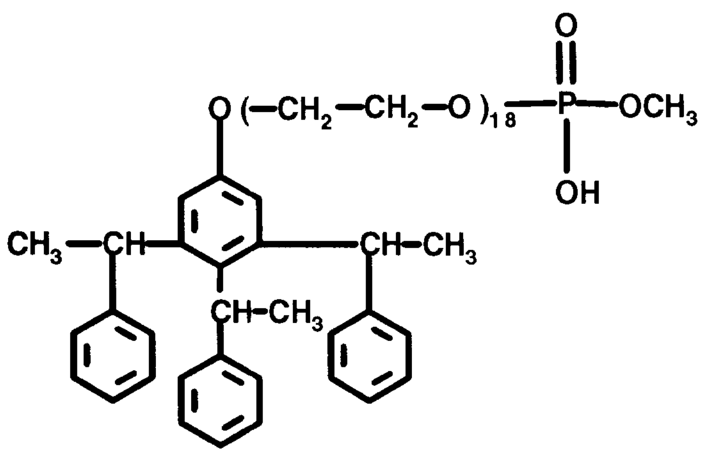

(Tristyrylpolyoxyethylene ether having 9 to 10 oxyethylene groups)
and 20 to 45 % by weight of the waterfree tert. octylphenylpolyoxyethylene ether having 9-10 oxyethylene groups.

**5.** Esters with vitamin-D compounds according to formulae (I) to (IV), for use as medicaments having antitumor efficacy :

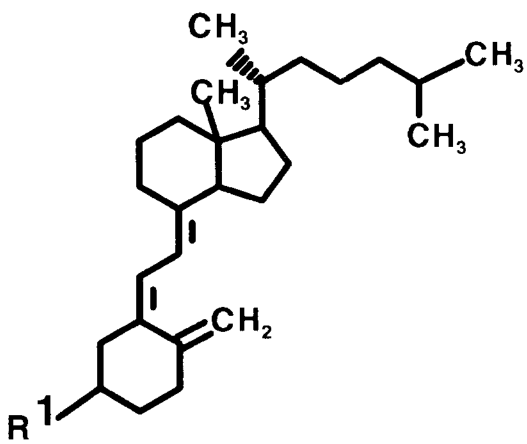

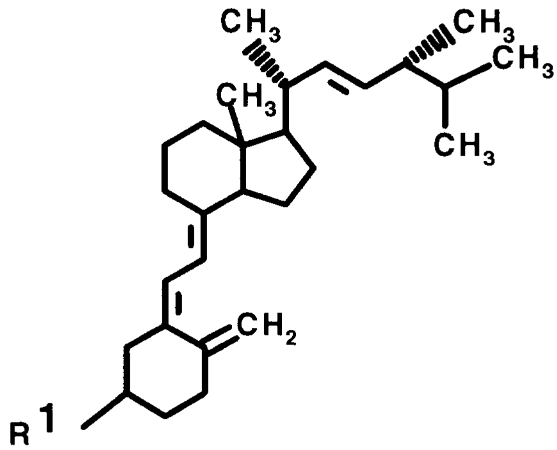

(I) (II)

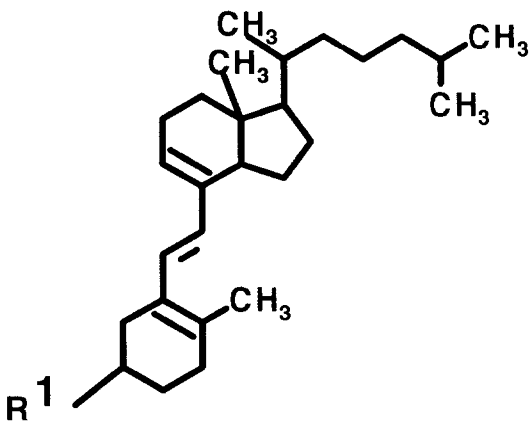

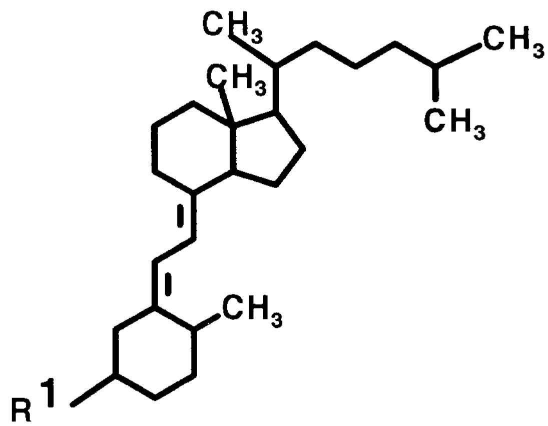

(III) (IV)

whereby the radical $R^1$ in the formulae (I) to (IV) is a $C_1$ to $C_{32}$ alkyl carbonyloxy and a $C_2$ to $C_{32}$ alkenyl carbonyloxy or alkapolyene carbonyloxy chain, respectively, or a group according to the formulae (V) or (VI) :

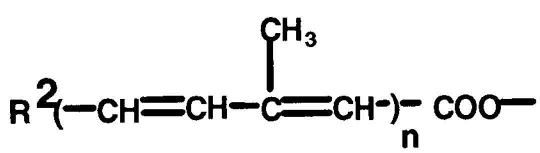

(V)

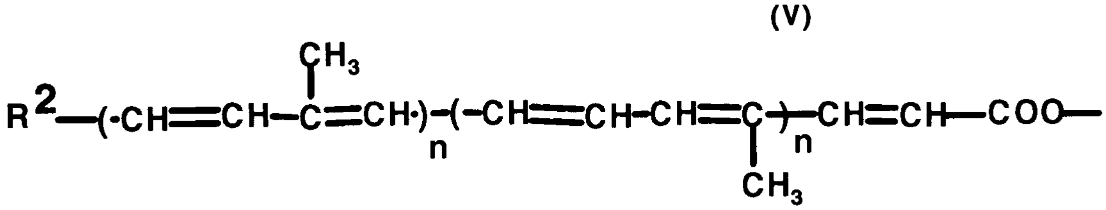

(VI)

in which the letter n designates the numbers 1, 2, 3, 4 or 5 and $R^2$ stands for one of the radicals according to the following formulae

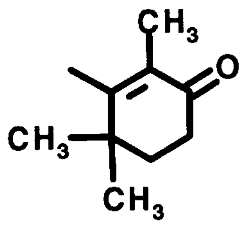
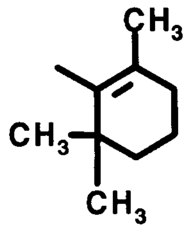
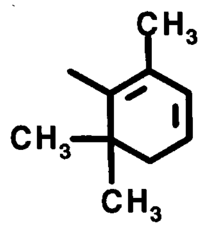
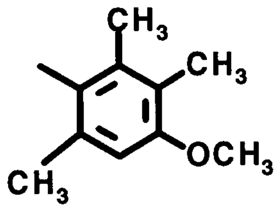
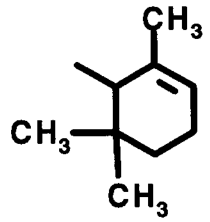
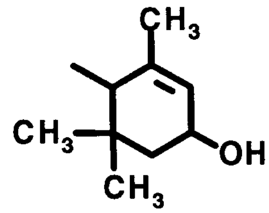
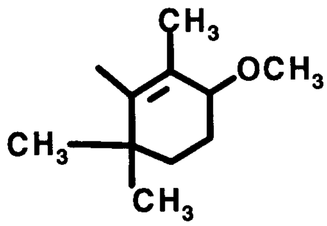
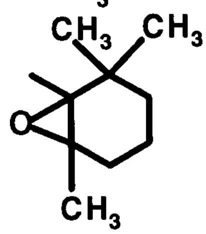
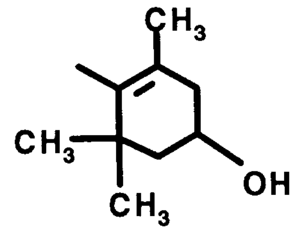
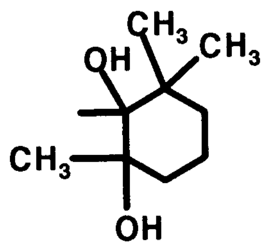
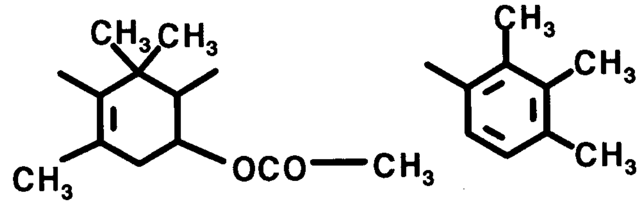
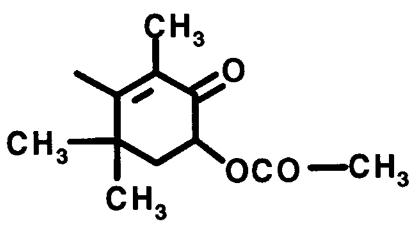

or

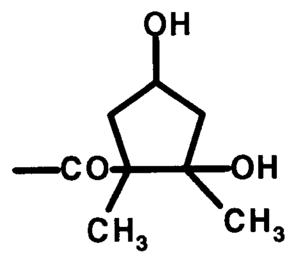

with the proviso that the compounds ergocalciferyl laurate, myristate, palmitate, stearate, oleate, linoleate and linolenate, as well as the cholecalciferyl esters formed with eicosa-5,8,11,14,17 pentaenoic acid and with docosa-4,7,10,13,16,19 hexaenoic acid respectively, are excluded.

**6.** A compound according to claim 5), whereby the radical $R^1$ in the formulae (I) to (IV) is a $C_4$ to $C_{22}$ alkyl carbonyloxy and a $C_4$ to $C_{22}$ alkenyl carbonyloxy or alkapolyene carbonyloxy chain, respectively, or a group according to the formulae (VII), (VIII) or (IX) :

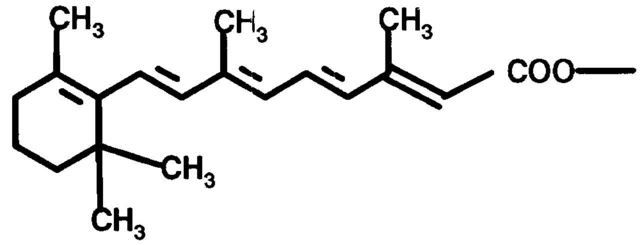

(VII)

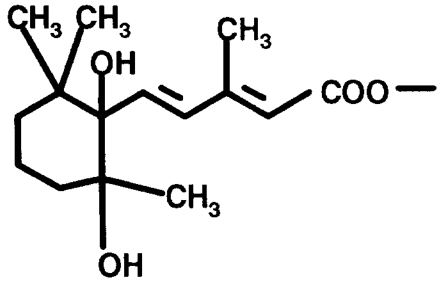

(VIII)

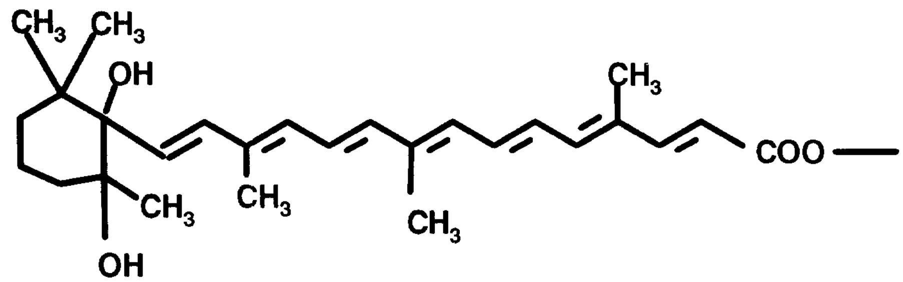

(IX).

7. Esters with vitamin-D compounds according to formulae (I) to (IV):

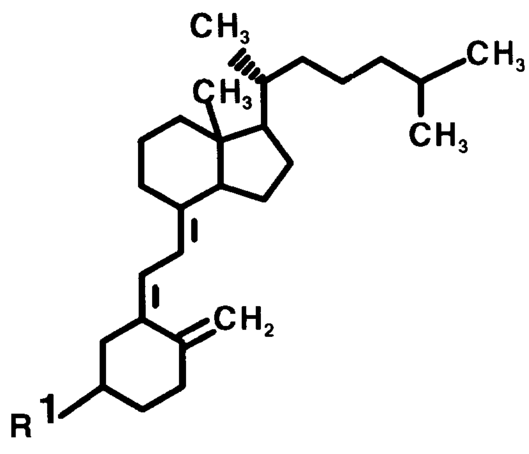

(I)

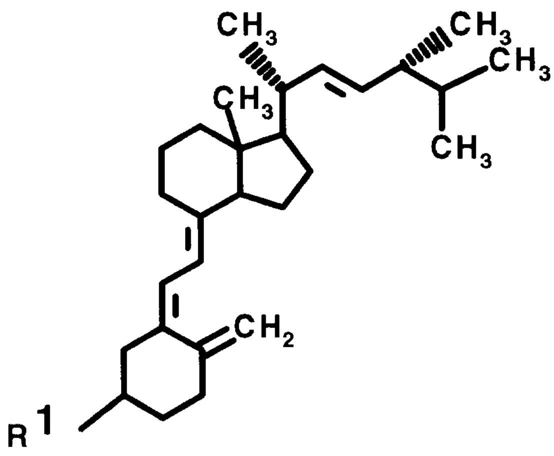

(II)

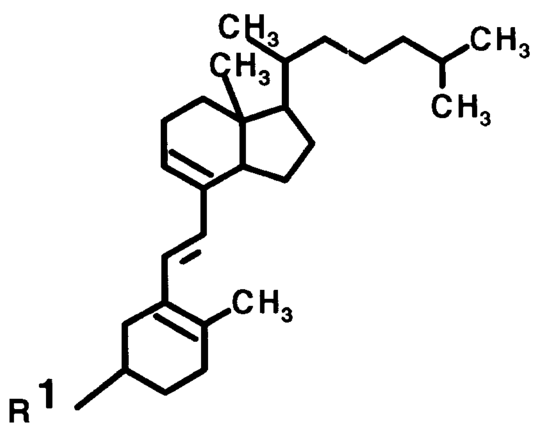

(III)

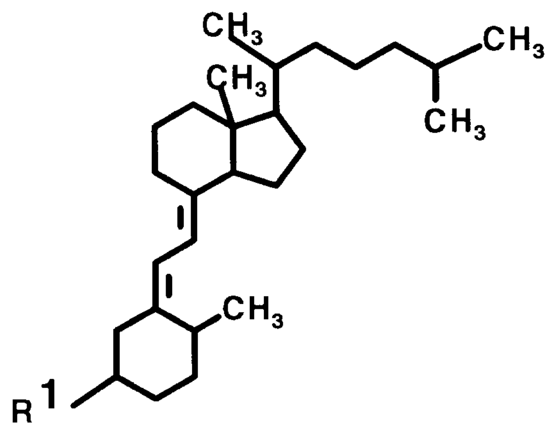

(IV)

whereby the radical $R^1$ in the formulae (I) to (IV) is a $C_1$ to $C_{32}$ alkyl carbonyloxy and a $C_2$ to $C_{32}$ alkenyl carbonyloxy or alkapolyene carbonyloxy chain, respectively, or a group according to the formulae (V) or (VI) :

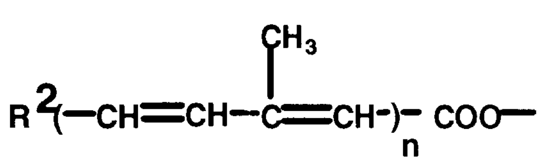

(V)

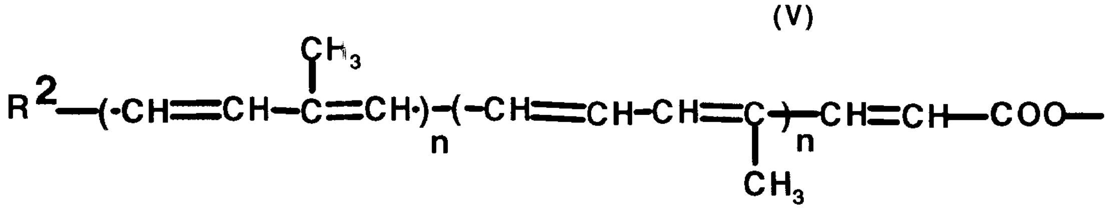

(VI)

in which the letter n designates the numbers 1, 2, 3, 4 or 5 and $R^2$ stands for one of the radicals according to the following formulae

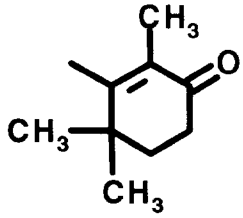
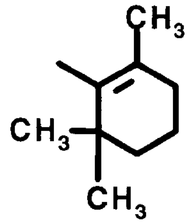
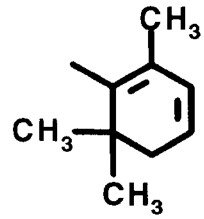
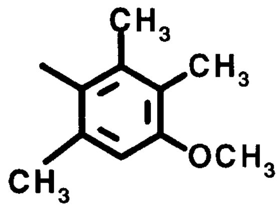
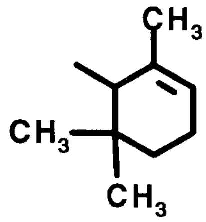
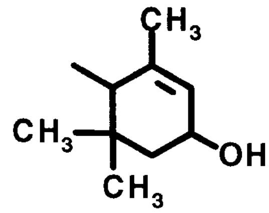
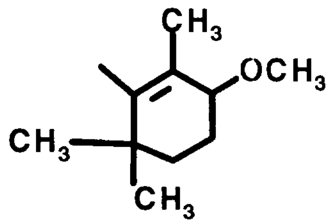
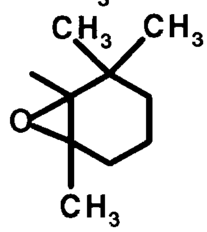
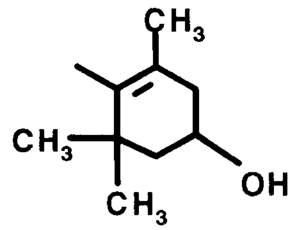
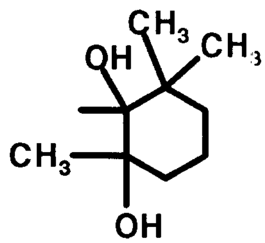
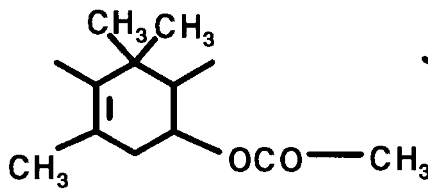
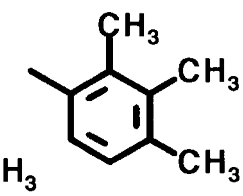
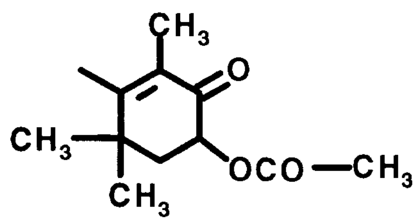

or

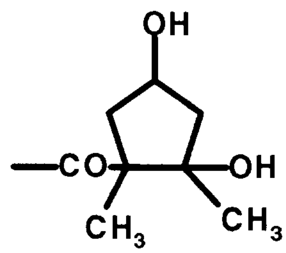

with the proviso that the compounds ergocalciferyl acetate, laurate, myristate, palmitate, stearate, oleate, linoleate and linolenate, as well as the cholecalciferyl esters formed with eicosa-5,8,11,14,17 pentaenoic acid and with docosa-4,7,10,13,16,19 hexaenoic acid respectively, are excluded.

**8.** Esters with Vitamin D compounds according to claim 7), whereby the radical $R^1$ in the formulae (I) to (IV) is a $C_4$ to $C_{22}$ alkyl carbonyloxy and a $C_4$ to $C_{22}$ alkenyl carbonyloxy or alkapolyene carbonyloxy chain, respectively, or a radical according to the formulae (VII), (VIII) or (IX) :

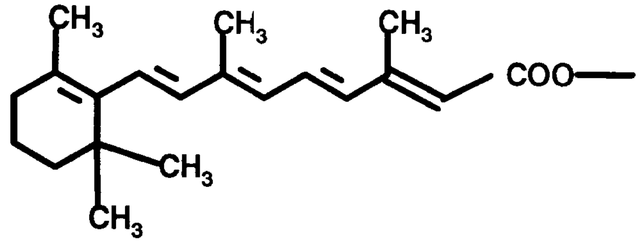

(VII)

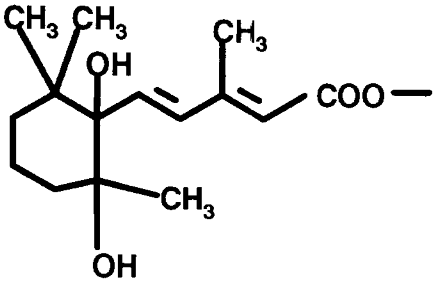

(VIII)

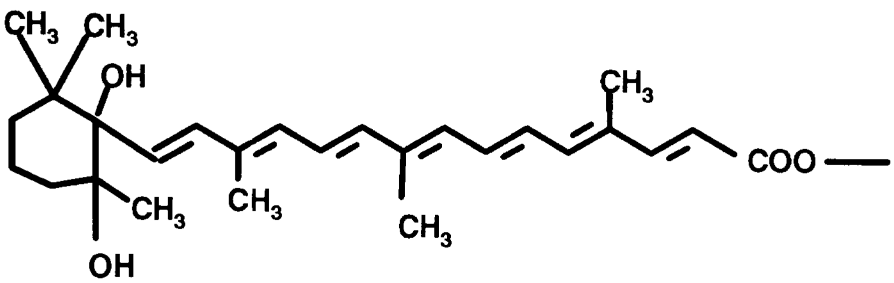

(IX)

with the proviso that the compounds ergocalciferyl laurate, myristate, palmitate, stearate, oleate, linoleate and linolenate, as well as the cholecalciferyl esters formed with eicosa-5,8,11,14,17 pentaenoic acid and with docosa-4,7,10,13,16,19 hexaenoic acid respectively, are excluded.

**9.** The compounds cholecalciferyl-10-undecenoate, cholecalciferyl-all trans retinate and ergocalciferyl-all trans retinate according to claim 7).

**10.** A process for the preparation of compounds corresponding to the formulae (I) to (IV) according to claim 6), consisting of reacting a compound of formulae (X) or (XI)

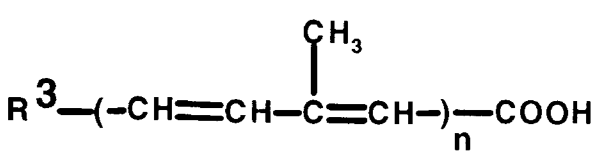

(X)

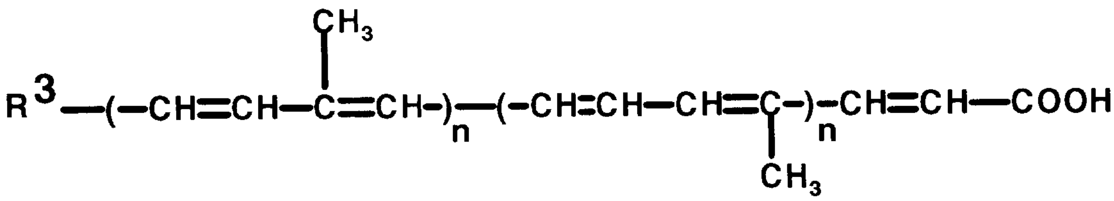

(XI)

in which the letter n designates the numbers 1, 2, 3, 4 or 5 and $R^2$ stands for one of the radicals according to the following formulae

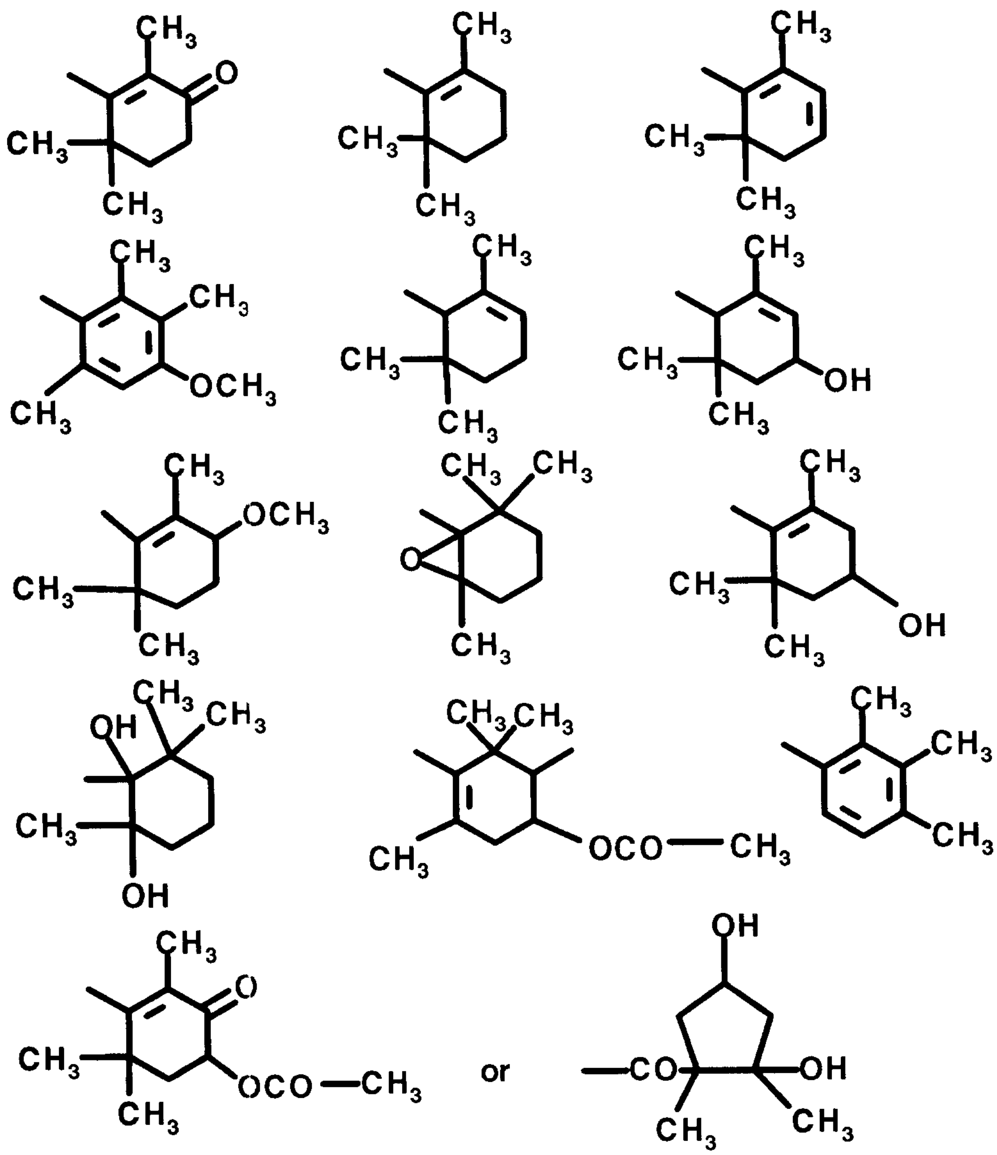

or

with N,N'-carbonyl-diimidazole at a temperature of 25 to 70 °C and in the presence of a catalytic amount of an alcoholate in an indifferent solvent and subsequently reacting the generated imidazole with a vitamin-D compound.

**11.** A process for preparing compounds of the formulae (I) to (IV) according to claim 7), consisting of reacting a compound of the formulae (X), (XI) and (XII) respectively

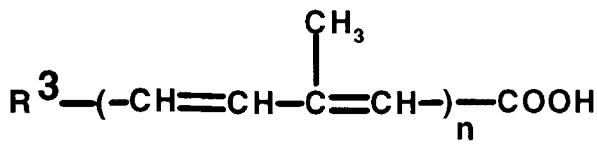

(X)

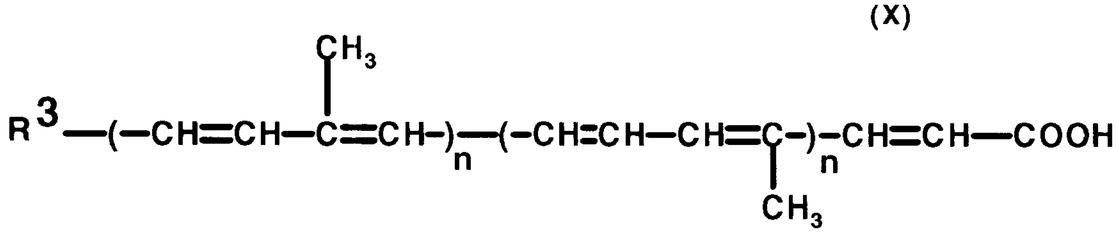

(XI)

$R^4$—COOH (XII)

in which $R^4$ means a $C_1$ to $C_{32}$ alkyl or a $C_2$ to $C_{32}$ alkenyl and a $C_2$ to $C_{32}$ alkapolyene group respectively, with a chlorination or bromination agent such as thionylchloride, oxalylchloride or oxalylbromide, followed by the reaction of the chloride or bromide formed with a vitamin-D compound at a temperature of between 40 and 120 °C in an indifferent solvent and in the presence of a catalyst.

**12.** A therapeutic system's preparation, which contains 1 to 95% by weight of the spontaneously dispersible concentrate as claimed in claims 1) to 4), and which is processed into an unit-type dosage form as micropellets, granules, dragées, suppositories, capsules or ampuls.

**13.** A therapeutic system's preparation according to claim 12), which contains 44 parts of core material for granules or pellets, 25 parts of the spontaneously dispersible concentrate and 31 parts of enteric and slow-release-coating on the basis of hydroxylpropyl-methylcellulose-acetatesuccinate.

**14.** A therapeutic system's preparation according to claim 12), which contains 64 parts of sore material for granules and pellets respectively, and 36 parts of the inventive concentrate according to claims 1) to 4), and which is filled into capsules made from hydroxypropyl-methyl-cellulose-acetate-succinate.

**Revendications**

**1.** Un concentré spontanément dispersible qui avec l'adjonction d'eau forme des microémulsions de stabilité de phase excellente et d'un pouvoir de pénétration fortement amélioré, contenant comme composé antitumoral :
0,001 à 15 % par poids soit d'un composé ester de vitamine-D correspondant aux formules (I) à (IV), soit d'une combinaison de tels composés:

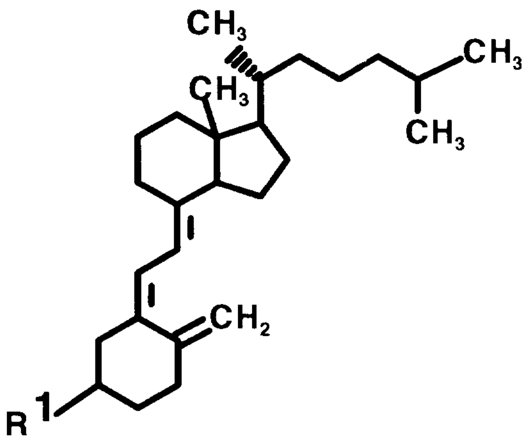

(I)

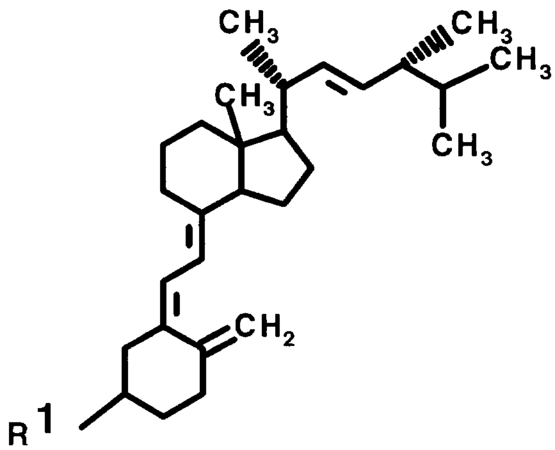

(II)

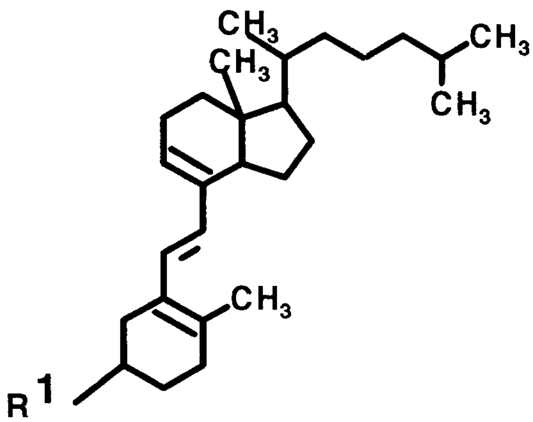

(III)

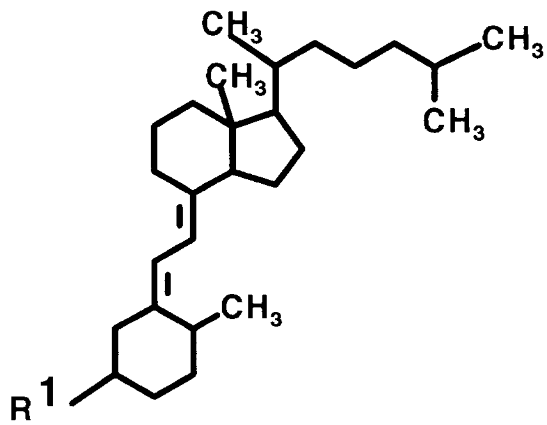

(IV)

où le radical $R^1$ détermine un groupe alkyle $C_1$ à $C_{32}$ et un groupe alkenyle $C_2$ à $C_{32}$ avec un groupe fonctionnel carboxyle respectivement, ou un groupe selon les formules (V) et (VI) :

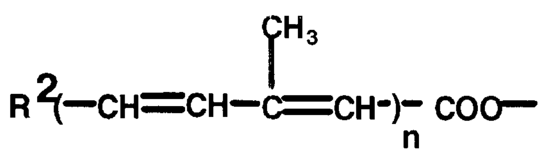

(V)

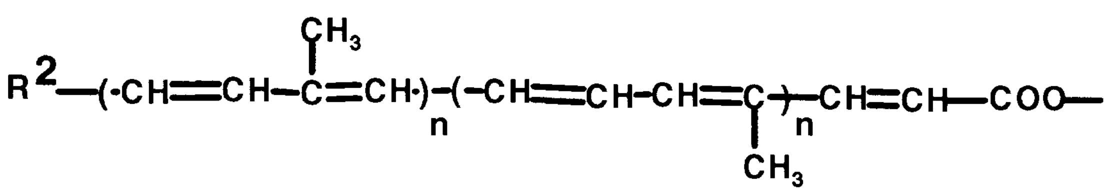

(VI)

dans lesquelles la lettre n désigne les nombres 1, 2, 3, 4 ou 5 et $R^2$ caractérise l'un des radicaux

marqués par les formules suivantes :

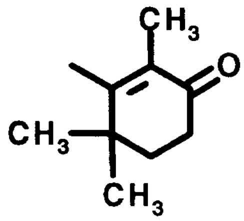
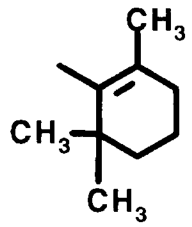
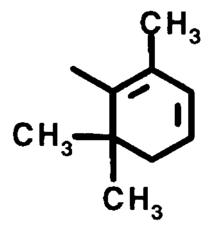
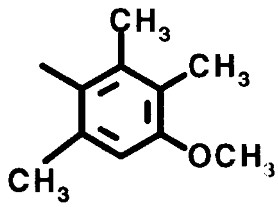
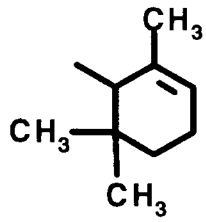
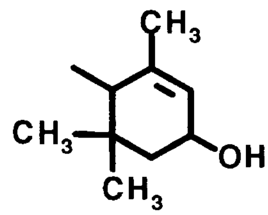
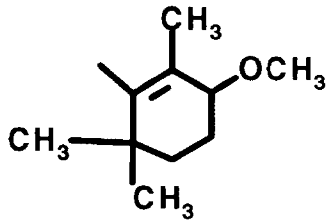
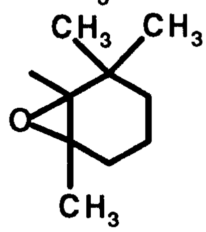
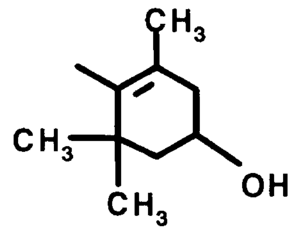
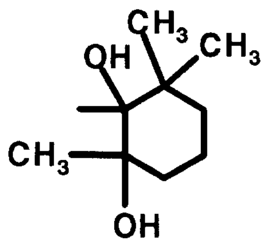
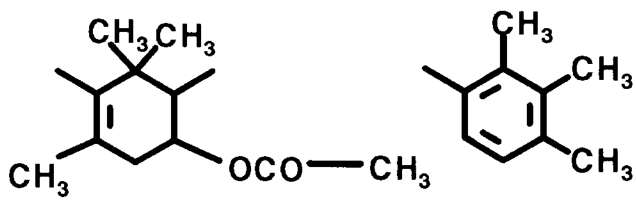
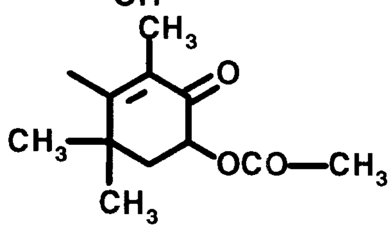

ou

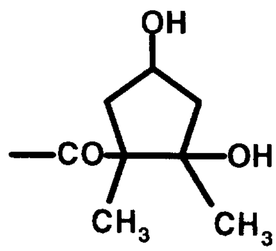

0 à 40 % par poids d'un solvant ou d'un mélange de solvants acceptés en pharmacie qui servent d'agents hydrotropiques ou comme coémulsifiants
0,001 à 90 % par poids d'un surfactant ou d'une combinaison de surfactants acceptés en pharmacie
0 à 10 % par poids de vitamine ou de provitamine
0 à 10 % par poids d'acide gras libre et des auxiliaires et/ou diluants habituels.

**2.** Un concentré spontanément dispersible conforme à la revendication 1), qui contient comme surfactant un émulgateur non-ionique, accepté en pharmacie et ayant une valeur hydrophilique-lipophilique entre 2 et 18, de préférence entre 2 et 6, et/ou un surfactant ester d'acide phosphorique.

**3.** Un concentré spontanément dispersible conforme à la revendication 2), qui contient
7,5 à 15 % par poids d'un composé ester de vitamine-D correspondant aux formules (I) à (IV), et une combinaison de tels composés respectivement
0 à 40 % par poids d'isopropylmyristate, d'isopropylpalmitate ou d'huile neutre
20 à 45 % par poids des esters acide phosphorique mono/diméthyl nonylphenol-10-polyoxyéthylène, selon les formules:

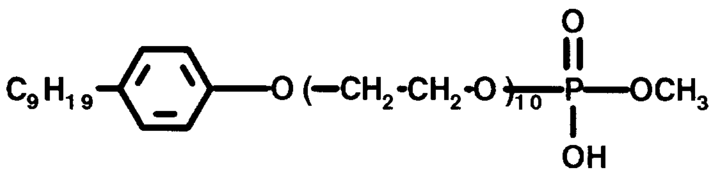

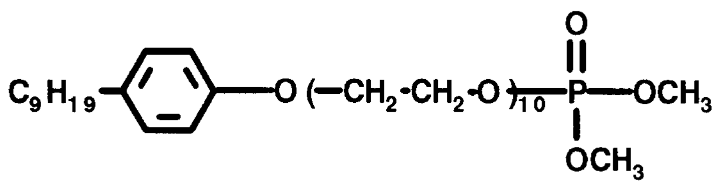

20 à 45 % par poids de l'éther tertiaire d'octylphenylpolyoxyéthylène avec 9-10 groupes oxyéthylène, sans eau.

**4.** Un concentré spontanément dispersible conforme à la revendication 2), qui contient
7,5 à 15 % par poids d'un composé ester de vitamine-D correspondant aux formules (I) à (IV), et une combinaison de tels composés respectivement
0 à 40 % par poids d'isopropylmyristate, d'isopropylpalmitate ou d'huile neutre
20 à 45 % par poids de l'ester acide phosphorique-18-mono/dimethyl-tristyrylphénolpolyoxyéthylène, selon formule:

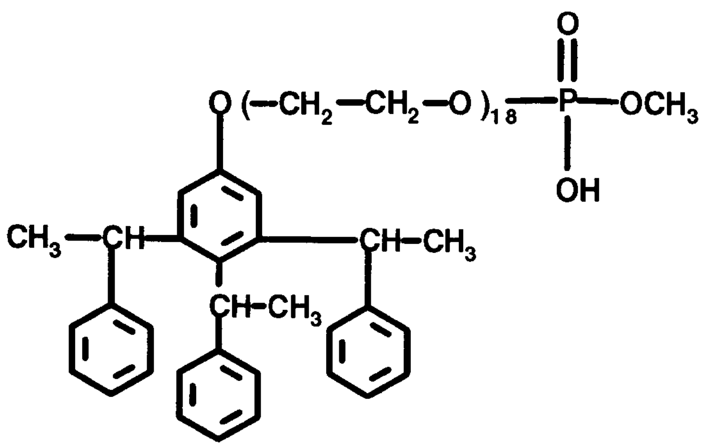

20 à 45 % par poids de l'éther tertiaire d'octylphénylpolyoxyéthylène avec 9 à 10 groupes oxyéthylène, sans eau.

**5.** Les esters formés avec des composés de vitamine-D selon les formules générales (I) à (IV), trouvant leur usage comme médicaments avec activité antitumorale

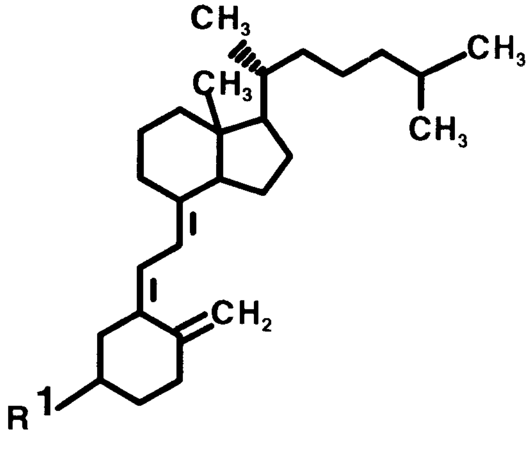

(I)

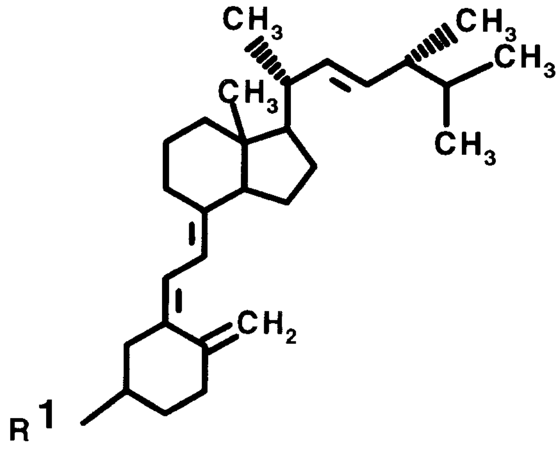

(II)

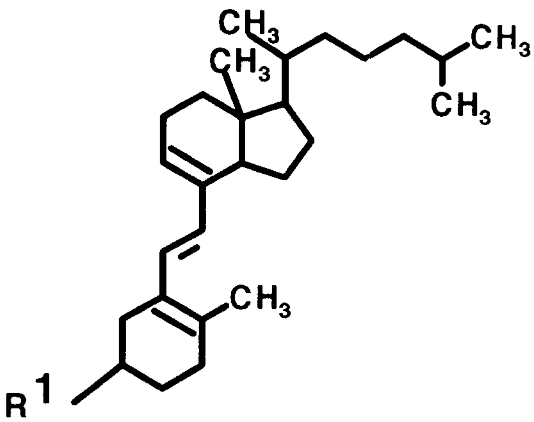

(III)

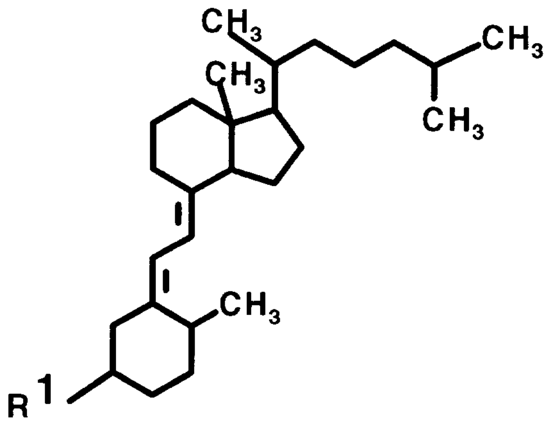

(IV)

où le radical $R^1$ dans les formules (I) à (IV) détermine un groupe alkyle $C_1$ à $C_{32}$ et un groupe alkenyle $C_2$ à $C_{32}$ avec un groupe fonctionnel carboxyle respectivement, ou un groupe selon les formules (V) et (VI) :

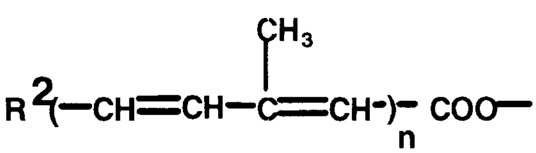

(V)

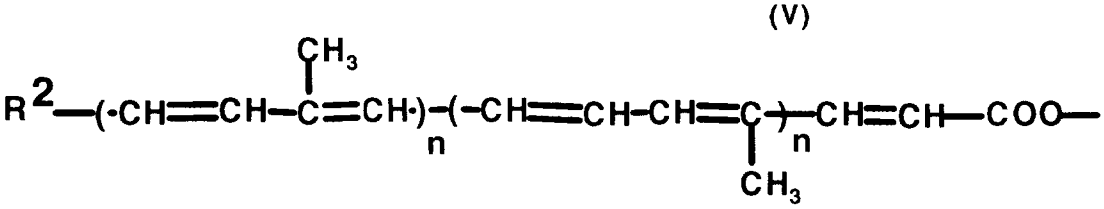

(VI)

dans lesquelles la lettre n désigne les nombres 1, 2, 3, 4 ou 5 et $R^2$ caractérise l'un des radicaux marqués par les formules suivantes :

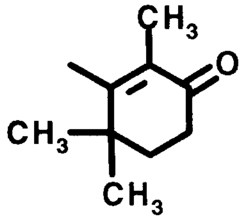 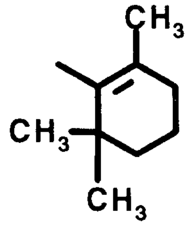 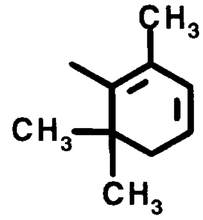

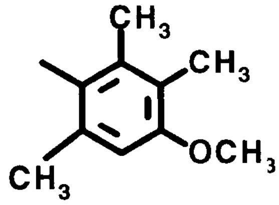 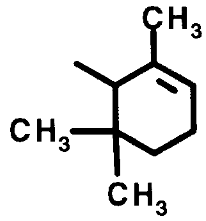 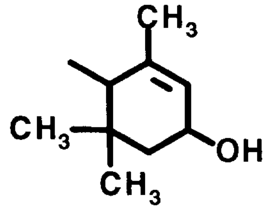

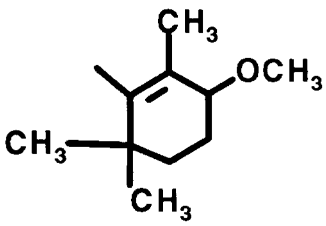 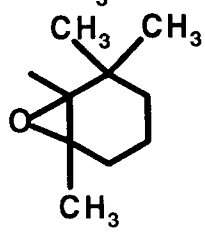 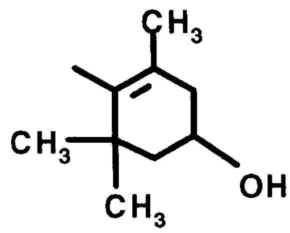

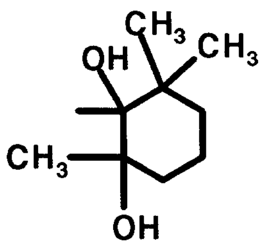 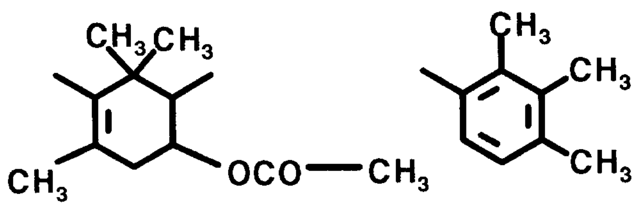

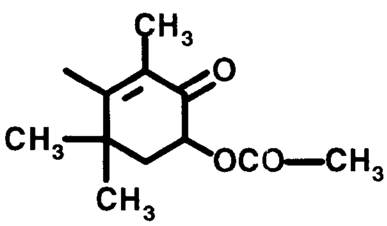 ou 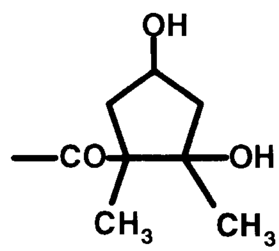

sous réserve que les composés Ergocalciferyl laurate, myristate, palmitate, stéarate, oléate, linoléate et linolénate, ainsi que les cholecalciferyl esters formés avec l'acide eicosa-5,8,11,14,17-pentaenoic et avec l'acide docosa-4,7,10,13,16,19-hexaenoic sont exclus.

**6.** Un composé conforme à la revendication 5, où le radical $R^1$ représente un groupe alkyle carboxyle $C_4$ à $C_{22}$ ou un groupe alkenyle carboxyle $C_4$ à $C_{22}$ respectivement ou désigne le groupe selon formule (IX) ou signifie l'un des radicaux selon les formules (VII), (VIII) et (IX)

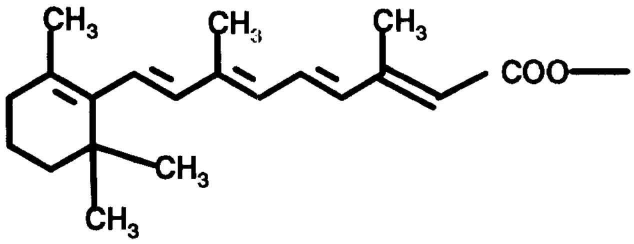

(VII)

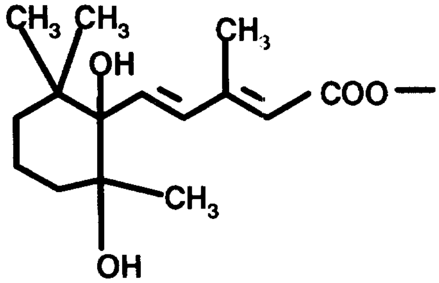

(VIII)

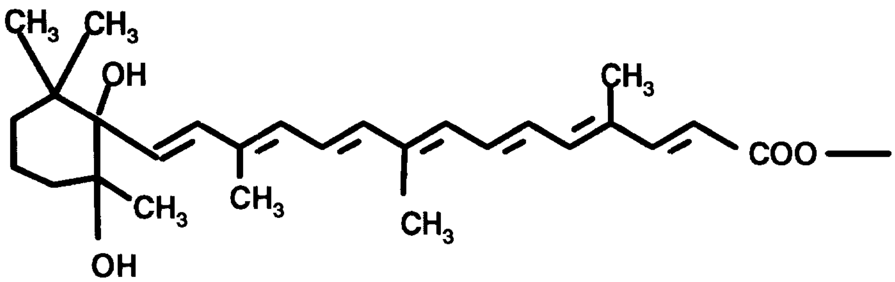

(IX).

7. Les esters formés avec des composés de vitamine-D selon les formules générales (I) à (IV) :

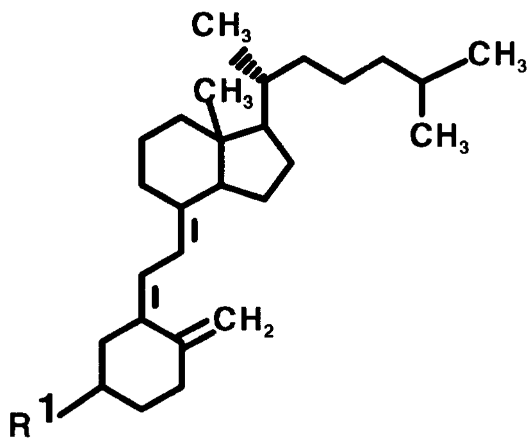

(I)

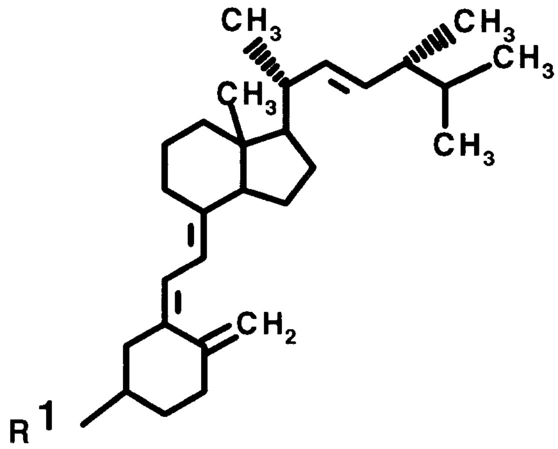

(II)

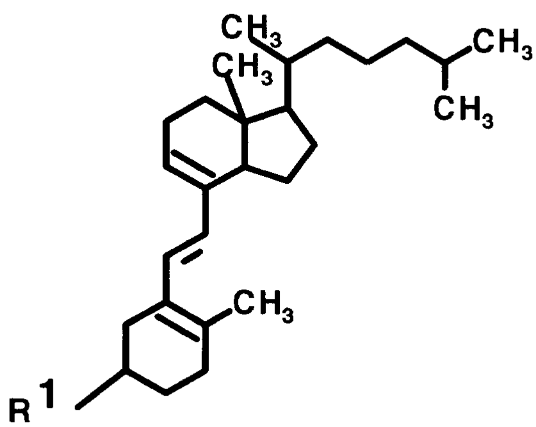

(III)

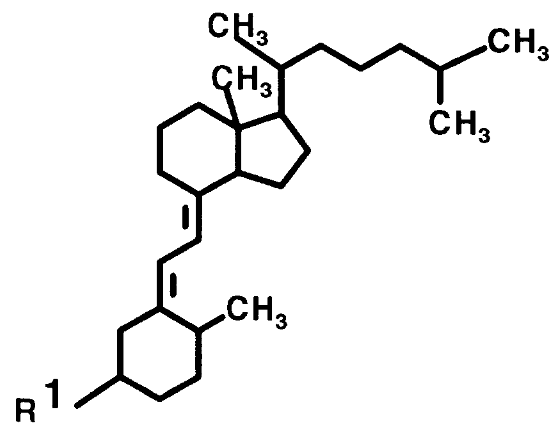

(IV)

où le radical $R^1$ dans les formules (I) à (IV) détermine un groupe alkyle $C_1$ à $C_{32}$ et un groupe alkenyle $C_2$ à $C_{32}$ avec un groupe fonctionnel carboxyle respectivement, ou un groupe selon les formules (V) et (VI) :

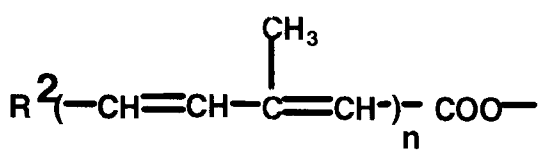

(V)

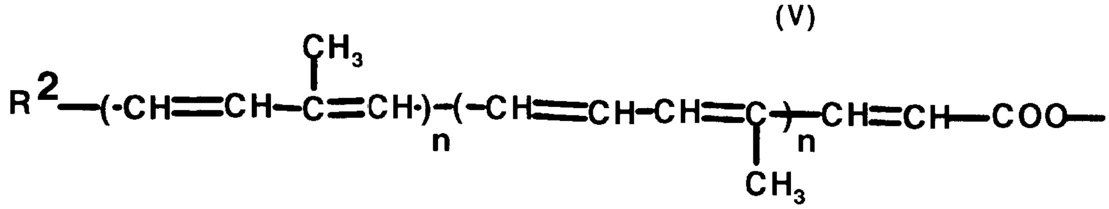

(VI)

dans lesquelles la lettre n désigne les nombres 1 2, 3, 4 ou 5 et $R^2$ caractérise l'un des radicaux marqués par les formules suivantes :

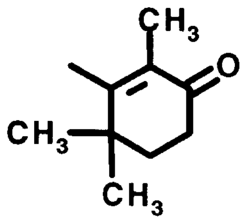 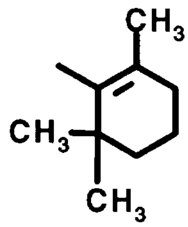 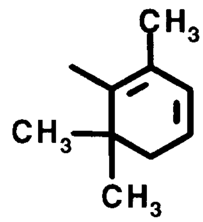

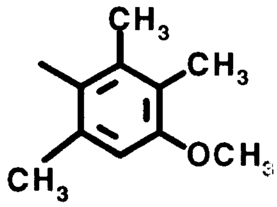 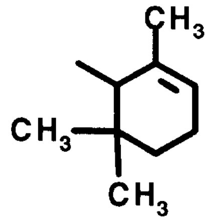 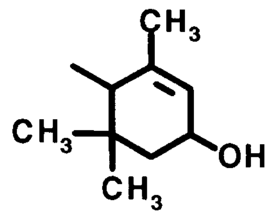

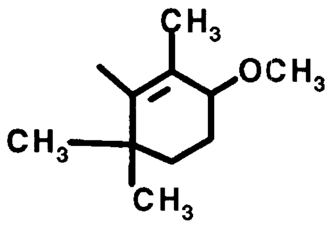 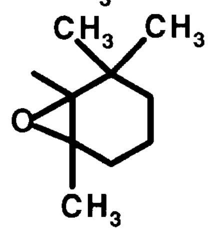 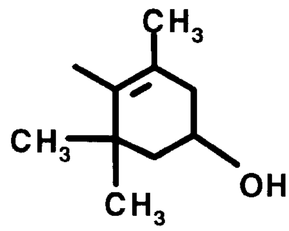

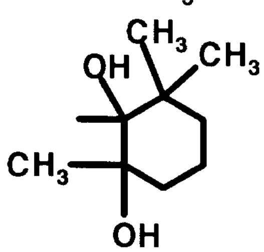 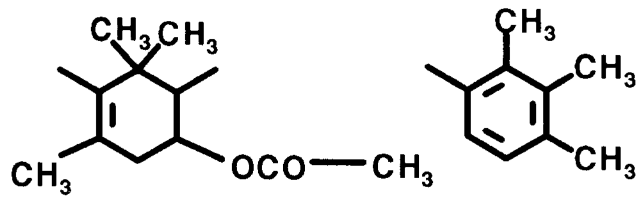

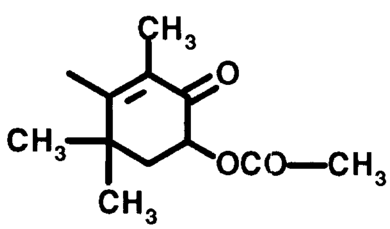 ou 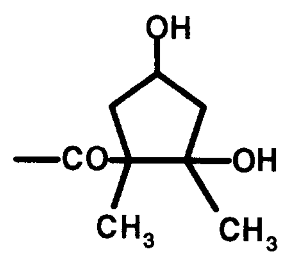

sous réserve que les composés ergocalciferyl acétate, laurate, myristate, palmitate, stéarate, oléate, linoléate et linolénate, ainsi que les cholecalciferyl esters formés avec l'acide eicosa-5,8,11,14,17-pentaenoic et avec l'acide docosa-4,7,10,13,16,19-hexaenoic sont exclus.

**8.** Les esters formés avec des composés de vitamine-D en accord avec la revendication 7, où le radical $R^1$ dans les formules (I) à (IV) détermine un groupe alkyle $C_4$ à $C_{32}$ et un groupe alkenyle $C_4$ à $C_{32}$ avec un groupe fonctionnel carboxyle respectivement, ou signifie l'un des radicaux selon les formules (VII), (VIII) et (IX) :

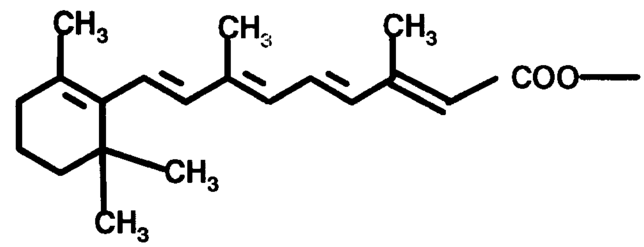

(VII)

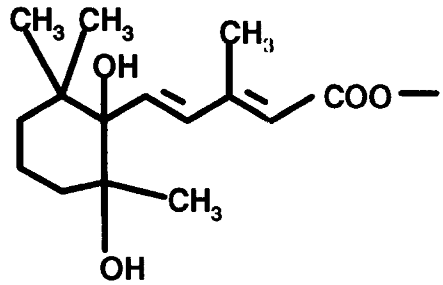

(VIII)

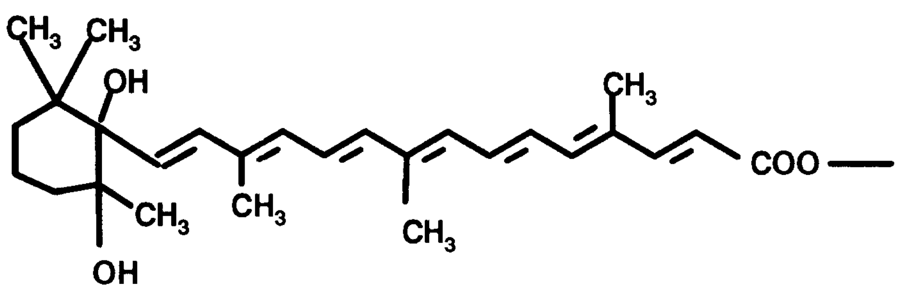

(IX)

sous réserve que les composés ergocalciferyl laurate, myristate, palmitate, stéarate, oléate, linoléate et linolénate, ainsi que les cholecalciferyl esters formés avec l'acide eicosa-5,8,11,14,17-pentaenoic et avec l'acide docosa-4,7,10,13,16,19-hexaenoic sont exclus.

**9.** Les composés Cholecalciferyl-10-undecenoate, Cholecalciferyl-all trans-rétinate et Ergocalciferyl-all trans-rétinate, conformes à la revendication 7).

**10.** Un procédé pour la préparation de composés selon les formules (I) à (IV), conformes à la revendication 7) :

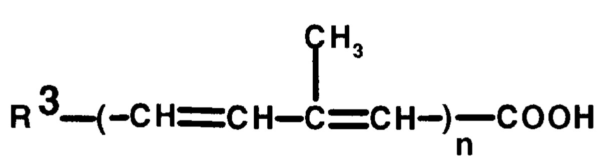

(X)

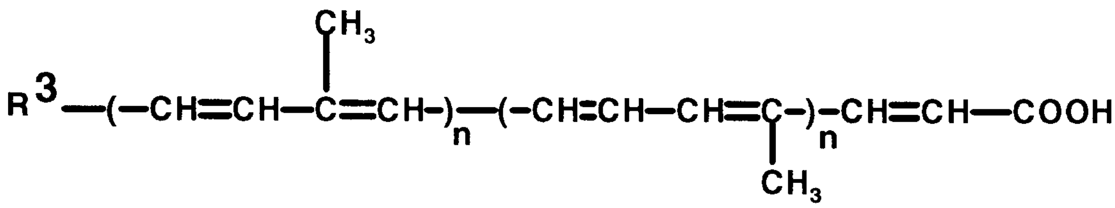

(XI)

dans lesquelles la lettre n désigne les nombres 1, 2, 3, 4 ou 5 et $R_5$ détermine l'un des radicaux selon les formules suivantes

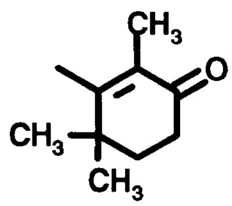
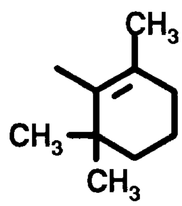
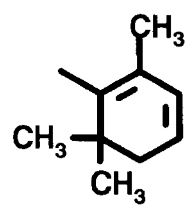
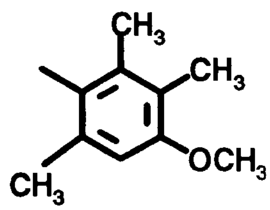
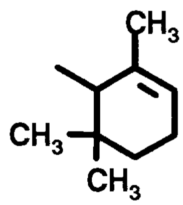
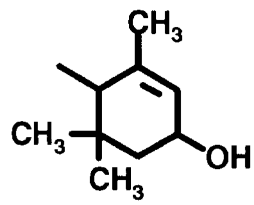
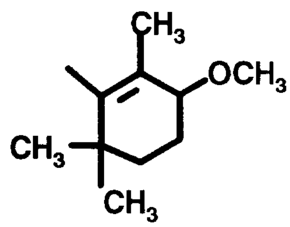
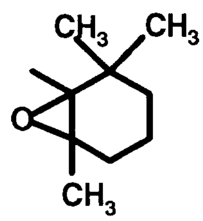
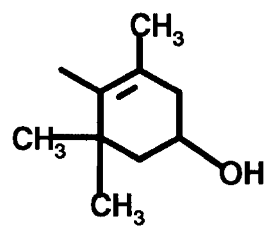
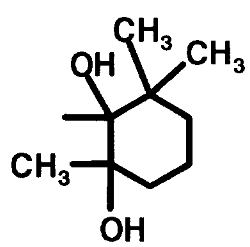
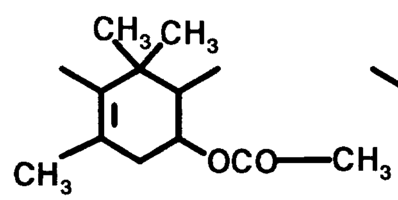
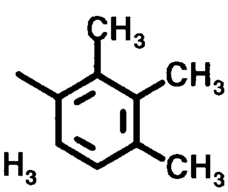
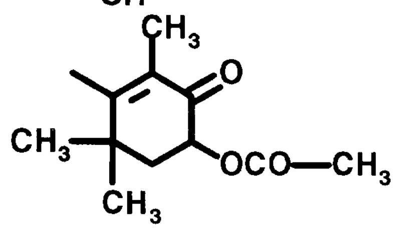

ou

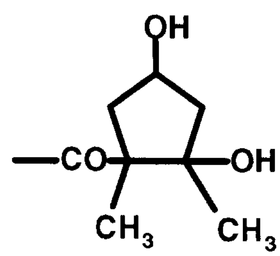

avec N,N'-carbonyle diimidazole à une température de 25 à 70 °C, en présence d'un montant catalytique d'un alcoholat, dans un solvant, suivi par la réaction des imidazoles formés avec un composé de vitamine-D.

**11.** Un procédé pour la préparation de composés en accord avec les formules (I) à (VI) conformes à la revendication 7), qui consiste dans la réaction de composés selon les formules (X), et (XI) et (XII)

respectivement

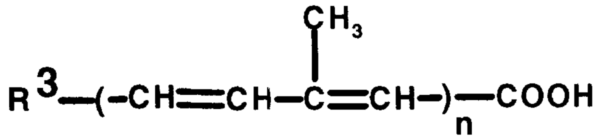

(X)

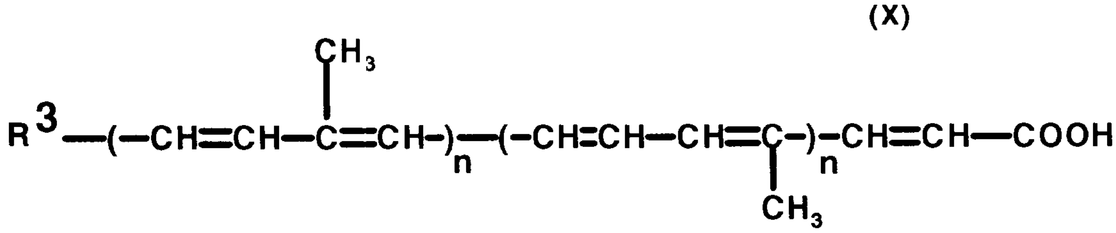

(XI)

$R^4$—COOH (XII)

dans lesquelles $R^4$ signifie un groupe alkyle $C_1$ à $C_{32}$ ou un groupe alkenyle $C_2$ à $C_{32}$ avec un agent de chlorination ou de bromination, suivi par la réaction des chlorures ou bromures formés avec un composé de vitamine-D à une température de 40 à 120 °C dans un solvant et en présence d'un catalysateur.

**12.** Un système thérapeutique qui contient 1 à 95 % par poids de concentré spontanément dispersible conforme aux revendications 1) à 4), et qui est disponible sous forme d'unités de dosage et d'administration comme "micropellets", granules, dragées, suppositoires, ampoules et capsules.

**13.** Un système thérapeutique conforme à la revendication 12), qui contient 44 parts de matériaux pour former les noyaux des granules ou "pellets", 25 parts de concentré préparé en accord avec l'invention et 31 parts de "coating" fait avec le succinate de hydroxyle-méthyle cellulose-acetate.

**14.** Un système thérapeutique conforme à la revendication 12), qui contient 64 parts de matériaux pour former les noyaux des granules ou "pellets" et 36 % de concentré selon l'invention, et qui est conditionné dans des capsules fabriquées avec du succinate de hydroxyle-méthyle cellulose-acetate.

## BATTELLE INSTITUT, FRANKFURT

## IN VITRO TESTS MIT SOLIDEN HUMANTUMOREN

### ZYTOTOXIZITAET LC 50
### EJ 28 BLASEN KARZINOM

72 h Exposition, 3 Tage Kultur

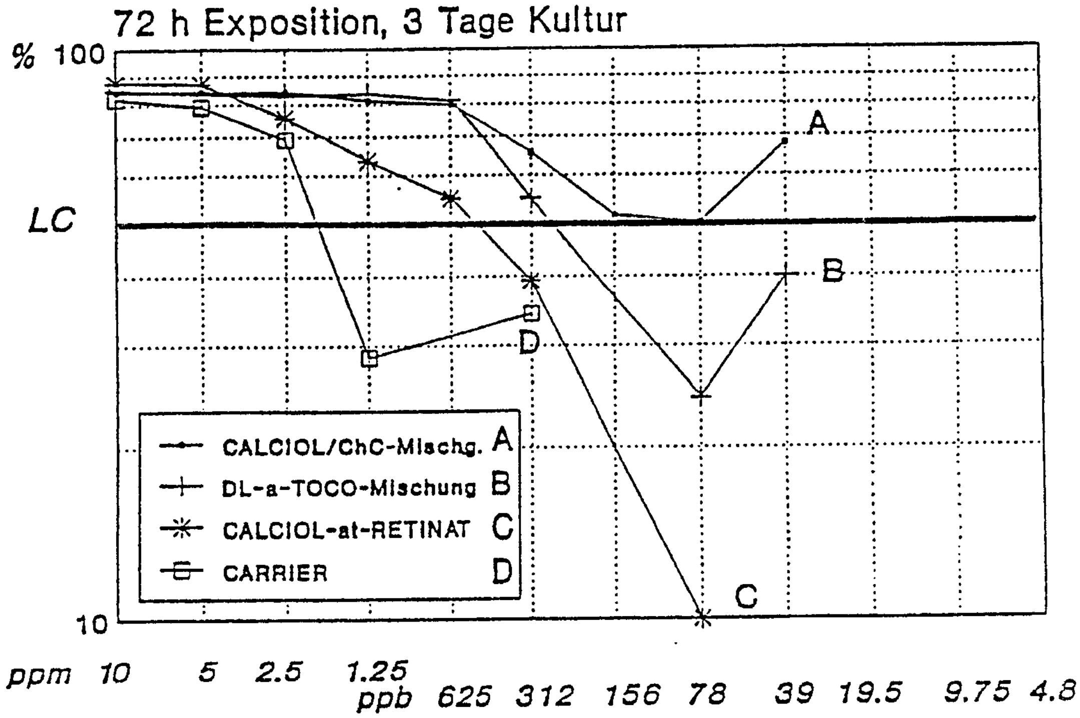


ABBILDUNG 1

### LX-1 LUNGEN KARZINOM

72 h Exposition, 3 Tage Kultur

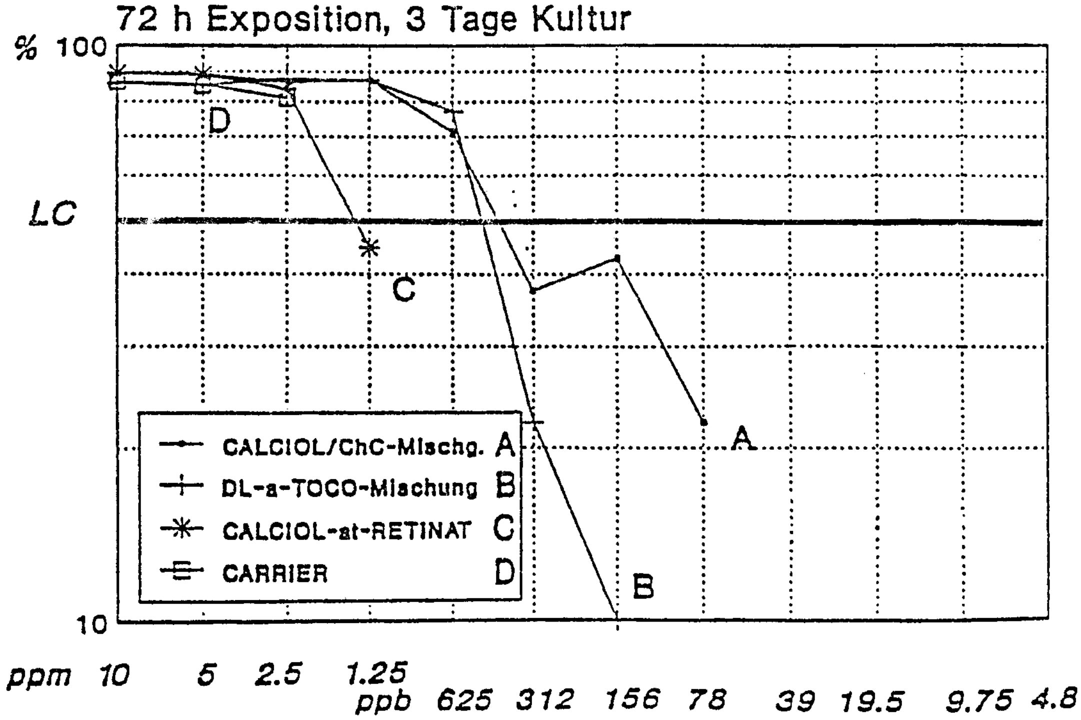


ABBILDUNG 2

CALCIOL-UNDECENOATE

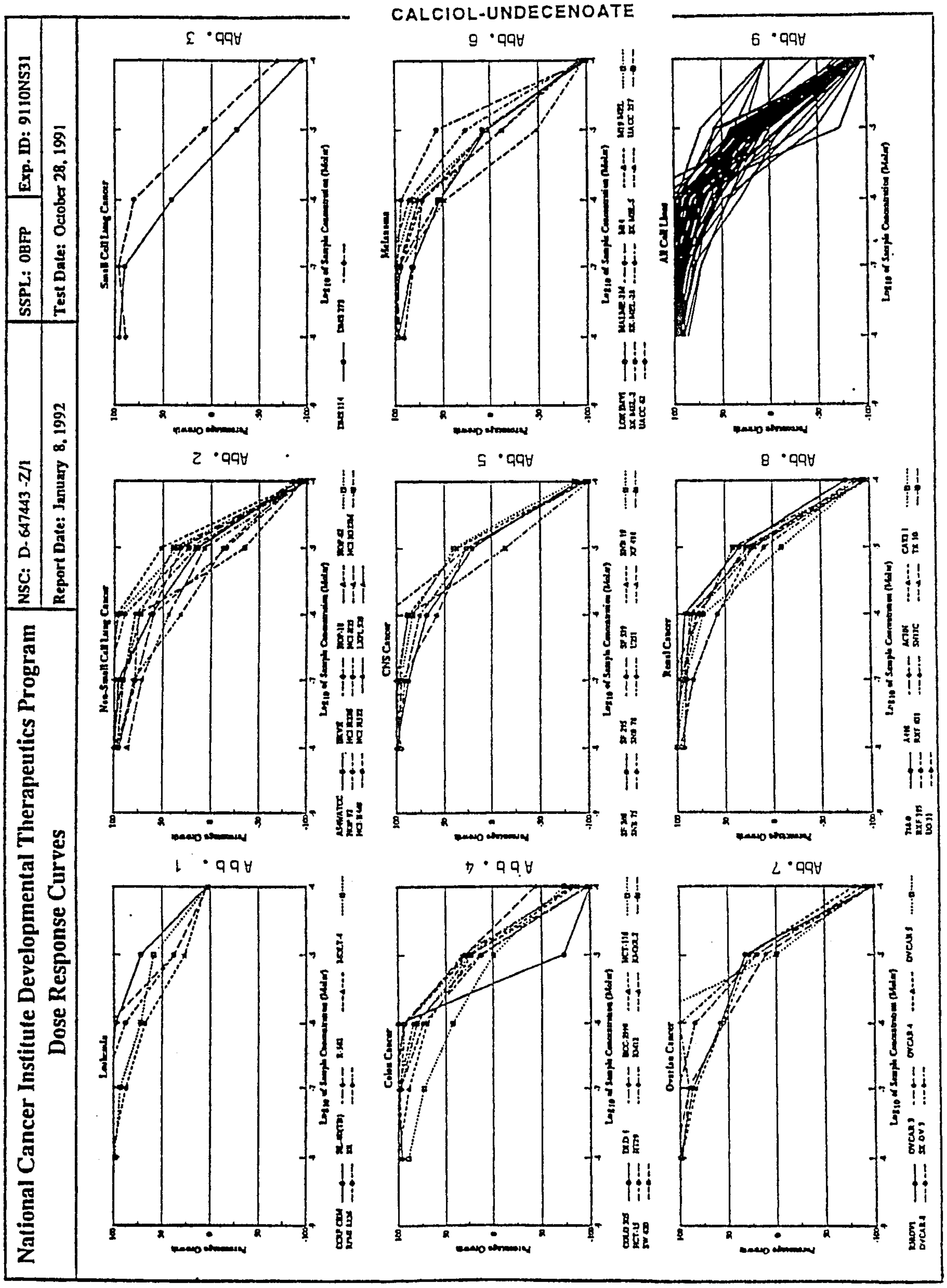

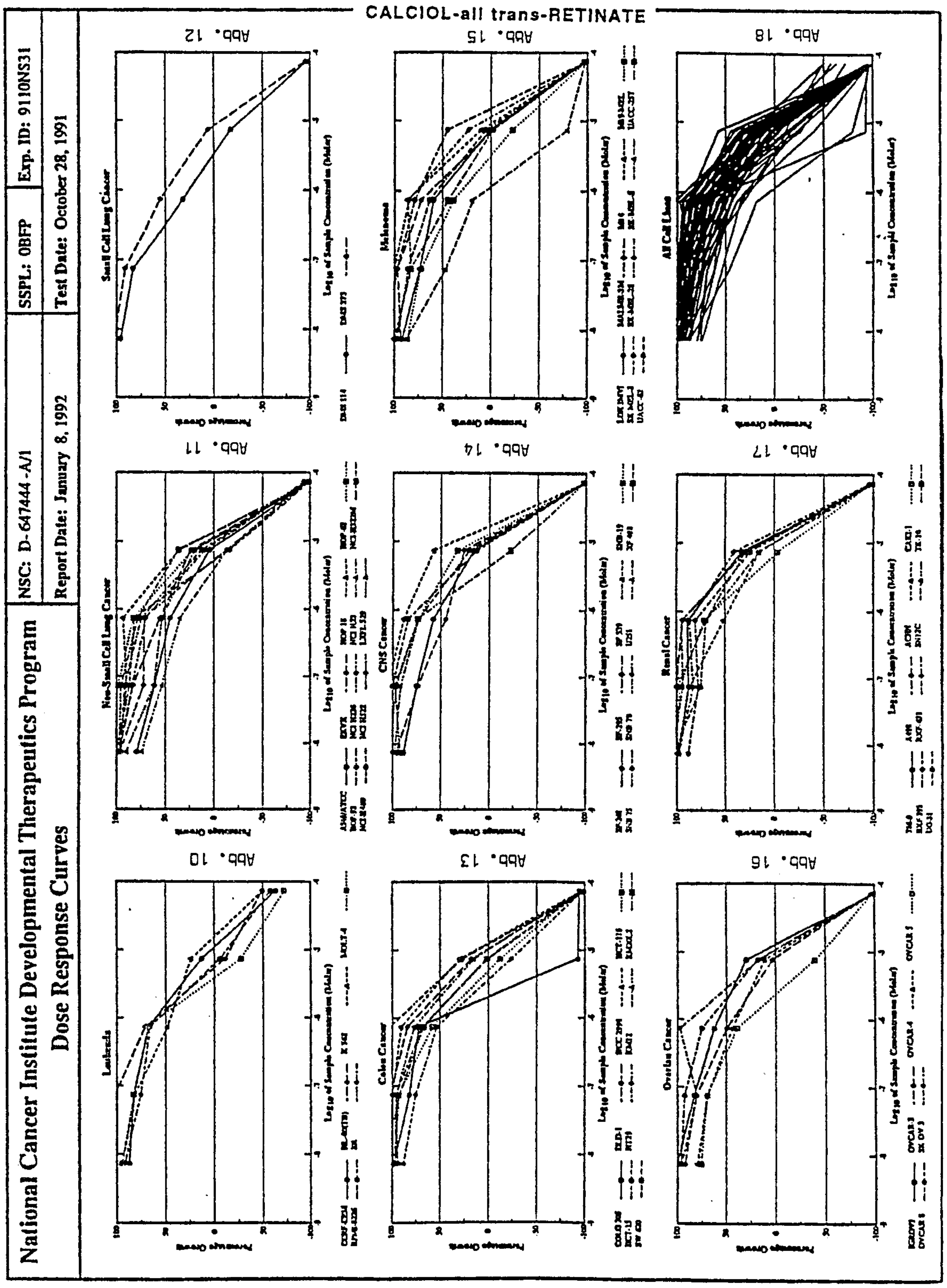
CALCIOL-all trans-RETINATE
National Cancer Institute Developmental Therapeutics Program
Dose Response Curves
NSC: D-647444 -A/1
Report Date: January 8, 1992
SSPL: 0BFP
Test Date: October 28, 1991
Exp. ID: 9110NS31
Leukemia
Non-Small Cell Lung Cancer
Small Cell Lung Cancer
Colon Cancer
CNS Cancer
Melanoma
Ovarian Cancer
Renal Cancer
All Cell Lines
Abb. 10
Abb. 11
Abb. 12
Abb. 13
Abb. 14
Abb. 15
Abb. 16
Abb. 17
Abb. 18

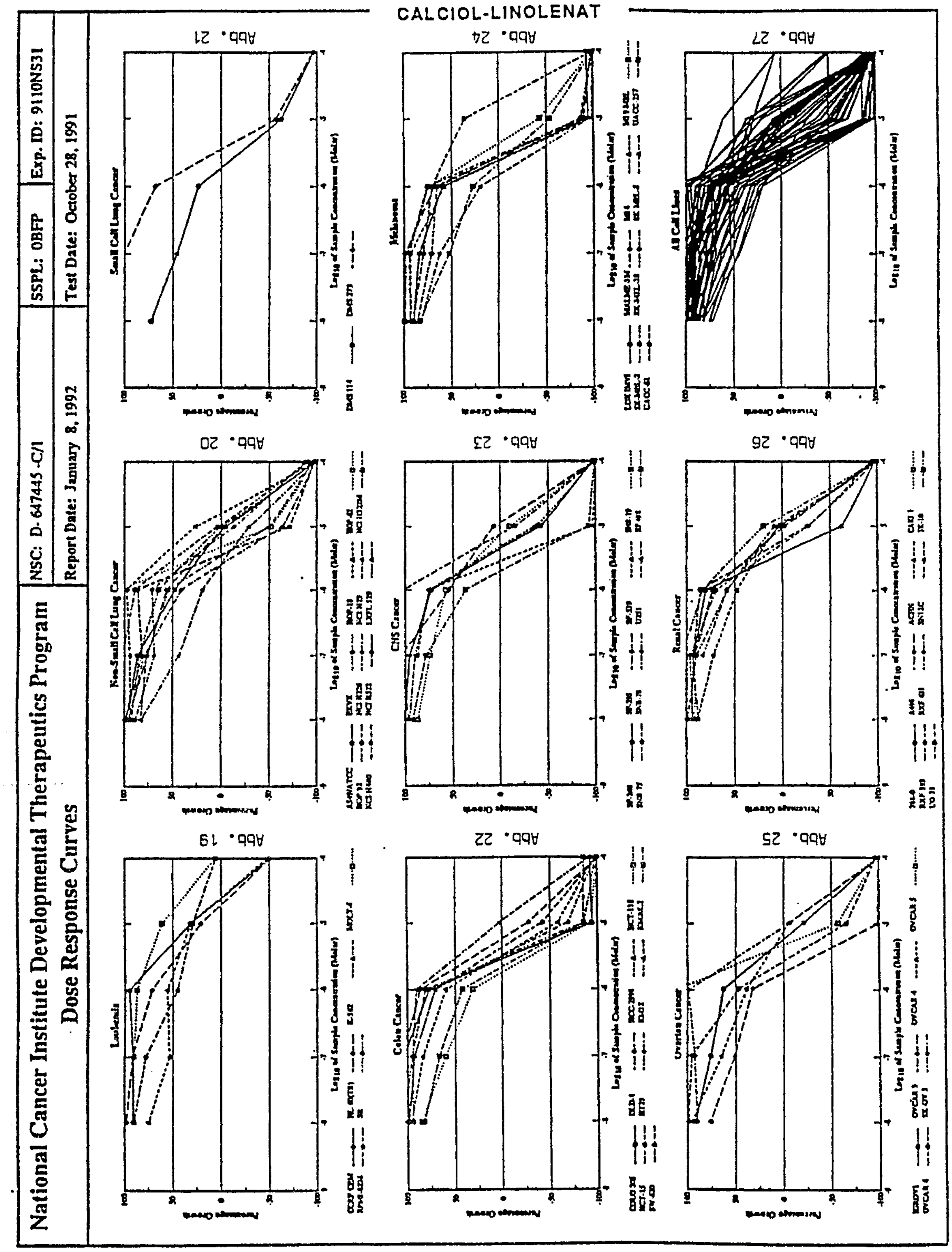
CALCIOL-LINOLENAT
National Cancer Institute Developmental Therapeutics Program
Dose Response Curves
NSC: D-647445-C/1
Report Date: January 8, 1992
SSPL: 0BFP
Test Date: October 28, 1991
Exp. ID: 9110NS31
Leukemia
Abb. 19
Non-Small Cell Lung Cancer
Abb. 20
Small Cell Lung Cancer
Abb. 21
Colon Cancer
Abb. 22
CNS Cancer
Abb. 23
Melanoma
Abb. 24
Ovarian Cancer
Abb. 25
Renal Cancer
Abb. 26
All Cell Lines
Abb. 27
Percentage Growth
Log10 of Sample Concentration (Molar)